# EUROPEAN PATENT SPECIFICATION

(11) **EP 1 917 246 B1**
(45) Date of publication and mention of the grant of the patent: **11.11.2009**
(21) Application number: 06776845.7
(22) Date of filing: 14.08.2006
(51) Int. Cl.: C07D 217/12, C07D 217/26, C07D 239/70, C07D 401/06, C07D 401/12, C07D 403/06, C07D 403/12, C07D 405/06, C07D 405/12, C07D 409/06, C07D 409/12, C07D 417/06, C07D 417/12, A61K 31/395, A61K 31/435

(54) **BENZOQUINAZOLINE DERIVATIVES AND THEIR USE IN TREATING BONE DISORDERS**
BENZOCHINAZOLINDERIVATE UND IHRE VERWENDUNG BEI DER BEHANDLUNG VON KNOCHENERKRANKUNGEN
DERIVES DE BENZOQUINAZOLINE ET UTILISATION DE CEUX-CI DANS LE TRAITEMENT DE MALADIES OSSEUSES

(30) Priority: 15.08.2005 GB 0516723
(43) Date of publication of application: 07.05.2008
(73) Proprietor: Novartis AG, 4056 Basel (CH)
(72) Inventor: AMMON, Sandra, CH-4058 Basel (CH); BEERLI, René, CH-4102 Binningen (CH); WIDLER, Leo, CH-4142 Muenchenstein (CH)
(74) Representative: Vögeli-Lange, Regina
(86) International application number: PCT/EP2006/008036
(87) International publication number: WO 2007/020046

(56) References cited:
- WO-A2-02/102782
- WO-A2-2004/056365

## Description

The present invention relates to bicyclic compounds, in particular to 2-benzoquinazoline derivatives and to pharmaceutical uses thereof.

Accordingly the invention provides a compound of formula (I) or a pharmaceutically acceptable salt or prodrug ester thereof: 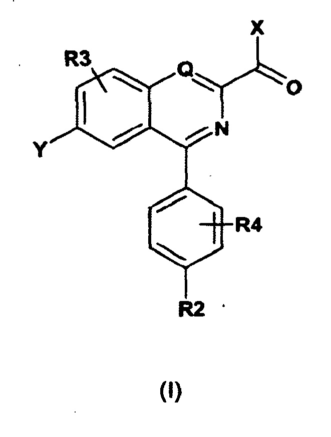 wherein:
Q is CH or N;
R2 is C₁-C₄ alkyl;
Y is R5-O-;
where R5 is ethynyl or propynyl;
X is selected from the group consisting of aryl, heteroaryl, C₁-C₁₀ alkyl, C₁-C₁₀ alkyloxy, cycloalkyl, heterocycloalkyl, aryl C₁-C₄ alkyl, heteroaryl C₁-C₄ alkyl, cycloalkyl C₁-C₄ alkyl, heterocycloalkyl C₁-C₄ alkyl, arylamino, heteroarylamino, aryl C₁-C₄ alkylamino, heteroaryl C₁-C₄ alkylamino, C₁-C₆ alkylamino, C₁-C₆ dialkylamino, aryloxy, heteroaryloxy, aryl C₁-C₄ alkyloxy, heteroaryl C₁-C₄ alkyloxy, cycloalkyl C₁-C₄ alkylamino, heterocycloalkyl C₁-C₄ alkylamino, cycloalkyl C₁-C₄ alkyloxy or heterocycloalkyl C₁-C₄ alkyloxy,
each of which is optionally substituted once or more;
the optional substituent or substituents on X being independently selected from the group consisting of halo, cyano, trifluoromethyl, nitro, hydroxy and optionally substituted (C₁-C₄ alkyl, C₁-C₄ alkyloxy, amino, sulfanyl, sulfonyl, amino, oxycarbonyl, hydroxyl, sulfinyl, aminosulfonyl, sulfonylamino, carbonyl, carbonyloxy, carbonyl amino, carboxyl, acyl, acylamino, or carbamoyl), the optional substituent or substituents being selected from C₁-C₆ alkyl, C₁-C₈ alkyloxy, carboxyl, hydroxyl, hydroxy C₁-C₄ alkyl, each of which in turn may be optionally substituted by C₁-C₆ alkyloxy, C₁-C₆ alkyl, C₁-C₃ fluorinated alkyl, C₁-C₆ alkyloxy, carboxyl, hydroxyl, hydroxy C₁-C₄ alkyl, halo, cyano, nitro;
R3 and R4 each represent one or more substituents independently selected from: H, halo, C₁-C₄ alkyl, C₁-C₄ alkyloxy and CF₃;
the optional substituent or substituents on R3 or R4 being independently selected from the group consisting of C₁-C₄ alkyl, halo, C₁-C₄ alkyloxy, cyano, sulfanyl, sulfonyl, amino, oxycarbonyl, hydroxyl which may in turn be optionally substituted once or more by C₁-C₄ alkyl, halo, C₁-C₄ alkyloxy, cyano, sulfanyl, sulfonyl, amino, oxycarbonyl or hydroxyl.

In a second aspect, the present invention provides a compound of formula (I') or a pharmaceutically acceptable salt or prodrug ester thereof: 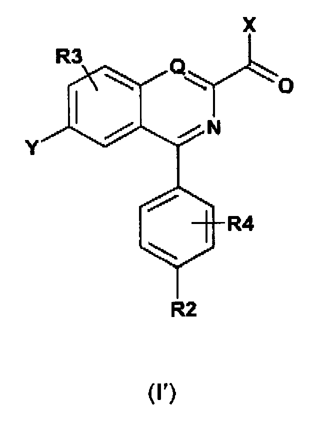 wherein:
Q is CH or N;
R2 is C₁-C₄ alkyl;
Y is R5-O-;
where R5 is ethynyl or propargyl;
X is selected from the group consisting of aryl, heteroaryl, C₁-C₆ alkyl, cycloalkyl, heterocycloalkyl, aryl C₁-C₄ alkyl, heteroaryl C₁-C₄ alkyl, arylamino, aryl C₁-C₄ alkylamino, heteroaryl C₁-C₄ alkylamino, C₁-C₆ alkylamino, C₁-C₆ dialkylamino amino, aryloxy, heteroaryloxy, aryl C₁-C₄ alkyloxy, or heteroaryl C₁-C₄ alkyloxy, each of which is optionally substituted once or more;
the optional substituent or substituents on X being independently selected from the group consisting of C₁-C₄ alkyl, halo, C₁-C₄ alkyloxy, cyano, trifluoromethyl, hydroxy, amino, nitro and C₁-C₄ alkyl substituted (sulfanyl, sulfonyl, amino, oxycarbonyl, hydroxyl, sulfinyl, carbonyl, carboxyl or carbamoyl);
R3 and R4 each represent one or more substituents independently selected from: H, halo, optionally substituted C₁-C₄ alkyl and optionally substituted C₁-C₄ alkyloxy;
the optional substituent or substituents on R3 or R4 being independently selected from the group consisting of C₁-C₄ alkyl, halo, C₁-C₄ alkyloxy, cyano, sulfanyl, sulfonyl, amino, oxycarbonyl and hydroxyl.

With reference to the compounds of formula (I) and (I'), Q is preferably N.

R2 is preferably isopropyl, cyclopropyl or t-butyl. More preferably, R2 is isopropyl. Alternatively, R2 is preferably cyclopropyl.

R3 and R4 are preferably halo or H. More preferably, R3 and R4 are H.

Y is preferably propargyl.

Preferably X is optionally substituted (aryl, heteroaryl, arylamino, heteroarylamino, aryl C₁-C₄ alkylamino, heteroaryl C₁-C₄ alkylamino, aryloxy, heteroaryloxy, C₁-C₆ alkyloxy, aryl C₁-C₄ alkyloxy or heteroaryl C₁-C₄ alkyloxy). More preferably, X is optionally substituted (aryl, heteroaryl, arylamino, heteroarylamino, aryl C₁-C₄ alkylamino, heteroaryl C₁-C₄ alkylamino, aryloxy, C₁-C₆ alkyloxy or aryl C₁-C₄ alkyloxy). Alternatively preferably X is optionally substituted (aryl, heteroaryl or heterocycloalkyl). Alternatively preferably X is optionally substituted aryl, preferably phenyl or naphthalenyl. More preferably, X is optionally substituted phenyl. Alternatively, X is optionally substituted heteroaryl. Preferred heteroaryl groups are furanyl, thienyl, pyrrolyl, thiazolyl, benzothiazolyl, pyridinyl and benz[b]thiophen-2-yl. Alternatively preferably, X is arylamino or heteroarylamino. Preferred arylamino and heteroarylamino groups are pyridinylamino, pyrazolylamino, thioazolylamino, naphthalenylamino, quinolinaylamino, isoquinolinaylamino, phthalazinylamino, benzoimidazolylamino and benzothiazolylamino. Alternatively preferably, X is aryloxy, C₁-C₆ alkyloxy or aryl C₁-C₄ alkyloxy. Alternatively preferably, X is optionally substituted heterocycloalkyl. A preferred heterocycloalkyl substituent is piperidinyl.

Alternatively, X is preferably optionally substituted (aryl, heteroaryl, cycloalkyl or heterocycloalkyl). More preferably, X is optionally substituted phenyl. Yet more preferably, X is a phenyl group substituted in the ortho- or para- position. Alternatively preferably, X is a heteroaryl which is optionally substituted. Alternatively preferably, X is optionally substituted arylamino. More preferably, X is substituted arylamino containing substituent at the meta position.

A third aspect of the invention provides a compound having the formula (II) or a pharmaceutically acceptable salt or prodrug ester thereof: 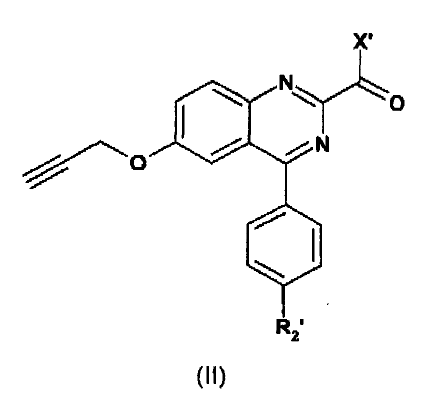 wherein:
X' is selected from the group consisting of aryl, heteroaryl, cycloalkyl, heterocycloalkyl, -C₁-C₄ alkylaryl, -C₁-C₄ alkylheteroaryl, arylamino, heteroarylamino, aryl C₁-C₄ alkylamino, heteroaryl C₁-C₄ alkylamino, aryloxy, heteroaryloxy, aryl C₁-C₄ alkyloxy, heteroaryl C₁-C₄ alkyloxy, aryl C₁-C₄ alkyl, heteroaryl C₁-C₄ alkyl, C₁-C₆ alkyl, -C₁-C₄ alkylamino or amino, each of which is optionally substituted once or more;
the optional substituent or substituents on X' being independently selected from the group consisting of halo, cyano, trifluoromethyl, nitro, hydroxy and optionally substituted (C₁-C₄ alkyl, C₁-C₄ alkyloxy, amino, sulfanyl, sulfonyl, amino, oxycarbonyl, hydroxyl, sulfinyl, carbonyl, carboxyl, acyl, acylamino or carbamoyl); the optional substituent or substituents being selected from C₁-C₆ alkyl, C₁-C₆ alkyloxy, carboxyl, hydroxyl and hydroxy C₁-C₄ alkyl; each of which in turn may be optionally substituted by C₁-C₆ alkyloxy, C₁-C₆ alkyl, C₁-C₆ alkyloxy, carboxyl, hydroxyl, hydroxy C₁-C₄ alkyl, halo, cyano, nitro, and
R₂' is C₁-C₄ alkyl.

With reference to compounds of formula (II), preferably R₂' is isopropyl, t-butyl or cyclopropyl. More preferably, R₂' is isopropyl. Alternatively, R₂' is preferably cyclopropyl.

Preferably X' is optionally substituted (aryl, heteroaryl, arylamino, heteroarylamino, aryl C₁-C₄ alkylamino, heteroaryl C₁₋C₄ alkylamino, aryloxy, heteroaryloxy, C₁-C₆ alkyloxy, aryl C₁-C₄ alkyloxy or heteroaryl C₁-C₄ alkyloxy). More preferably, X' is optionally substituted (aryl, heteroaryl, arylamino, heteroarylamino, aryl C₁-C₄ alkylamino, heteroaryl C₁-C₄ alkylamino, aryloxy, C₁-C₆ alkyloxy or aryl C₁-C₄ alkyloxy). Alternatively preferably X' is optionally substituted (aryl, heteroaryl or heterocycloalkyl). Alternatively preferably X' is optionally substituted aryl, preferably phenyl or naphthalenyl. More preferably, X' is optionally substituted phenyl. Alternatively, X' is optionally substituted heteroaryl. Preferred heteroaryl groups are furanyl, thienyl, pyrrolyl, thiazolyl, benzothiazolyl, pyridinyl and benz[b]thiophen-2-yl. Alternatively preferably, X' is arylamino or heteroarylamino. Preferred heteroarylamino groups are pyridinylamino, pyrazolylemino, thioazolylamino, naphthalenylamino, quinolinaylamino, isoquinolinaylamino, phthalazinylamino, benzoimidazotylamino and benzothiazolylamino. Alternatively preferably, X' is aryloxy, C₁-C₆ alkyloxy or aryl C₁-C₄ alkyloxy. Alternatively preferably, X' is optionally substituted heterocycloalkyl.

WO 2002/102782 and WO 2004/056365 show quinazoline compounds with differing substitution from that of the compounds of the present invention.

For the avoidance of doubt, the terms listed below are to be understood to have the following meaning throughout the present description and claims:

The term "lower", when referring to organic radicals or compounds means a compound or radical with may be branched or unbranched with up to and including 7 carbon atoms.

A lower alkyl group may be branched, unbranched or cyclic and contains 1 to 7 carbon atoms, preferably 1 to 4 carbon atoms. Lower alkyl represents, for example: methyl, ethyl, propyl, butyl, isopropyl, isobutyl, tertiary butyl or 2,2-dimethylpropyl.

A lower alkoxy group may be branched or unbranched and contains 1 to 6 carbon atoms. Lower alkoxy represents, for example: methoxy, ethoxy, propoxy, butoxy, isopropoxy, isobutoxy or tertiary butoxy.

Halo or halogen represents chloro, fluoro, bromo or iodo.

Aryl represents carbocyclic aryl or biaryl.

Carbocyclic aryl is an aromatic cyclic hydrocarbon containing from 6 to 18 ring atoms. It can be monocyclic, bicyclic or tricyclic, for example naphthyl, phenyl, or phenyl mono-, di- or trisubstituted by one, two or three substituents as defined above or below.

Heterocyclic aryl or heteroaryl is an aromatic monocyclic or bicyclic hydrocarbon containing from 5 to 18 ring atoms one or more of which are heteroatoms selected from O, N or S. Preferably there are one or two heteroatoms. Heterocyclic aryl represents, for example: pyridyl, indolyl, quinoxalinyl, quinolinyl, isoquinolinyl, benzothienyl, benzofuranyl, benzopyranyl, benzothiopyranyl, furanyl, pyrrolyl, thiazolyl, oxazolyl, isoxazolyl, triazolyl, tetrazolyl, pyrazolyl, imidazolyl, thienyl, oxadiazolyl, benzimidazolyl. Heterocyclic aryl also includes such substituted radicals with one or more substituents as defined above or below.

Cycloalkyl represents a cyclic hydrocarbon containing from 3 to 12 ring atoms preferably from 3 to 6 ring atoms. Cycloalkyl represents, for example: cyclopropyl, cyclobutyl, cyclopentyl, or cyclohexyl. The cycloalkyl may optionally be substituted with one or more substituents as defined above or below.

Heterocycloalkyl represents a mono-, di- or tricyclic hydrocarbon which may be saturated or unsaturated and which contains one or more, preferably one to three heteroatoms selected from O, N or S. Preferably it contains between three and 18 ring atoms, more preferably between 3 and 8 ring atoms. The term heterocycloalkyl is intended also to include bridged heterocycloalkyl groups such as 3-hyroxy-8-aza-bicyclo[3.2.1]oct-8-yl.

Pharmaceutically acceptable salts include acid addition salts with conventional acids, for example mineral acids, e.g. hydrochloric acid, sulfuric or phosphoric acid, or organic acids, for example aliphatic or aromatic carboxylic or sulfonic acids, e.g. acetic, trifluoroacetic, propionic, succinic, glycolic, lactic, malic, tartaric, citric, ascorbic, maleic, fumaric, hydroxylmaleic, pyruvic, pamoic, methanesulfonic, toluenesulfonic, naphthalenesulfonic, sulfanilic or cyclohexylsulfamic acid; also amino acids, such as arginine and lysine. For compounds of the invention having acidic groups, for example a free carboxy group, pharmaceutically acceptable salts also represent metal or ammonium salts, such as alkali metal or alkaline earth metal salts, e.g. sodium, potassium, magnesium or calcium salts, as well as ammonium salts, which are formed with ammonia or suitable organic amines.

The agents of the invention which comprise free hydroxyl groups may also exist in the form of pharmaceutically acceptable, physiologically cleavable esters, and as such are included within the scope of the invention. Such pharmaceutically acceptable esters are preferably prodrug ester derivatives, such being convertible by solvolysis or cleavage under physiological conditions to the corresponding agents of the invention which comprise free hydroxyl groups. Suitable pharmaceutically acceptable prodrug esters are those derived from a carboxylic acid, a carbonic acid monoester or a carbamic acid, advantageously esters derived from an optionally substituted lower alkanoic acid or an arylcarboxylic acid.

Preferred compounds of formula (I) are:
(4-tert-Butyl-phenyl)-[4-(4-isopropyl-phenyl)-6-propargyloxy-quinazolin-2-yl]-methanone
[4-(4-lsopropyl-phenyl)-6-propargyloxy-quinazolin-2-yl]-phenyl-methanone
(2-Methoxy-phenyl)-[4-(4-Isopropyl-phenyl)-6- propargyloxy -quinazolin-2-yl]-methanone
(3-Methoxy-phenyl)-[4-(4-Isopropyl-phenyl)-6- propargyloxy -quinazolin-2-yl]-methanone
(4-Methoxy-phenyl)-[4-(4-Isopropyl-phenyl)-6- propargyloxy -quinazolin-2-yl]-methanone
(4-Fluoro-phenyl)-[4-(4-isopropyl-phenyl)-6-propargyloxy-quinazolin-2-yl]-methanone
(3-Fluoro-phenyl)-[4-(4-isopropyl-phenyl)-6-propargyloxy-quinazolin-2-yl]-methanone
(3-Chloro-phenyl)-[4-(4-isopropyl-phenyl)-6-propargyloxy-quinazolin-2-yl]-methanone
(4-Chloro-phenyl)-[4-(4-isopropyl-phenyl)-6-propargyloxy-quinazolin-2-yl]-methanone
(4-Fluoro-phenyl)-[4-(4-tert.butyl-phenyl)-6-propargyloxy-quinazolin-2-yl]-methanone
(3-Fluoro-phenyl)-[4-(4-tert.butyl-phenyl)-6-propargyloxy-quinazolin-2-yl]-methanone
(3-Bromo-phenyl)-[4-(4-isopropyl-phenyl)-6-propargyloxy-quinazolin-2-yl]-methanone
(4-Bromo-phenyl)-[4-(4-isopropyl-phenyl)-6-propargyloxy-quinazolin-2-yl]-methanone
(4-Methyl-phenyl)-[4-(4-isopropyl-phenyl)-6-propargyloxy-quinazolin-2-yl]-methanone
(4-lsopropyl-phenyl)-[4-(4-isopropyl-phenyl)-6-propargyloxy-quinazolin-2-yl]-methanone
(4-Ethyl-phenyl)-[4-(4-isopropyl-phenyl)-6-propargyloxy-quinazolin-2-yl]-methanone
(4-Propyl-phenyl)-[4-(4-isopropyl-pheny)-6-propargyloxy-quinazolin-2-yl]-methanone
(4-Cyano-phenyl)-[4-(4-isopropyl-phenyl)-6-propargyloxy-quinazolin-2-yl]-methanone
(4-Methylthio-phenyl)-[4-(4-isopropyl-phenyl)-6-propargyloxy-quinazolin-2-yl]-methanone
(4-Methansulfonyl-phenyl)-[4-(4-isopropyl-phenyl)-6-propargyloxy-quinazolin-2-yl]-methanone
(4-Dimethylamino-phenyl)-[4-(4-isopropyl-phenyl)-6-propargyloxy-quinazolin-2-yl]-methanone
(4-Ethoxy-phenyl)-[4-(4-isopropyl-phenyl)-6-propargyloxy-quinazolin-2-yl]-methanone
4-[4-(4-Isopropyl-phenyl)-6-propargyloxy-quinazoline-2-carbonyl]-benzoic acid methyl ester
(4-Dimethylamino-phenyl)-[4-(4-tert.butyl-phenyl)-6-propargyloxy-quinazolin-2-yl]-methanone
(4-Dimethylamino-phenyl)-[4-(4-cyclopropyl-phenyl)-6-propargyloxy-quinazolin-2-yl]-methanone
4-[4-(4-Isopropyl-phenyl)-6-propargyloxy-quinazoline-2-carbonyl]-benzoic acid ethyl ester
(4-Methoxy-phenyl)-[4-(4-tert.butyl-phenyl)-6-propargyloxy-quinazolin-2-yl]-methanone
(4-Ethoxy-phenyl)-[4-(4-cyclopropyl-phenyl)-6-propargyloxy-quinazolin-2-yl]-methanone
(3-Ethoxy-4-methoxy-phenyl)-[4-(4-isopropyl-phenyl)-6-propargyloxy-quinazolin-2-yl]-methanone
(4-tert.Butyloxy-phenyl)-[4-(4-isopropyl-phenyl)-6-propargyloxy-quinazolin-2-yl]-methanone
(4-Hydroxy)-[4-(4-isopropyl-phenyl)-6-propargyloxy-quinazolin-2-yl]-methanone
(4-Butyloxy)-[4-(4-isopropyl-phenyl)-6-propargyloxy-quinazolin-2-yl]-methanone
Furan-2-yl-[4-(4-isopropyl-phenyl)-6-propargyloxy-quinazolin-2-yl]-methanone
Furan-3-yl-[4-(4-tert.butyl-phenyl)-6-propargyloxy-quinazolin-2-yl]-methanone
Furan-3-yl-[4-(4-isopropyl-phenyl)-6-propargyloxy-quinazolin-2-yl]-methanone
Thiophen-2-yl-[4-(4-isdpropyl-phenyl)-6-propargyloxy-quinazolin-2-yl]-methanone
(3-Methyl-thiophen-2-yl-[4-(4-isopropyl-phenyl)-6-propargyloxy-quinazolin-2-yl]-methanone
Benz[b]thiophen-2-yl-[4-(4-isopropyl-phenyl)-6-propargyloxy-quinazolin-2-yl]-methanone
Thiophen-3-yl-[4-(4-Isopropyl-phenyl)-6-propargyloxy-quinazolin-2-yl]-methanone
(1-Methyl-1H-pyrrol)-2-yl-[4-(4-isopropyl-phenyl)-6-propargyloxy-quinazolin-2-yl]-methanone
4-(4-Isopropyl-phenyl)-6-propargyloxy-quinazoline-2-carboxylic acid ethyl ester
[4-(4-isopropyl-phenyl)-6-propargyloxy-quinazolin-2-yl]-pyridine-3-yl-methanone
[4-(4-Isopropyl-phenyl)-6-propargyloxy-quinazolin-2-yl]-naphthalen-1-yl-methanone
[4-(4-Isopropyl-phenyl)-6-propargyloxy-naphathalen-2-yl]-methanone
Benzothiazol-2-yl -[4-(4-isopropyl-phenyl)-6-propargyloxy-quinazolin-2-yl]-methanone
[4-(4-Isopropyl-phenyl)-6-propargyloxy-quinazolin-2-yl]-thiazol-5-yl-methanone
[4-(4-Isopropyl-phenyl)-6-propargyloxy-quinazolin-2-yl]-piperidin-1-yl-methanone
4-(4-Isopropyl-phenyl)-6-prop-2-ynyloxy-quinazoline-2-carboxylic acid (3-chloro-phenyl)-amide
4-(4-Isopropyl-phenyl)-6-prop-2-ynyloxy-quinazoline-2-carboxylic acid (3-methoxy-phenyl)-amide
4-(4-Isopropyl-phenyl)-6-prop-2-ynyloxy-3,4-dihydro-quinazoline-2-carboxylic acid (3-methylsulfanyl-phenyl)-amide
4-(4-Isopropyl-phenyl)-6-prop-2-ynyloxy-3,4-dihydro-quinazoline-2-carboxylic acid (3-methanesulfonyl-phenyl)-amide
4-(4-Isopropyl-phenyl)-6-prop-2-ynyloxy-quinazoline-2-carboxylic acid (3-trifluoromethylsulfanyl-phenyl)-amide
4-(4-Isopropyl-phenyl)-6-prop-2-ynyloxy-3,4-dihydro-quinazoline-2-carboxylic acid (3-sulfamoyl-phenyl)-amide
4-(4-Isopropyl-phenyl)-6-prop-2-ynyloxy-3,4-dihydro-quinazoline-2-carboxylic acid [3-(2-hydroxy-ethanesulfonyl)-phenyl]-amide
4-(4-Isopropyl-phenyl)-6-prop-2-ynyloxy-3,4-dihydro-quinazoiine-2-carboxylic acid (5-ethanesulfonyl-2-hydroxy-phenyl)-amide
4-(4-Isopropyl-phenyl)-6-prop-2-ynyloxy-quinazoline-2-carboxylic acid (3-nitro-phenyl)-amide
4-(4-Isopropyl-phenyl)-6-prop₋2-ynyloxy-quinazoline-2-carboxylic acid (3-cyano-phenyl)-amide
3-{[4-(4-Isopropyl-phenyl)-6-prop-2-ynyloxy-quinazoline-2-carbonyl]-amino}-benzoic acid methyl ester
3-{[4-(4-Isopropyl-phenyl)-6-prop-2-ynyloxy-quinazoline-2-carbonyl]-amino}-benzoic acid ethyl ester
3-{[4-(4-Isopropyl-phenyl)-6-prop-2-ynyloxy-quinazoline-2-carbonyl]-amino}-benzoic acid isopropyl ester
3-{[4-(4-Isopropyl-phenyl)-6-prop-2-ynyloxy-quinazoline-2-carbonyl]-amino}-benzoic acid tert-butyl ester
4-(4-Isopropyl-phenyl)-6-prop-2-ynyloxy-quinazoline-2-carboxylic acid (3-carbamoyl-phenyl)-amide
4-(4-Isopropyl-phenyl)-6-prop-2-ynyloxy-quinazoline-2-carboxylic acid (3-acetyl-phenyl)-amide
3-{[4-(4-Isopropyl-phenyl)-6-prop-2-ynyloxy-quinazoline-2-carbonyl]-amino}-5-methoxybenzoic acid methyl ester
4-(4-Isopropyl-phenyl)-6-prop-2-ynyloxy-quinazoline-2-carboxylic acid (3-methylcarbamoyl-phenyl)-amide
4-(4-Isopropyl-phenyl)-6-prop-2-ynyloxy-quinazoline-2-carboxylic acid (3-tert-butylcarbamoyl-phenyl)-amide
4-(4-Isopropyl-phenyl)-6-prop-2-ynyloxy-quinazoline-2-carboxylic acid (3-dimethylcarbamoyl-5-trifluoromethyl-phenyl)-amide
3-{[4-(4-Isopropyl-phenyl)-6-prop-2-ynyloxy-quinazoline-2-carbonyl]-amino}-5-trifluoromethylbenzoic acid methyl ester
3-{[4-(4-Isopropyl-phenyl)-6-prop-2-ynyfoxy-quinazoline-2-carbonyl]-amino}-5-trifluoromethylbenzoic acid isopropyl ester
2-Fluoro-5-{[4-(4-isopropyl-phenyl)-6-prop-2-ynyloxy-quinazoline-2-carbonyl]-amino}-benzoic acid methyl ester
2-Fluoro-5-{[4-(4-isopropyl-phenyl)-6-prop-2-ynyloxy-quinazoline-2-carbonyl]-amino}-benzoic acid isopropyl ester
2-Chloro-5-{[4-(4-isopropyl-phenyl)-6-prop-2-ynyloxy-quinazoline-2-carbonyl]-amino}-benzoic acid methyl ester
2,5-Dichloro-3-{[4-(4-isopropyl-phenyl)-6-prop-2-ynyloxy-quinazoline-2-carbonyl]-amino}-benzoic acid methyl ester
2,5-Dichloro-3-{[4-(4-isopropyl-phenyl)-6-prop-2-ynyloxy-quinazoline-2-carbonyl]-amino}-benzoic acid isopropyl ester
4-(4-Isopropyl-phenyl)-6-prop-2-ynyloxy-quinazoline-2-carboxylic acid (3-cyano-5-fluorophenyl)-amide
4-(4-Isopropyl-phenyl)-6-prop-2-ynyloxy-quinazoline-2-carboxylic acid (3,4-dicyano-phenyl)-amide
4-(4-Isopropyl-phenyl)-6-prop-2-ynyloxy-quinazoline-2-carboxylic acid (4-cyano-3-trifluoromethyl-phenyl)-amide
4-(4-Isopropyl-phenyl)-6-prop-2-ynyloxy-quinazoline-2-carboxylic acid (3-trifluoromethylphenyl)-amide
4-(4-Isopropyl-phenyl)-6-prop-2-ynyloxy-quinazoline-2-carboxylic acid (4-acetylamino-3-trifluoromethyl-phenyl)-amide
4-(4-Isopropyl-phenyl)-6-prop-2-ynyloxy-quinazoline-2-carboxylic acid (3-methoxy-5-trifluoromethyl-phenyl)-amide
4-(4-Isopropyl-phenyl)-6-prop-2-ynyloxy-quinazoline-2-carboxylic acid (3,5-bis-trifluoromethyl-phenyl)-amide
4-(4-Isopropyl-phenyl)-6-prop-2-ynyloxy-quinazoline-2-carboxylic acid (3-fluoro-5-trifluoromethyl-phenyl)-amide
4-(4-Isopropyl-phenyl)-6-prop-2-ynyloxy-quinazoline-2-carboxylic acid (4-fluoro-3-trifluoromethyl-phenyl)-amide
3-{[4-(4-Isopropyl-phenyl)-6-prop-2-ynyloxy-quinazoline-2-carbonyl]-amino}-2-methyl-benzoic acid methyl ester
3-{[4-(4-Isopropyl-phenyl)-6-prop-2-ynyloxy-quinazoline-2-carbonyl]-amino}-4-methyl-benzoic acid methyl ester
3-{[4-(4-Isopropyl-phenyl)-6-prop-2-ynyloxy-quinazoline-2-carbonyl]-amino}-4-methoxybenzoic acid methyl ester
5-{[4-(4-Isopropyl-phenyl)-6-prop-2-ynyloxy-quinazoline-2-carbonyl]-amino}-isophthalic acid dimethyl ester
4-{[4-(4-Isopropyl-phenyl)-6-prop-2-ynyloxy-quinazoline-2-carbonyl]-amino}-phthalic acid dimethyl ester
4-(4-Isopropyl-phenyl)-6-prop-2-ynyloxy-quinazoline-2-carboxylic acid (3,5-dichloro-phenyl)-amide
4-(4-Isopropyl-phenyl)-6-prop-2-ynyloxy-quinazoline-2-carboxylic acid (3,4-dichloro-phenyl)-amide
4-(4-Isopropyl-phenyl)-6-prop-2-ynyloxy-quinazoline-2-carboxylic acid (3-chloro-4-fluorophenyl)-amide
4-(4-Isopropyl-phenyl)-6-prop-2-ynyloxy-quinazoline-2-carboxylic acid (4-chloro-3-trifluoromethyl-phenyl)-amide
5-{[4-(4-Isopropyl-phenyl)-6-prop-2-ynyloxy-quinazoline-2-carbonyl]-amino}-pyridine-2-carboxylic acid methyl ester
5-{[4-(4-Isopropyl-phenyl)-6-prop-2-ynyloxy-quinazoline-2-carbonyl]-amino}-nicotinic acid methyl ester
5-{[4-(4-Isopropyl-phenyl)-6-prop-2-ynyloxy-quinazoline-2-carbonyl]-amino}-nicotinic acid isopropyl ester
[4-(4-Isopropyl-phenyl)-6-prop-2-ynyloxy-quinazolin-2-yl]-pyrrol-1-yl-methanone
4-(4-Isopropyl-phenyl)-6-prop-2-ynyloxy-quinazoline-2-carboxylic acid (5-methyl-1 H-pyrazol-3-yl)-amide
(2-{[4-(4-Isopropyl-phenyl)-6-prop-2-ynyloxy-quinazoline-2-carbonyl]-a mino}-thiazol-4-yl)-acetic acid ethyl ester
4-(4-Isopropyl-phenyl)-6-prop-2-ynyloxy-quinazoline-2-carboxylic acid naphthalen-1-ylamide
4-(4-Isopropyl-phenyl)-6-prop-2-ynyloxy-quinazoline-2-carboxylic acid isoquinolin-8-ylamide
4-(4-Isopropyl-phenyl)-6-prop-2-ynyloxy-quinazoline-2-carboxylic acid phthalazin-5-ylamide
4-(4-Isopropyl-phenyl)-6-prop-2-ynyloxy-quinazoline-2-carboxylic acid quinolin-5-ylamide
4-(4-Isopropyl-phenyl)-6-prop-2-ynyloxy-quinazoline-2-carboxylic acid quinolin-8-ylamide
4-(4-Isopropyl-phenyl)-6-prop-2-ynyloxy-quinazoline-2-carboxylic acid isoquinolin-4-ylamide
4-(4-Isopropyl-phenyl)-6-prop-2-ynyloxy-quinazoline-2-carboxylic acid (5-acetyl-quinolin-8-yl)-amide
4-(4-Isopropyl-phenyl)-6-prop-2-ynyloxy-quinazoline-2-carboxylic acid (3-bromo-6-methoxy-quinolin-8-yl)-amide
4-(4-Isopropyl-phenyl)-6-prop-2-ynyloxy-quinazoline-2-carboxylic acid quinolin-2-ylamide
4-(4-Isopropyl-phenyl)-6-prop-2-ynyloxy-quinazoline-2-carboxylic acid quinolin-6-ylamide
4-(4-Isopropyl-phenyl)-6-prop-2-ynyloxy-quinazoline-2-carboxylic acid (2-methyl-quinolin-6-yl)-amide
(6-{[4-(4-Isopropyl-phenyl)-6-prop-2-ynyloxy-quinazoline-2-carbonyl]-amino}-quinolin-8-yloxy)-acetic acid ethyl ester
4-(4-Isopropyl-phenyl)-6-prop-2-ynyloxy-quinazoline-2-carboxylic acid (1 H-benzoimidazol-4-yl)-amide
4-(4-Isopropyl-phenyl)-6-prop-2-ynyloxy-quinazoline-2-carboxylic acid benzothiazol-2-ylamide
4-(4-Isopropyl-phenyl)-6-prop-2-ynyloxy-quinazoline-2-carboxylic acid (benzo[1,3]dioxol-5-ylmethyl)-amide
4-(4-Isopropyl-phenyl)-6-prop-2-ynyloxy-quinazoline-2-carboxylic acid (thiophen-2-ylmethyl)-amide
4-(4-Isopropyl-phenyl)-6-prop-2-ynyloxy-quinazoline-2-carboxylic acid 3-methoxy-phenyl ester
1-(4-Isopropyl-phenyl)-7-prop-2-ynyloxy-isoquinoline-3-carboxylic acid ethyl ester
1-(4-Isopropyl-phenyl)-7-prop-2-ynyloxy-isoquinoline-3-carboxylic acid 1,2-dimethyl-propyl ester
1-(4-Isopropyl-phenyl)-7-prop-2-ynyloxy-isoquinoline-3-carboxylic acid isobutyl ester
1-(4-Isopropyl-phenyl)-7-prop-2-ynyloxy-isoquinoline-3-carboxylic acid cyclopropylmethyl ester
1-(4-Isopropyl-phenyl)-7-prop-2-ynyloxy-isoquinoline-3-carboxylic acid benzyl ester
1-(4-Isopropyl-phenyl)-7-prop-2-ynyloxy-isoquinoline-3-carboxylic acid 2-methoxy-benzyl ester
1-(4-Isopropyl-phenyl)-7-prop-2-ynyloxy-isoquinoline-3-carboxylic acid 3-methoxy-benzyl ester
1-(4-Isopropyl-phenyl)-7-prop-2-ynyloxy-isoquinoline-3-carboxylic acid 4-methoxycarbonylbenzyl ester
1-(4-Isopropyl-phenyl)-7-prop-2-ynyloxy-isoquinoline-3-carboxylic acid phenethyl ester
1-(4-Isopropyl-phenyl)-7-prop-2-ynyloxy-isoquinoline-3-carboxylic acid 1-phenyl-ethyl ester
1-(4-Isopropyt-phenyl)-7-prop-2-ynyloxy-isoquinoline-3-carboxylic acid [3-(2-hydroxy-ethanesulfonyl)-phenyl]-amide
[1-(4-Isopropyl-phenyl)-7-prop-2-ynyloxy-isoquinolin-3-yl]-(3-methoxy-phenyl)-methanone
[1-(4-Isopropyl-phenyl)-7-prop-2-ynyloxy-isoquinolin-3-yl]-(4-methoxy-phenyl)-methanone.

According to a fourth aspect of the invention there is provided a pharmaceutical composition comprising a compound of formula (I) in association with a pharmaceutically acceptable excipient, diluent or carrier.

According to a fifth aspect of the invention there is provided a compound of formula (I) for promoting the release of parathyroid hormone.

It is now well established that controlled treatment of patients with parathyroid hormone (PTH) and analogues and fragments thereof can have a pronounced anabolic effect on bone formation. Thus compounds which promote PTH release, such as the compounds of the present invention may be used for preventing or treating conditions of bone which are associated with increased calcium depletion or resorption or in which stimulation of bone formation and calcium fixation in the bone is desirable.

Thus the invention allows for a method for preventing or treating bone conditions which are associated with increased calcium depletion or resorption or in which stimulation of bone formation and calcium fixation in the bone is desirable in which an effective amount of a compound of formula (I) as defined above, or a pharmaceutically-acceptable and -cleavable ester, or acid addition salt thereof is administered to a patient in need of such treatment.

In a sixth aspect the invention provides a process for preparation of a compound of formula (I) in free or salt form, comprising the step of:
(i) for cases where Q is N, reacting a compound of formula (III) with a compound of formula (IV) and an ammonium salt in the presence of a suitable solvent: 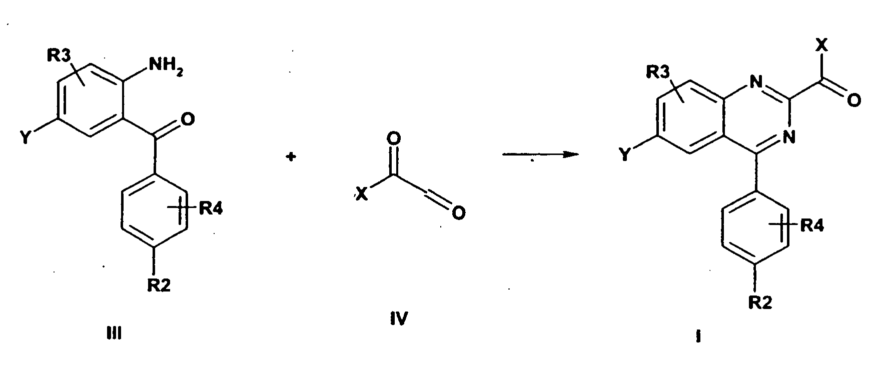
   A preferred ammonium salt is ammonium acetate. Preferably the solvent contains water. Suitable solvents are ethanol/water. The presence of an oxidizing agent, e.g. DDQ is also preferred.
   The compound of formula III may be prepared by any suitable route, for example, when Y is propargyloxy and R2 is isopropyl, as follows: 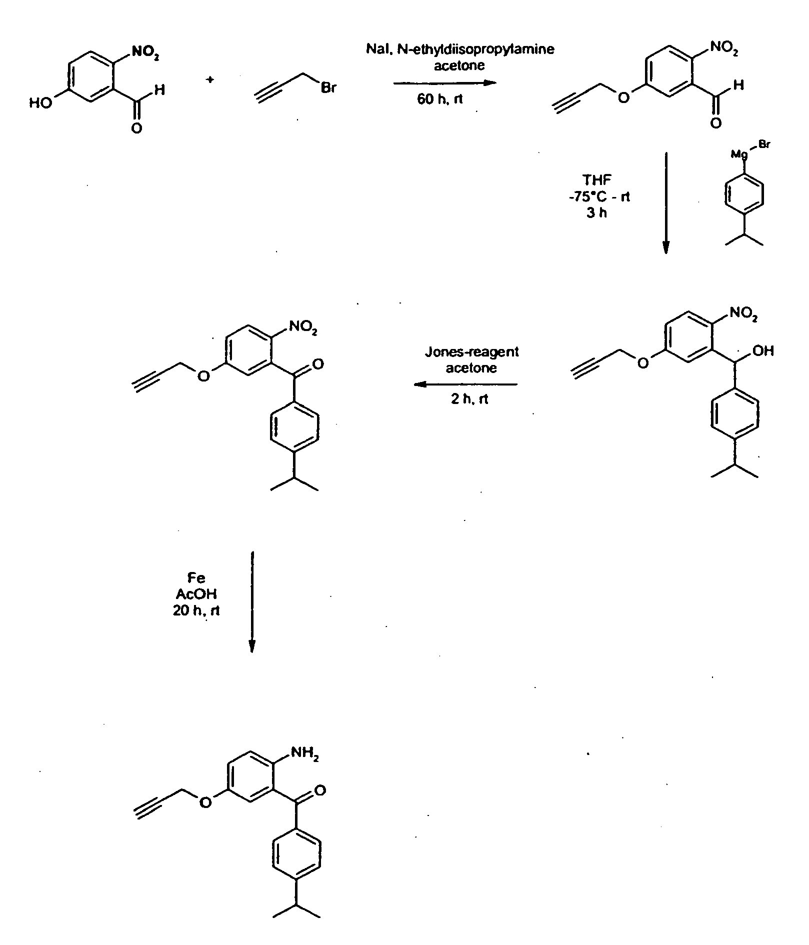
(ii) when Q is CH, reacting a compound of formula V 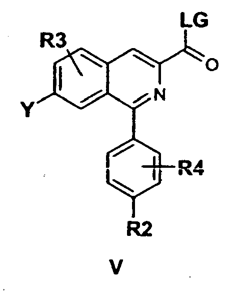 wherein LG represents a suitable leaving group, for example a Weinreb amide (N-methoxy-N-methylamide)
   with an organometallic reagent of formula VI:

   X-Met VI

   where Met is Li, a Grignard reagent (-MgBr) or other suitable organometallic under suitable anhydrous conditions; or
(iii) reacting a compound of formula Va 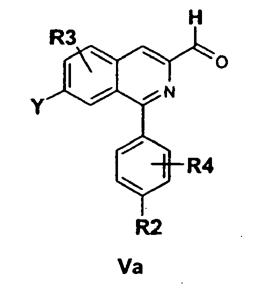 with an organometallic reagent of formula VI:

   X-Met VI

   under suitable anhydrous conditions followed by oxidation to the carbonyl compound by an appropriate oxidation agent; or
(iv) reacting a compound of formula VII 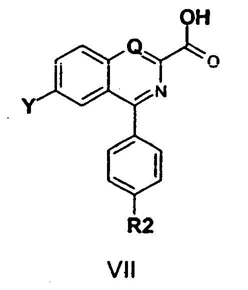 with a compound X-H wherein the H forms part of an amino or hydroxy group, the reaction being carried out in the presence of a coupling reagent; or
(v) reacting a compound of formula VIII 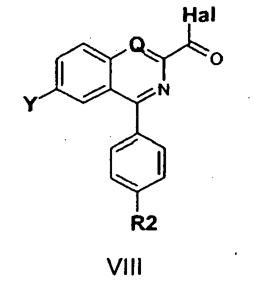
wherein Hal is halogen or a leaving group
with a compound X-H wherein the H forms part of an amino or hydroxy group, the reaction being carried out in the presence of a coupling reagent.

The compound of formula V can be prepared by any suitable route, for example as follows:

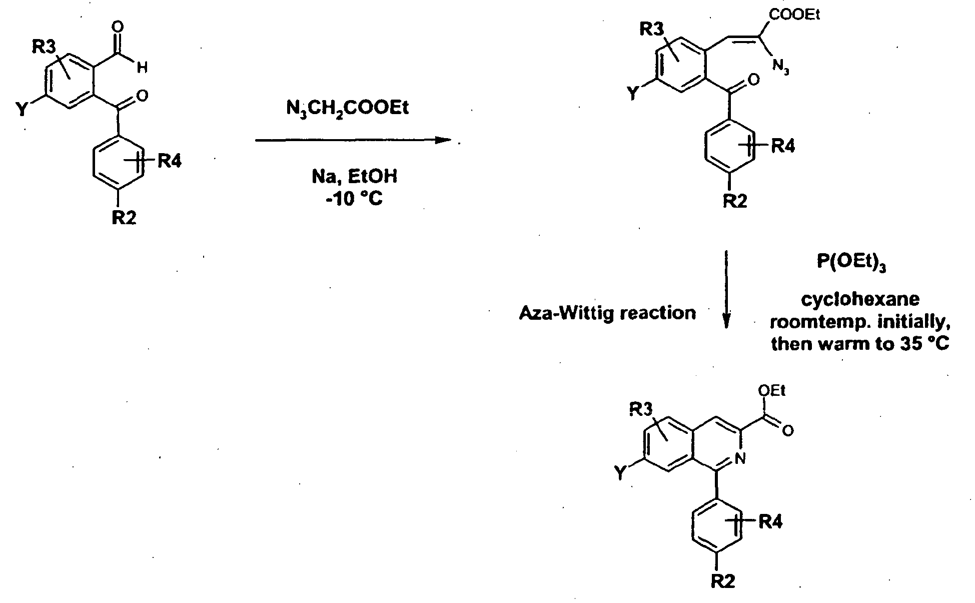

The compounds of formula I in free form may be converted into salt forms in conventional manner and vice-versa.

The compounds of the invention can be recovered from the reaction mixture and purified in conventional manner. Isomers, such as enantiomers, may be obtained in conventional manner, e.g. by fractional crystallization or asymmetric synthesis from corresponding asymmetrically substituted, e.g. optically active starting materials.

In a seventh aspect the invention includes the use of a compound of formula (I) in the manufacture of a medicament for preventing or treating bone conditions which are associated with increased calcium depletion or resorption or in which stimulation of bone formation and calcium fixation in the bone is desirable.

In an eighth aspect the invention provides a combination comprising a therapeutically effective amount of a compound as described above and a second drug substance selected from: calcium, a calcitonin or an analogue or derivative thereof, a steroid hormone, a partial estrogen agonist or estrogen-gestagen combination, a SERM (Selective Estrogen Receptor Modulator), vitamin D or an analogue thereof or PTH, a PTH fragment or a PTH derivative for simultaneous, separate or sequential treatment.

Agents of the invention may be prepared by processes described below, which are intended to be examples:

The analytical HPLC conditions are as follows:
- Instrument and settings:: Agilent 110 System with G1311A quaternary pump (0.8 ml dead volume), G1313A autosampler (1 µl injection volume), G1316A column compartment (35° C), G1315A diode array detector (detection by UV absorption at 210 nm - 250 nm wave length), G1946A mass spectrometer with APC ionization.
- Column:: Waters Symmetry C8, 50 x 2.1 mm, 3.5 µm mean particle size, flow rate 1.0 ml/min.
- Linear gradient:: 5% B in A to 95% B in A within 2.0 min. A: water containing 5% acetonitirile and 0.1 % TFA; B: acetonitrile containing 0.1 % TFA.

### Example 1: (4-tert-Butyl-phenyl)-[4-(4-isopropyl-phenyl)-6-propargyloxy-quinazolin-2-yl]-methanone

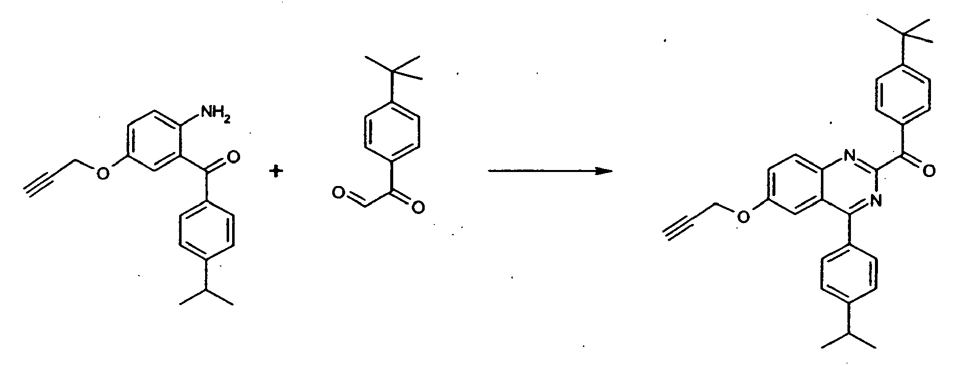

A mixture of 250 mg (0.85 mmol) (2-amino-5-propargyloxy-phenyl)-(4-isopropyl-phenyl)-methanone and 20 mg (2.6 mmol) ammonium acetate is dissolved in 2.5 ml ethanol and 0.82 ml water. To this mixture, 243 mg (1.28 mmol) of (4-tert-butyl-phenyl)-oxo-acetaldehyd is added and stirring continued for 8 hours at rt. After extraction with water / diethyl ether the organic layer is dried over magnesium sulfate and concentrated under reduced pressure to yield a yellow oil. This is purified by chromatography (hexane/ethyl acetate). After concentration and drying under HV the product is treated with a mixture of diethyl ether / petroleum ether to yield a yellow solid.
m.p. 166-168 °C.
¹H-NMR (300 MHz, CDCl₃): 8.21 (d, 1H), 8.13 (d, 2H), 7.85 (d, 2H), 7.66-7.72 (m, 2H), 7.52 (d, 2H), 7.44 (d, 2H), 4.82 (d, 2H), 3.05 (hept, 1H), 2.64 (t, 1H), 1.38 (s, 9H), 1.35 (d, 6H).
MS: 463 (M+1)⁺

### Preparation of the starting material:

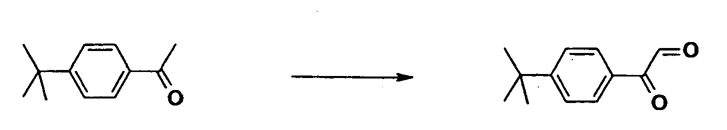

A mixture of 1.26 g (11.3 mmol) selenium dioxide in 11 ml of a dioxane/water (30:1) is warmed to 50 °C. On obtaining a clear solution 2.0 g (11.3 mmol) of 4-tert-butyl-acetophenone is added in portions and the resulting mixture is stirred overnight at reflux. The solid parts of the resulting suspension are separated off and the solution is concentrated *in vacuo.* The residue is distributed between ethyl acetate and water, the organic layer dried over magnesium sulfate and concentrated. Purification by chromatography (hexane/ethyl acetate) yields (4-tert-butyl-phenyl)-oxo-acetaldehyde as a yellow oil which solidifies after drying under HV.

The compounds of the following examples are prepared in an analogous manner using the appropriate starting materials:

### Example 2: [4-(4-lsopropyl-phenyl)-6-propargyloxy-quinazolin-2-yl]-phenyl-methanqne

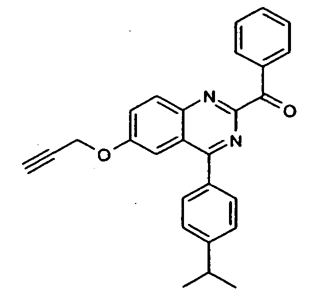

¹H-NMR (CDCl₃, 300 MHz): 8.15 (d, 1H), 8.12 (d, 2H), 7.80 (d, 2H), 7.65 - 7.60 (m, 2H), 7.56 (t, 1 H), 7.44 (t, 2H). 7.38 (d, 2H), 4.76 (d, 2H), 2.99 (hept, 1H), 2.59 (t, 1H), 1.29 (d, 6H).
MS: 407 (M+1)⁺

The starting material phenylglyoxal monohydrate is commercially available.

### Example 3: (2-Methoxy-phenyl)-[4-(4-Isopropyl-phenyl)-6- propargyloxy -quinazolin-2-yl]-methanone

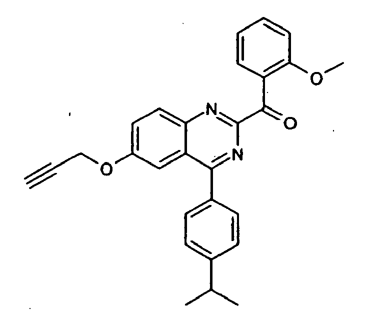

¹H-NMR (CDCl₃, 300 MHz): 8.17 (d, 1H), 7.83 (dd, 1H), 7.77 (d, 2H), 7.66 - 7.61 (m, 2H), 7.52 (td, 1H), 7.39 (d, 2H), 7.09 (td, 1 H), 6.95 (d, 1H), 4.79 (d, 2H), 3.53 (s, 3H), 3.01 (hept, 1 H), 2.63 (t, 1 H), 1.32 (d, 6H).
MS: 437 (M+1)⁺

The starting material 2-methoxyphenylglyoxal hydrate is commercially available.

### Example 4: (3-Methoxy-phenyl)-[4-(4-Isopropyl-phenyl)-6- propargyloxy -quinazolin-2-yl]-methanone

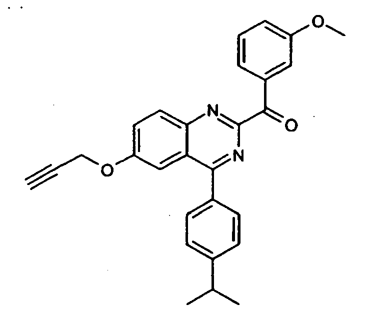

¹H-NMR (CDCl₃, 300 MHz): 8.23 (d, 1 H), 7.85 (d, 2H), 7.75 - 7.66 (m, 4H), 7.44 (d, 2H), 7.38 (t, 1 H), 7.17 (ddd, 1 H), 4.82 (d, 2H), 3.88 (s, 3H), 3.04 (hept, 1 H), 2.64 (t, 1 H), 1.34 (d, 6H).
MS: 437 (M+1)⁺

The starting material 3-methoxyphenylglyoxal hydrate is commercially available.

### Example 5: (4-Methoxy-phenyl)-[4-(4-Isopropyl-phenyl)-6- propargyloxy -quinazolin-2-yl]-methanone

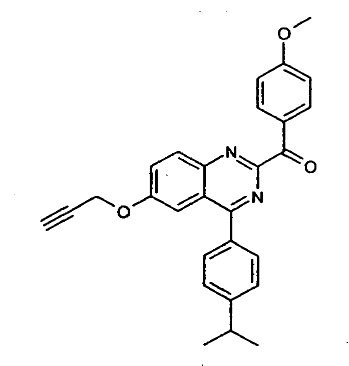

¹H-NMR (CDCl₃, 300 MHz): 8.23 (dd, 1H), 8.18 (d, 2H),7.84 (d, 2H), 7.70 - 7.65 (m, 2H), 7.43 (d, 2H), 6.97 (d, 2H), 4.81 (d, 2H), 3.90 (s, 3H), 3.03 (hept, 1H), 2.63 (t, 1H), 1.34 (d, 6H).
MS: 437 (M+1)⁺

The starting material 4-methoxyphenylglyoxal hydrate is prepared according to the literature, for example by SeO₂ oxidation of (4-methoxy-phenyl)-ethanone.

### Example 6: (4-Fluoro-phenyl)-[4-(4-isopropyl-phenyl)-6-propargyloxy-quinazolin-2-yl]-methanone

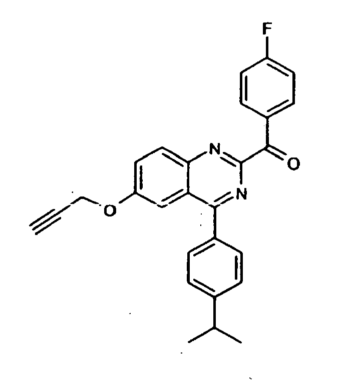

m. p. 124-126 °C
¹H-NMR (300 MHz, CDCl₃): 8.15-8.28 (m, 3H), 7.83 (d, 2H), 7.65-7.70 (m, 2H), 7.43 (d, 2H), 7.12-7.20 (m, 2H), 4.80 (d, 2H), 3.04 (hept, 1H), 2.63 (t, 1H), 1.33 (d, 6H).
MS: 425 (M+1)⁺

The starting material (4-fluoro-phenyl)-oxo-acetaldehyde is prepared according to the literature, for example by SeO₂ oxidation of 1-(4-fluoro-phenyl)-ethanone, analogously to Example 1.

### Example 7: (3-Fluoro-phenyl)-[4-(4-isopropyl-phenyl)-6-propargyloxy-quinazolin-2-yl]-methanone

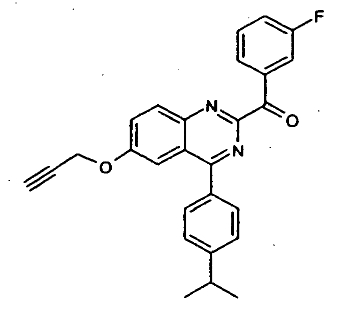

m. p. 118-120 °C
¹H-NMR (300 MHz, CDCl₃): 8.19 (d, 1H), 7.97 (d, 1H), 7.91 (d, 1H), 7.83 (d, 2H), 7.65-7.72 (m, 2H), 7.40-7.51 (m, 3H), 7.31 (td, 1H), 4.81 (d, 2H), 3.04 (hept, 1H), 2.63 (t, 1H), 1.34 (d, 6H).
MS: 425 (M+1)⁺

The starting material (3-fluoro-phenyl)-oxo-acetaldehyde is prepared according to the literature, for example by SeO₂ oxidation of 1-(3-fluoro-phenyl)-ethanone, analogously to Example 1.

### Example 8: (3-Chloro-phenyl)-[4-(4-isopropyl-phenyl)-6-propargytoxy-quinazolin-2-yl]-methanone

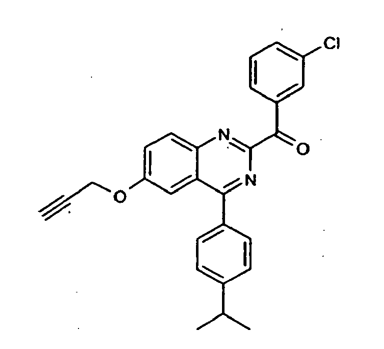

m. p. 128-130 °C
¹H-NMR (300 MHz, CDCl₃): 8.16-8.22 (m, 2H), 8.07 (d, 1H), 7.83 (d, 2H), 7.65-7.72 (m, 2H), 7.68 (d, 1H), 7.40-7.48 (m, 3H), 4.81 (d, 2H), 3.04 (hept, 1H), 2.63 (t, 1H), 1.34 (d, 6H).
MS: 441 (M+1)⁺

The starting material (3-chloro-phenyl)-oxo-acetaldehyde is prepared according to the literature, for example by SeO₂ oxidation of (3-chloro-phenyl)-ethanone, analogously to Example 1.

### Example 9: (4-Chloro-phenyl)-[4-(4-isopropyl-phenyl)-6-propargyloxy-quinazolin-2-yl]-methanone

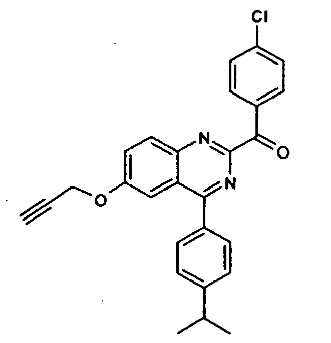

m. p. 108-110 °C
¹H-NMR (300 MHz, CDCl₃): 8.12-8.20 (m, 3H), 7.83 (d, 2H), 7.65-7.72 (m, 2H), 7.40-7.50 (m, 4H), 4.81 (d, 2H), 3.04 (hept, 1H), 2.63 (t, 1H), 1.34 (d, 6H).
MS: 441 (M+1)⁺

The starting material (4-chloro-phenyl)-oxo-acetaldehyde is prepared according to the literature, for example by SeO₂ oxidation of (4-chloro-phenyl)-ethanone, analogously to Example 1.

### Example 10: (4-Fluoro-phenyl)-[4-(4-tert.butyl-phenyl)-6-propargyloxy-quinazolin-2-yl]-methanone

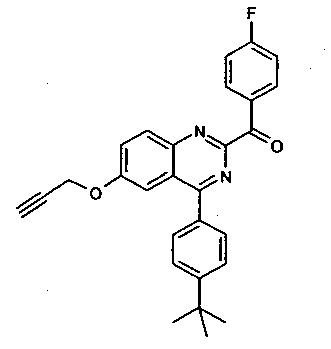

m. p. 105-107 °C
¹H-NMR (300 MHz, CDCl₃): 8.15-8.27 (m, 3H), 7.84 (d, 2H), 7.65-7.72 (m, 2H), 7.59 (d, 2H), 7.17 (t, 2H), 4.81 (d, 2H), 2.63 (t, 1H), 1.41 (s, 9H).
MS: 439 (M+1)⁺

The starting material (4-fluoro-phenyl)-oxo-acetaldehyde is prepared according to the literature, for example by SeO₂ oxidation of (4-fluoro-phenyl)-ethanone, analogously to Example 1.

### Example 11: (3-Fluoro-phenyl)-[4-(4-tert.butyl-phenyl)-6-propargyloxy-quinazolin-2-yi]-methanone

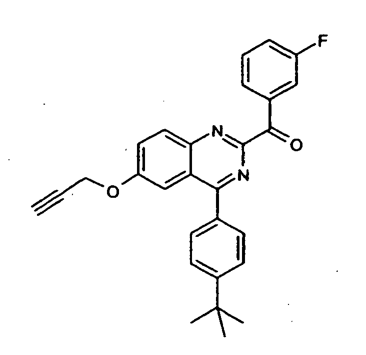

m. p. 156-158 °C
¹H-NMR (300 MHz, CDCl₃): 8.20 (d, 1 H), 7.97 (d, 1H), 7.91 (d, 1H), 7.84 (d, 2H), 7.65-7.73 (m, 2H), 7.59 (d, 2H), 7.33-7.52 (m 1H), 7.31 (td, 1 H), 4.81 (d, 2H), 2.63 (t, 1H), 1.41 (s, 9H).
MS: 439 (M+1)⁺

The starting material (3-fluoro-phenyl)-oxo-acetaldehyde is prepared according to the literature, for example by SeO₂ oxidation of (3-fluoro-phenyl)-ethanone, analogously to Example 1.

### Example 12: (3-Bromo-phenyl)-[4-(4-isopropyl-phenyl)-6-propargyloxy-quinazolin-2-yl]-methanone

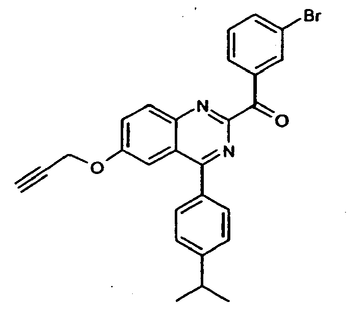

m. p. 132-132 °C
¹H-NMR (300 MHz, CDCl₃): 8.34 (br t, 1H), 8.19 (d, 1H), 8.10 (d, 1H), 7.83 (d, 2H), 7.65-7.76 (m, 3H), 7.43 (d, 2H), 7.37 (t, 1H), 4.81 (d, 2H), 3.03 (hept, 1H), 2.63 (t, 1 H), 1.33 (d, 6H). MS: 485/487 (M+1)⁺

The starting material (3-bromo-phenyl)-oxo-acetaldehyde is prepared according to the literature, for example by SeO₂ oxidation of (3-bromo-phenyl)-ethanone, analogously to Example 1.

### Example 13: (4-Bromo-phenyl)-[4-(4-isopropyl-phenyl)-6-propargyloxy-quinazolin-2-yl]-methanone

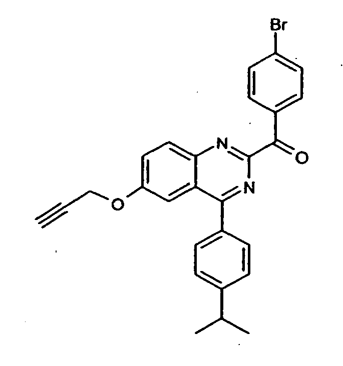

m. p. 97-100 °C
¹H-NMR (300 MHz, CDCl₃): 8.18 (d, 1H), 8.07 (d, 2H), 7.82 (d, 2H), 7.61-7.72 (m, 4H), 7.43 (m, 2H), 4.81 (d, 2H), 3.04 (hept, 1H), 2.63 (t, 1H), 1.34 (d, 6H).
MS: 485/487 (M+1)⁺

The starting material (4-bromo-phenyl)-oxo-acetaldehyde is prepared according to the literature, for example by SeO₂ oxidation of (4-bromo-phenyl)-ethanone, analogously to Example 1.

### Example 14: (4-Methyl-phenyl)-[4-(4-isopropyl-phenyl)-6-propargyloxy-quinazolin-2-yl]-methanone

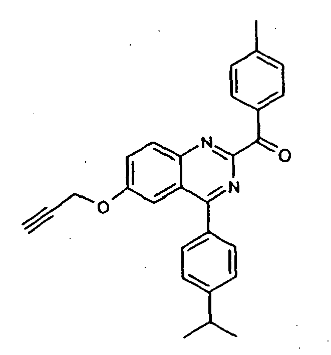

m. p. 130-132 °C
¹H-NMR (300 MHz, CDCl₃): 8.18 (d, 1H), 8.06 (d, 2H), 7.83 (d, 2H), 7.63-7.70 (m, 2H), 7.12 (d, 2H), 7.29 (d, 2H), 4.80 (d, 2H), 3.03 (hept, 1H), 2.60 (t, 1H), 2.44 (s, 3H), 1.33 (d, 6H).
MS: 421 (M+1)⁺

The starting material (4-methyl-phenyl)-oxo-acetaldehyde is prepared according to the literature, for example by SeO₂ oxidation of (4-methyl-phenyl)-ethanone, analogously to Example 1.

### Example 15: (4-Isopropyl-phenyl)-[4-(4-isopropyl-phenyl)-6-propargyloxy-quinazolin-2-yl]-methanone

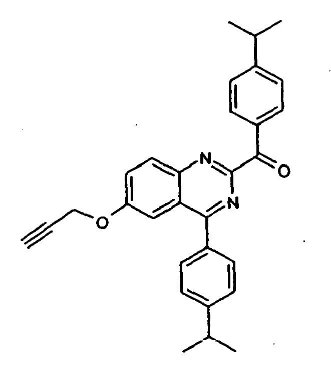

m. p. 132-134 °C
¹H-NMR (300 MHz, CDCl₃): 8.18 (d, 1H), 8.09 (d, 2H), 7.83 (d, 2H), 7.63-7.70 (m, 2H), 7.42 (d, 2H), 7.34 (d, 2H), 4.80 (d, 2H), 3.03 (hept, 1H), 2.99 (hept, 1H), 2.62 (t, 1H), 1.32 (d, 6H), 1.30 (d, 6H).
MS: 449 (M+1)⁺

The starting material (4-isopropyl-phenyl)-oxo-acetaldehyde is prepared according to the literature, for example by SeO₂ oxidation of (4-isopropyl-phenyl)-ethanone, analogously to Example 1.

### Example 16: (4-Ethyl-phenyl)-[4-(4-isopropyl-phenyl)-6-propargyloxy-quinazolin-2-yl]-methanone

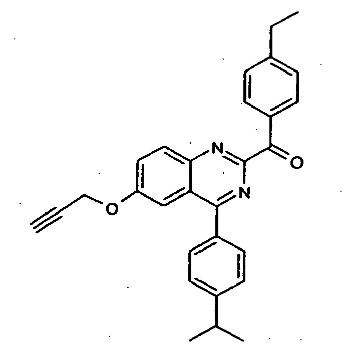

m. p. 108-111 °C
¹H-NMR (300 MHz, CDCl₃): 8.16 (d, 1H), 8.08 (d, 2H), 7.83 (d, 2H), 7.63-7.70 (m, 2H), 7.42 (d, 2H), 7.31 (d, 2H), 4.80 (d, 2H), 3.03 (hept, 1H), 2.74 (q, 2H), 2.62 (t, 1H), 1.33 (d, 6H), 1.28 (t, 3H).
MS: 435 (M+1)⁺

The starting material (4-ethyl-phenyl)-oxo-acetaldehyde is prepared according to the literature, for example by SeO₂ oxidation of (4-ethyl-phenyl)-ethanone, analogously to Example 1.

### Example 16a: (4-Propyl-phenyl)-[4-(4-isopropyl-phenyl)-6-propargyloxy-quinazolin-2-yl]-methanone

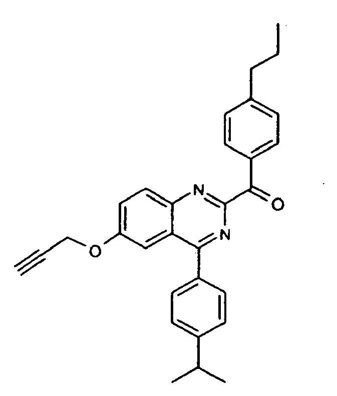

m. p. 140-142 °C
¹H-NMR (300 MHz, CDCl₃): 8.18 (d, 1H), 8.05 (d, 2H), 7.83 (d, 2H), 7.62-7.70 (m, 2H), 7.42 (d, 2H), 7.28 (d, 2H), 4.80 (d, 2H), 3.03 (hept, 1H), 2.67 (t, 2H), 2.63 (t, 1H), 1.68 (m, 2H) 1.32 (d, 6H), 0.97 (t, 3H).
MS: 449 (M+1)⁺

The starting material (4-n-propyl-phenyl)-oxo-acetaldehyde is prepared according to the literature, for example by SeO₂ oxidation of (4-n-propyl-phenyl)-ethanone, analogously to Example 1.

### Example 17: (4-Cyano-phenyl)-[4-(4-isopropyl-phenyl)-6-propargyloxy-quinazolin-2-yl]-methanone

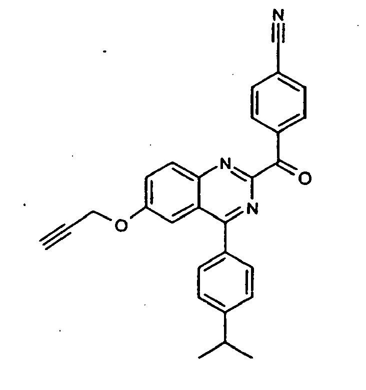

m. p. 130-132 °C
¹H-NMR (300 MHz, CDCl₃): 8.31 (d, 2H), 8.20 (d, 1H), 7.82 (t, 4H), 7.64-7.75 (m, 2H), 7.45 (d, 2H), 4.82 (d, 2H), 3.05 (hept, 1H), 2.62 (broad, 1H), 1.35 (d, 6H).
MS: 432 (M+1)⁺

The starting material (4-cyano-phenyl)-oxo-acetaldehyde is prepared according to the literature, for example by SeO₂ oxidation of (4-cyano-phenyl)-ethanone, analogously to Example 1.

### Example 18: (4-Methylthio-phenyl)-[4-(4-isopropyl-phenyl)-6-propargyloxy-quinazolin-2-yl]-methanone

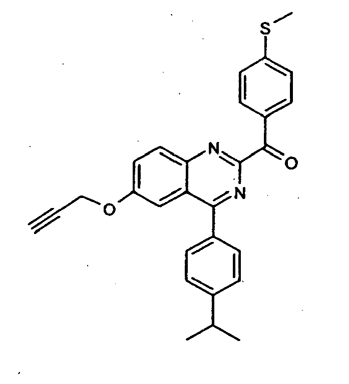

m. p. 161-164 °C
¹H-NMR (300 MHz, CDCl₃): 8.19 (d, 1H), 8.11 (d, 2H), 7.84 (d, 2H), 7.64-7.72 (m, 2H), 7.43 (d, 2H), 7.31 (d, 2H), 4.81 (d, 2H), 3.04 (hept, 1H), 2.63 (t, 1H), 2.55 (s, 3H), 1.34 (d, 6H).
MS: 453 (M+1)⁺

The starting material (4-methylthiophenyl)-oxo-acetaldehyde is prepared according to the literature, for example by SeO₂ oxidation of (4-methylthiophenyl)-ethanone, analogously to Example 1.

### Example 19: (4-Methansulfonyl-phenyl)-[4-(4-isopropyl-phenyl)-6-propargyloxy-quinazolin-2-yl]-methanone

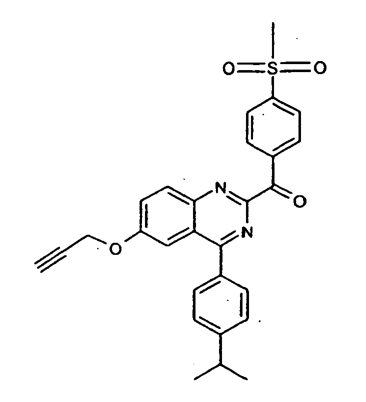

m. p. 181-184 °C
¹H-NMR (300 MHz, CDCl₃): 8.40 (d, 2H), 8.21 (d, 1H), 8.10 (d, 2H), 7.84 (d, 2H), 7.70-7.75 (m, 2H), 7.46 (d, 2H), 4.83 (d, 2H), 3.13 (s, 3H), 3.06 (hept, 1H), 2.65 (broad, 1H), 1.36 (d, 6H).
MS: 485 (M+1)⁺

The starting material (4-methanesulfonyl-phenyl)-oxo-acetaldehyde is prepared according to the literature, for example by SeO₂ oxidation of (4-methanesulfonyl-phenyl)-ethanone, analogously to Example 1.

### Example 20: (4-Dimethylamino-phenyl)-[4-(4-isopropyl-phenyl)-6-propargyloxy-quinazolin-2-yl]-methanone

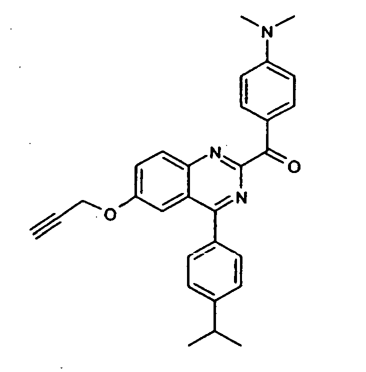

m. p. 148-151 °C
¹H-NMR (300 MHz, CDCl₃): 8.18 (d, 1H), 8.08 (d, 2H), 7.85 (d, 2H), 7.60-7.70 (m, 2H), 7.42 (d, 2H), 6.68 (d, 2H), 4.80 (d, 2H), 3.09 (s, 6H), 3.03 (hept, 1H), 2.63 (t, 1H), 1.34 (d, 6H).
MS: 450 (M+1)⁺

The starting material (4-dimethylamino-phenyl)-oxo-acetaldehyde is prepared according to the literature, for example by SeO₂ oxidation of (4-dimethylamino-phenyl)-ethanone, analogously to Example 1.

### Example 21: (4-Ethoxy-phenyl)-[4-(4-isopropyl-phenyl)-6-propargyloxy-quinazolin-2-yl]-methanone

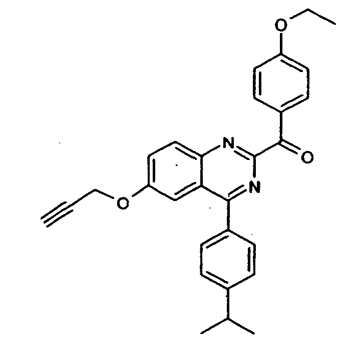

m. p. 117-119 °C
¹H-NMR (300 MHz, CDCl₃): 8.15-8.22 (m, 3H), 7.85 (d, 2H), 7.65-7.73 (m, 2H), 7.45 (d, 2H), 6.97 (d, 2H), 4.82 (br s, 2H), 4.11 (q, 2H), 3.06 (hept, 1H), 2.65 (broad, 1H), 1.48 (t, 3H), 1.36 (d, 6H).
MS: 451 (M+1)⁺

The starting material (4-ethoxy-phenyl)-oxo-acetaldehyde is prepared according to the literature, for example by SeO₂ oxidation of (4-ethoxy-phenyl)-ethanone, analogously to Example 1.

### Example 22: 4-[4-(4-Isopropyl-phenyl)-6-propargyloxy-quinazoline-2-carbonyl]-benzoic acid methyl ester

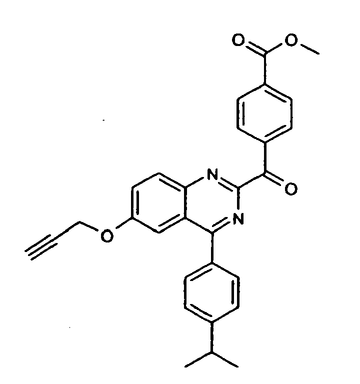

m. p. 108-110 °C
¹H-NMR (300 MHz, CDCl₃): 8.12-8.25 (m, 5H), 7.82 (d, 2H), 7.65-7.72 (m, 2H), 7.42 (d, 2H), 4.81 (d, 2H), 3.96 (s, 3H), 3.03 (hept, 1H), 2.63 (t, 1H), 1.33 (d, 6H).
MS: 465 (M+1)⁺

The appropriate glyoxal starting material is prepared according to the literature, for example by SeO₂ oxidation of 4-(2-oxo-acetyl)-benzoic acid methyl ester, analogously to Example 1.

### Example 23: (4-Dimethylamino-phenyl)-[4-(4-tert.butyl-phenyl)-6-propargyloxy-quinazolin-2-yl]-methanone

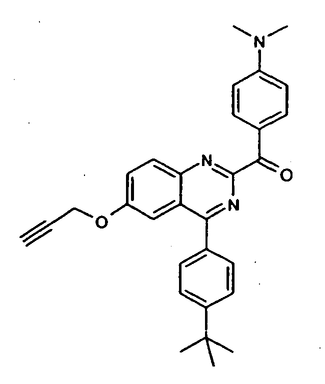

m. p. 150-152 °C
¹H-NMR (300 MHz, CDCl₃): 8.17 (d, 1H), 8.07 (d, 2H), 7.85 (d, 2H), 7.61-7.70 (m, 2H), 7.57 (d, 2H), 6.67 (d, 2H), 4.79 (d, 2H), 3.08 (s, 6H), 2.62 (t, 1H), 1.40 (s, 9H).
MS: 464 (M+1)⁺

The appropriate glyoxal starting material is prepared according to the literature, for example by SeO₂ oxidation of the corresponding ketone, analogously to Example 1.

### Example 24: (4-Dimethylamino-phenyl)-[4-(4-cyclopropyl-phenyl)-6-propargyloxy-quinazolin-2-yl]-methanone

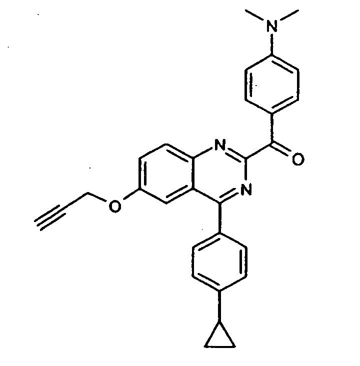

m. p. 169-172 °C
¹H-NMR (300 MHz, CDCl₃): 8.16 (d,1H), 8.07 (d, 2H), 7.80 (d, 2H), 7.60-7.66 (m, 2H), 7.23 (d, 2H), 6.67 (d, 2H), 4.78 (d, 2H), 3.08 (s, 6H), 2.62 (t, 1H), 1.96-206 (m, 1H), 1.04-1.11 (m, 2H), 0.78-0.85 (m, 2H).
MS: 448 (M+1)⁺

### Example 25: 4-[4-(4-Isopropyl-phenyl)-6-propargyloxy-quinazoline-2-carbonyl]-benzoic acid ethyl ester

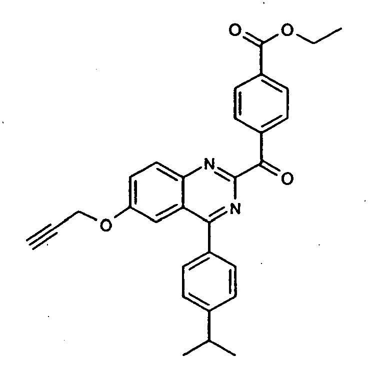

m. p. 132-134 °C
¹H-NMR (300 MHz, CDCl₃): 8.13-8.26 (m, 5H), 7.83 (d, 2H), 7.65-7.72 (m, 2H), 7.41 (d, 2H), 4.81 (d, 2H), 4.42 (q, 2H), 3.04 (hept, 1H), 2.63 (t, 1H), 1.43 (t, 3H), 1.33 (d, 6H).
MS: 479 (M+1)⁺

### Example 26: (4-Methoxy-phenyl)-[4-(4-tert.butyl-phenyl)-6-propargyloxy-quinazolin-2-yl]-methanone

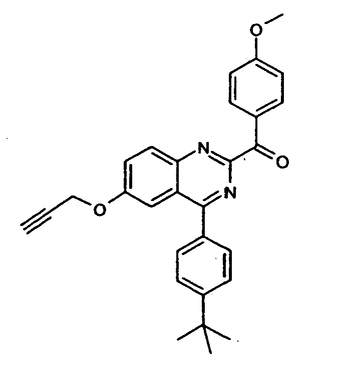

m. p. 150-152 °C
¹H-NMR (300 MHz, CDCl₃): 8.13-8.21 (m, 3H), 7.84 (d, 2H), 7.62-7.71 (m, 2H), 7.57 (d, 2H), 6.96 (d, 2H), 4.80 (d, 2H), 3.89 (s, 3H), 2.62 (broad, 1H), 1.40 (s; 9H).
MS: 451 (M+1)⁺

### Example 27: (4-Ethoxy-phenyl)-[4-(4-cyclopropyl-phenyl)-6-propargyloxy-quinazolin-2-yl]-methanone

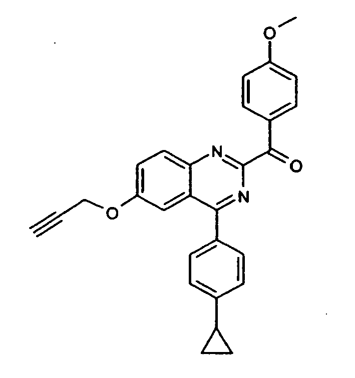

m. p. 147-149 °C
¹H-NMR (300 MHz, CDCl₃): 8.13-8.20 (m, 3H), 7.80 (d, 2H), 7.62-7.68 (m, 2H), 7.24 (d, 2H), 6.96 (d, 2H), 4.78 (d, 2H), 3.89 (s, 3H), 2.62 (broad, 1H), 1.95-2.06 (m, 1H), 1.03-1.13 (m, 2H), 0.77-0.86 (m, 2H).
MS: 435 (M+1)⁺

### Example 28: (3-Ethoxy-4-methoxy-phenyl)-[4-(4-isopropyl-phenyl)-6-propargyloxy-quinazolin-2-yl]-methanone

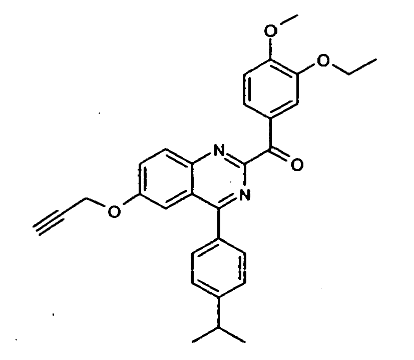

m. p. 148 °C
¹H-NMR (300 MHz, CDCl₃): 8.17 (d, 1H), 7.80-7.86 (m, 3H), 7.63-7.75 (m, 3H), 7.12 (d, 2H), 6.88 (d, 1H), 4.80 (d, 2H), 4.18 (q, 2H), 3.95 (s, 3H), 3.03 (hept, 1 H), 2.62 (t, 1H), 1.50 (t, 3H), 1.33 (d, 6H).
MS: 481 (M+1)⁺

### Preparation of starting material:

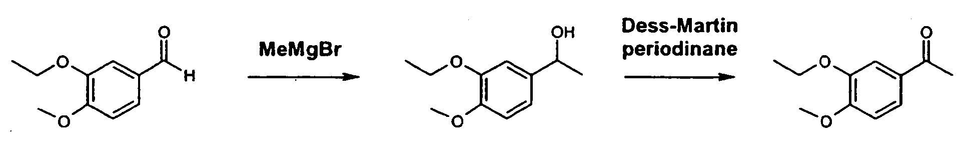

### A) 1-(3-Ethoxy-4-methoxy-phenyl)-ethanol

A solution of 2.0 g (11.1 mmol) 3-ethoxy-4-methoxy-benzaldehyde in 15 ml tetrahydrofurane is slowly trated with 4.4 ml of a etheral 3 M methylmagesiumbromide solution such that the temperature is maintained between -65 and -70 °C. After ca. 15 minutes the cooling bath is removed and the mixture allowed to come to room temperature. The resutling mixture is poured into saturated ammonium chloride solution and the alcohol extracted with diethyl ether. The combined organic layers are washed several times with brine, dried over MgSO₄ and concentrated in vacuao. The crude product is directly used for the following oxidation.
¹H-NMR (300 MHz, CDCl₃): 6.94 (d, 1H), 6.88 (dd, 1H), 6.82 (d, 1H), 4.84 (q, 1H), 4.12 (q, 2H), 3.86 (s, 3H), 1.77 (br, OH), 1.48 (d, 3H), 1.47 (t, 3H).

### B) 1-(3-Ethoxy-4-methoxy-phenyl)-ethanone

The crude product (1.0 g; 5.10 mmol) obtained in step A is dissolved in 30 ml dichloromethane and treated at room temperature with 2.38 g (5.61 mmol) Dess-Martin periodinane. The oxidation is complete after 4 hours. The white suspension is concentrated i.V. and the prduct purified by chromatography (hexane/ethyl acetate).
m. p. 71-72 °C
¹H-NMR (300 MHz, CDCl₃): 7.56 (dd, 1H), 7.51 (d, 1H), 6.88 (d, 1H), 4.16 (q, 2H), 3.94 (s, 3H), 2.56 (s, 3H), 1.49 (t, 3H).

The 1-(3-ethoxy-4-methoxy-phenyl)-ethanone thus obtained is oxidized to the corresponding glyoxal as described in example 1.

### Example 29: (4-tert.Butyloxy-phenyl)-[4-(4-isopropyl-phenyl)-6-propargyloxy-quinazolin-2-yl]-methanone

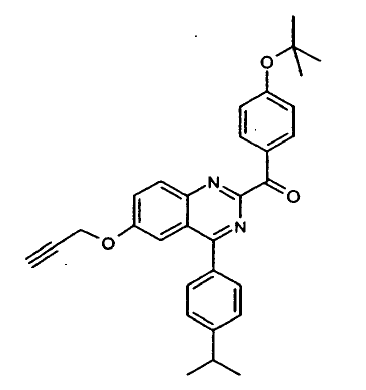

m. p. 130-132 °C
¹H-NMR (300 MHz, CDCl₃): 8.17 (d, 1H), 8.10 (d, 2H), 7.82 (d, 2H), 7.62-7.70 (m, 2H), 7.41 (d, 2H), 7.04 (d, 2H), 4.79 (d, 2H), 3.02 (hept, 1H), 2.62 (t, 1H), 1.49 (s, 9H), 1.32 (d, 6H).
MS: 479 (M+1)⁺

Synthesis of 1-(4-tert.butoxy-phenyl)-ethanone as described for example 29.

### Example 30: (4-Hydroxy)-[4-(4-isopropyl-phenyl)-6-propargyloxy-quinazolin-2-yl]-methanone

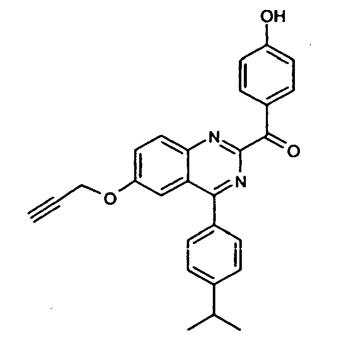

m. p. 185-187 °C
¹H-NMR (300 MHz, CDCl₃): 8.16 (d, 1H), 8.10 (d, 2H), 7.82 (d, 2H), 7.63-7.70 (m, 2H), 7.41 (d, 2H), 6.88 (d, 2H), 5.82 (broad, OH), 4.79 (d, 2H), 3.02 (hept, 1H), 2.62 (t, 1H), 1.32 (d, 6H).
MS: 423 (M+1)⁺

### Example 31: (4-Butyloxy)-[4-(4-isopropyl-phenyl)-6-propargyloxy-quinazolin-2-yl]-methanone

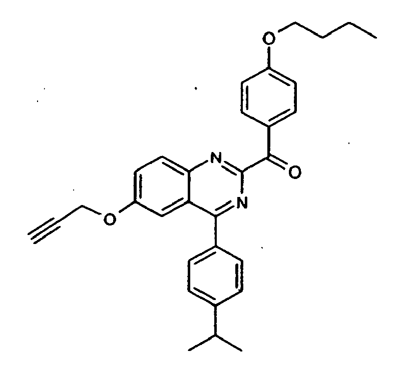

m. p. 91-93 °C
¹H-NMR (300 MHz, CDCl₃): 8.12-8.20 (m, 3H), 7.83 (d, 2H), 7.63-7.70 (m, 2H), 7.42 (d, 2H), 6.94 (d, 2H), 4.81 (d, 2H), 4.05 (t, 2H), 3.03 (hept, 1H), 2.62 (t, 1H), 1.75-1.86 (m, 2H), 1.44-1.55 (m, 2H), 1.33 (d, 6H), 0.99 (t, 3H).
MS: 479 (M+1)⁺

### Example 32: Furan-2-yl-[4-(4-isopropyl-phenyl)-6-propargyloxy-quinazolin-2-yl]-methanone

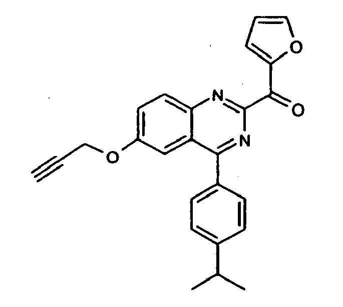

m. p. 150-151 °C
¹H-NMR (300 MHz, CDCl₃): 8.23-8.29 (m, 1H), 8.03 (d, 1H), 7.84 (d, 2H), 7.75-7.78 (m, 1H), 7.64-7.70 (m, 2H), 7.45 (d, 2H), 6.63 (dd, 1H), 4.80 (d, 2H), 3.05 (hept., 1H), 2.62 (t, 1H), 1.35 (d, 6H).
MS: 397 (M+1)⁺

Preparation of furan-2-yl-oxo-acetaldehyde as described in EP 201 221.

### Example 33: Furan-3-yl-[4-(4-tert.butyl-phenyl)-6-propargyloxy-quinazolin-2-yl]-methanone

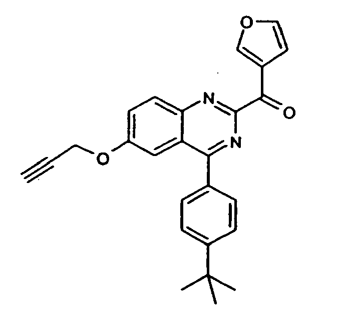

m. p. 170 °C
¹H-NMR (300 MHz, CDCl₃): 8.93-8.95 (m, 1H), 8.24-8.27 (m, 1H), 7.84 (d, 2H), 7.63-7.70 (m, 2H), 7.61 (d, 2H), 7.48 (t, 1H), 7.16 (dd, 1H), 4.81 (d, 2H), 2.62 (t, 1H), 1.43 (s, 9H).
MS: 411 (M+1)⁺

Preparation of 1-furan-3-yl-ethanone as described in EP 230 053, followed by SeO₂ oxidation as described above.

### Example 34: Furan-3-yl-[4-(4-isopropyl-phenyl)-6-propargyloxy-quinazolin-2-yl]-methanone

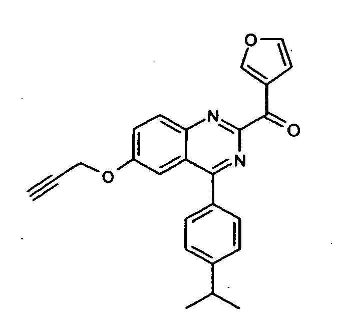

m. p. 133 °C
¹H-NMR (300 MHz, CDCl₃): 8.93-8.95 (m, 1H), 8.24-8.27 (m, 1H), 7.84 (d, 2H), 7.64-7.70 (m, 2H), 7.43-7.49 (m, 3H), 7.15-7.17 (m, 1H), 4.80 (d, 2H), 3.06 (hept., 1H), 2.62 (t, I H), 1.36 (d, 6H).
MS: 397 (M+1)⁺

### Example 35: Thiophen-2-yl-[4-(4-isopropyl-phenyl)-6-propargyloxy-quinazolin-2-yl]-methanone

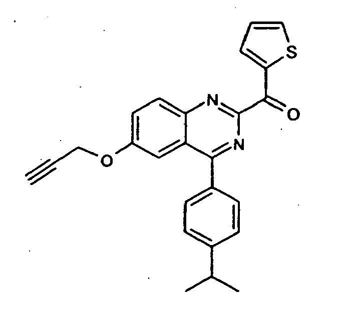

m. p. 113-117 °C
¹H-NMR (300 MHz, CDCl₃): 8.25-8.30 (m, 1H), 7.89 (d, 2H), 7.76 (dd, 2H), 7.65-7.71 (m, 2H), 7.46 (d, 2H), 7.20 (dd, 1H), 4.80 (d, 2H), 3.06 (hept., 1H), 2.63 (t, 1H), 1.35 (d, 6H).
MS: 413 (M+1)⁺

### Example 36: (3-Methyl-thiophen-2-yl-[4-(4-isopropyl-phenyl)-6-propargyloxy-quinazolin-2-yl]-methanone

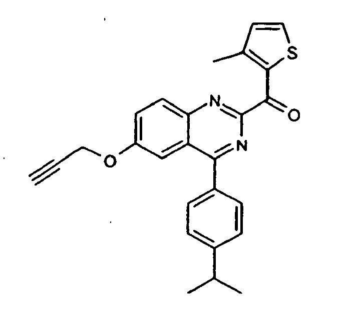

m. p. 130-132 °C
¹H-NMR (300 MHz, CDCl₃): 8.24 (d, 1H), 7.90 (d, 2H), 7.63-7.70 (m, 2H), 7.58 (d, 1H), 7.44 (d, 2H), 7.00 (d, 1H), 4.79 (d, 2H), 3.04 (hept., 1H), 2.71 (s, 3H), 2.61 (t, 1H), 1.34 (d, 6H).
MS: 427 (M+1)⁺

### Example 37: Benz[b]thiophen-2-yl-[4-(4-isopropyl-phenyl)-6-propargyloxy-quinazolin-2-yl]-methanone

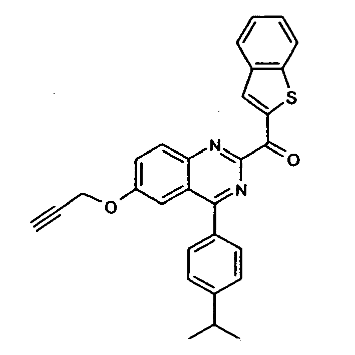

m. p. 167-169 °C
¹H-NMR (300 MHz, CDCl₃): 8.68 (s, 1H), 8.28-8.33 (m, 1H), 7.89-7.96 (m, 4H), 7.68-7.74 (m, 2H), 7.37-7.50 (m, 4H), 4.81 (d, 2H), 3.07 (hept., 1H), 2.64 (t, 1H), 1.37 (d, 6H).
MS: 463 (M+1)⁺

Preparation of benzo[b]thiophen-2-yl-oxo-acetaldehyde as described in EP 201 221.

### Example 38: Thiophen-3-yl-[4-(4-isopropyl-phenyl)-6-propargyloxy-quinazolin-2-yl]-methanone

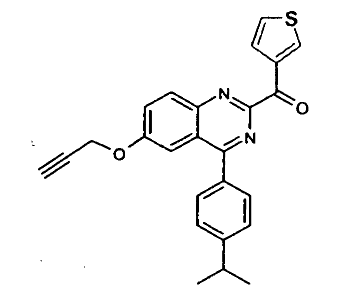

¹H-NMR (300 MHz, CDCl₃): 8.80-8.84 (dd, 1H), 8.20-8.25 (m, 1H), 7.94 (dd, 1H), 7.83 (d, 2H), 7.61-7.70 (m, 2H), 7.44 (d, 2H), 7.34 (dd, 1H), 4.80 (d, 2H), 3.04 (hept., 1H), 2.62 (t, 1H), 1.34 (d, 6H).
MS: 413 (M+1)⁺

### Example 39: (1-Methyl-1H-pyrrol)-2-yl-[4-(4-isopropyl-phenyl)-6-propargyloxy-quinazolin-2-yl]-methanone

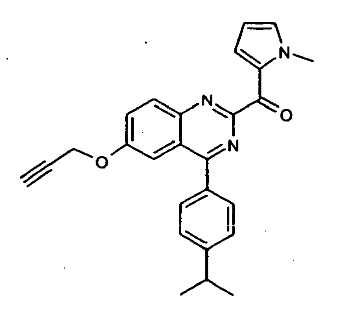

m. p. 126-128 °C
¹H-NMR (300 MHz, CDCl₃): 8.15-8.20 (m, 1H), 7.83 (d, 2H), 7.61-7.67 (m, 2H), 7.42 (d, 2H), 7.28 (dd, 1H), 6.94 (t, 1H), 6.18 (dd, 1H), 4.78 (d, 2H), 3.03 (hept., 1H), 4.12 (s, 3H), 2.61 (t, 1 H), 1.33 (d, 6H).
MS: 410 (M+1)⁺

Preparation of (1-methyl-1H-pyrrol-2-yl)-oxo-acetaldehyde as described in EP 201 221.

### Example 40: 4-(4-Isopropyl-phenyl)-6-propargyloxy-quinazoline-2-carboxylic acid ethyl ester

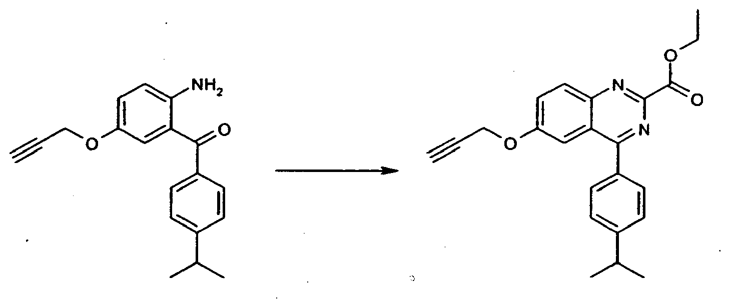

To a mixture of 2 g (6.8 mmol) (2-amino-5-propargyloxy-phenyl)-(4-isopropyl-phenyl)-methanone and 1.6 g ammonium acetate are added 7 ml water and 1.4 g (6.8 mmol) ethyl glyoxylate (50% in toluene). After vigorously stirring in the presence of air for 3 days the reaction mixture is extracted with water and CH₂Cl₂. The organic layers are dried over MgSO₄ and evaporated. Purification by flash chromatography (hexane / ethyl acetate) affords 4-(4-isopropyl-phenyl)-6-propargyloxy-quinazoline-2-carboxylic acid ethyl ester.
¹H-NMR (300 MHz, CDCl₃): 8.30 (d, 1H), 7.83 (d, 2H), 7.67 (dd, 1 H), 7.65 (s, 1 H), 7.43 (d, 2H), 4.79 (d, 2H), 4.60 (q, 2H), 3.04 (hept, 1H), 2.61 (t, 1H), 1.50 (t, 3H), 1.34 (d, 6H)
MS: 375 (M+1)⁺

### Example 41: [4-(4-isopropyl-phenyl)-6-propargyloxy-quinazolin-2-yl]-pyridine-3-yl-methanone

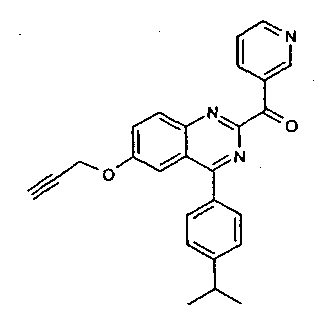

To a solution of 35 mg (0.085 mmol) of [4-(4-isopropyl-phenyl)-6-propargyloxy-quinazolin-2-yl]-pyridine-3-yl-methanol in 2 ml of acetone is added dropwise 49 µl (0.128 mmol) 2.6 M Jones reagent. An exothermic reaction takes place and the mixture turns dark. The oxidation reaction is complete after stirring for two hrs at rt. The chromium salts are filtered off and washed several times with acetone. After concentration i.V. the residue is distributed between ethyl acetate and water. Drying of the organic phase over anhydrous magnesium sulfate and evaporation of the solvent affords a yellow oil, which is purified by chromatography (dichloromethane / methanol). The product is obtained as a yellow solid.
¹H-NMR (400 MHz, CDCl₃):9.46 (d, 1H), 8.82 (dd, 1H), 8.52-8.56 (m, 1H), 8.22 (dd, 1H), 7.84 (d, 2H), 7.68-7.71 (m, 2H), 7.42-7.49 (m, 3H), 4.81 (d, 2H), 3.03 (hept., 1H), 2.63 (t, 1H), 1.33 (d, 6H).
MS: 408 (M+1)⁺

### Preparation of the starting material:

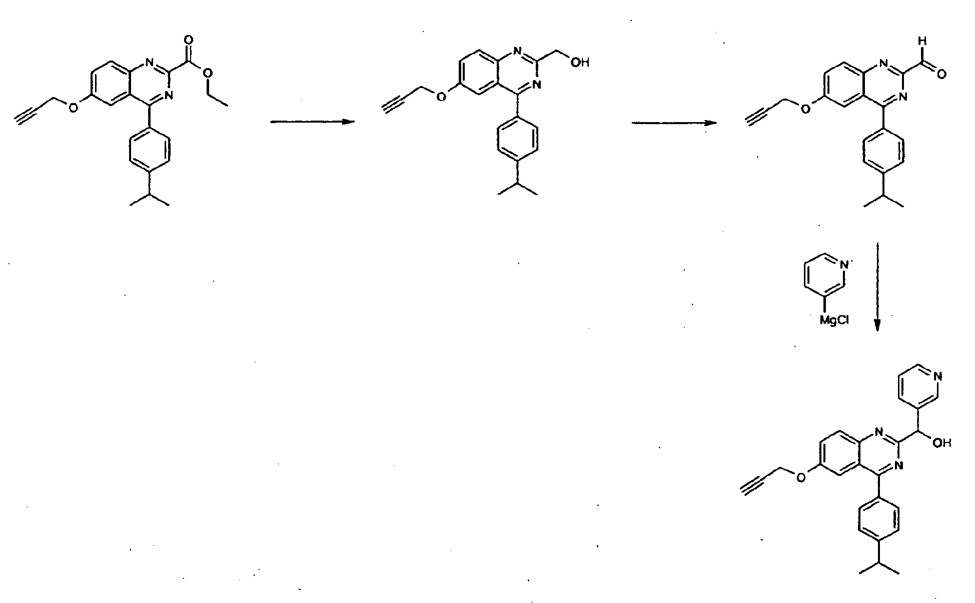

### A) [4-(4-Isopropyl-phenyl)-6-propargyloxy-quinazoline-2-yl-]methanol

A solution of 1.0 g (2.67 mmol) of 4-(4-isopropyl-phenyl)-6-propargyloxy-quinazoline-2-carboxylic acid ethyl ester in 20 ml THF is cooled with a water/ice bath and treated with 1.6 ml 1M lithium aluminum hydride solution. After complete addition the reaction mixture is quenched by pouring it into a saturated ammonium chloride / ethyl acetate solution. Extraction and concentration i.V. yields the product in the form of a yellow oil. The crude material is directly used in the following oxidation step.

### B) 4-(4-Isopropyl-phenyl)-6-propargyloxy-quinazoline-2-carbaldehyde

A solution of 3.9 g (11.7 mmol) of the alcohol prepared in step A in 40 ml dichloromethane is oxidized at rt with 1.1 eq Dess-Martin reagent. The mixture is filtered after stirring for 3 hrs. Distribution between ethyl acetate, water and sodium thiosulfate solution affords after concentration of the organic phases the crude aldehyde. This is purified by recrystallization from a mixture of ethyl acetate / hexanes to give a yellow-brown solid.
¹H-NMR (400 MHz, CDCl₃):10.29 (s, 1H), 8.25 (d, 1H), 7.82 (d, 2H), 7.67-7.72 (m, 2H), 7.45 (d, 2H), 4.80 (d, 2H), 3.04 (hept., 1H), 2.62 (t, 1H), 1.33 (d, 6H)

### C) [4-(4-Isopropyl-phenyl)-6-propargyloxy-quinazolin-2-yl]-pyridine-3-yl-methanol

To a solution of 0.5 ml 2 M isopropyl magnesium chloride in THF is added dropwise 3-bromo-pyridine (80 mg in 0.5 ml THF) at 0 °C. After the addition stirring is continued for another 30 minutes at rt, then the reaction mixture is cooled to -70 °C and the aldehyde obtained in step B is added (120 mg in 2 ml THF). The cooling bath is removed and the mixture is warmed to rt. Extraction with dichloromethane / water affords a yellow oil which is purified by chromatography (dichloromethane / methanol).
¹H-NMR (400 MHz, CDCl₃): 8.93 (d, 1H), 8.50 (dd, 1H), 8.03 (d, 1H), 7.93 (dd, 1H), 7.70-7.75 (m, 2H), 7.60-7.64 (m, 2H), 7.42 (d, 2H), 7.20-7.25 (m, 1H), 6.08 (d, 1H), 5.34 (d. 1H), 4.74-4.75 (m, 2H), 3.03 (hept., 1H), 2.58 (t, 1H), 1.34 (d, 6H)

The compounds of the following examples are prepared in an analogous manner using the appropriate starting materials:

### Example 42: [4-(4-Isopropyl-phenyl)-6-propargyloxy-quinazolin-2-yl]-naphthalen-1-yl-methanone

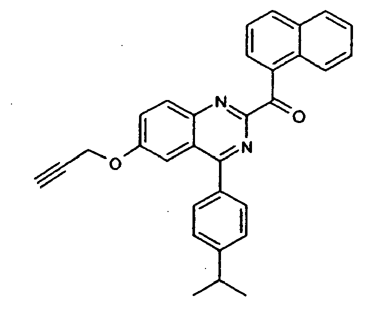

¹H-NMR (400 MHz, CDCl₃): 8.62 (d, 1H), 8.16 (d, 1H), 8.05 (d, 1H), 7.90-7.94 (m, 1H), 7.86 (dd, 1H), 7.76-7.80 (m, 2H), 7.69 (d, 1H), 7.65 (dd, 1H), 7.48-7.60 (m, 3H), 7.39 (d, 2H), 4.79 (d, 2H), 3.00 (hept., 1H), 2.62 (t, 1H), 1.30 (d, 6H).
MS: 457 (M+1)⁺

### Example 43: [4-(4-Isopropyl-phenyl)-6-propargyloxy-naphathalen-2-yl]-methanone

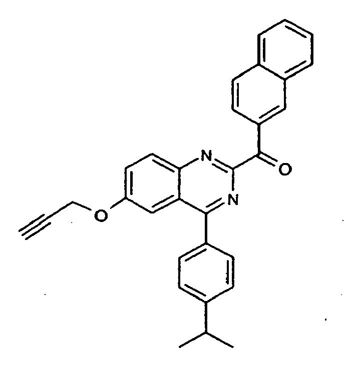

¹H-NMR (400 MHz, CDCl₃): 8.68 ( br s, 1H), 8.24 (dd, 1H), 8.21 (d, 1H), 7.84-7.97 (m, 5H), 7.73 (d, 1H), 7.69 (dd, 1H), 7.59-7.63 (m, 1H), 7.51-7.56 (m, 1H), 7.43 (d, 2H), 4.81 (d, 2H), 3.02 (hept., 1H), 2.63 (t, 1H), 1.32 (d, 6H).
MS: 457 (M+1)⁺

### Example 44: Benzothiazol-2-yl-[4-(4-isopropyl-phenyl)-6-propargyloxy-quinazolin-2-yl]-methanone

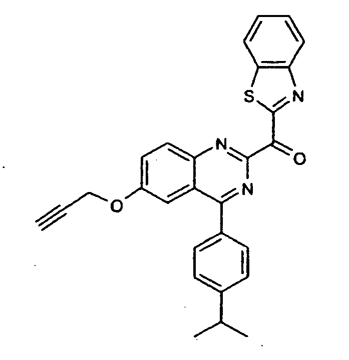

¹H-NMR (400 MHz, CDCl₃): 8.30-8.33 (m, 2H), 8.02-8.06 (m, 1H), 7.89-7.93 (m, 2H), 7.70-7.74 (m, 2H), 7.53-7.62 (m, 2H), 7.45-7.49 (m, 2H), 4.82 (d, 2H), 3.05 (hept., 1H), 2.63 (t, 1H), 1.35 (d, 6H).
MS: 457 (M+1)⁺

### Example 45: [4-(4-Isopropyl-phenyl)-6-propargyloxy-quinazolin-2-yl]-thiazol-5-yl-methanone

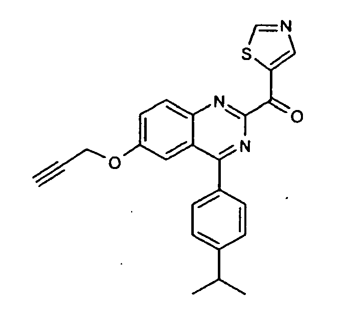

A mixture of 28.3 mg (0.18 mmol) 5-trimethylsilanyl-thiazole (preparation cf. J. Org. Chem. 1988, 53, 1748), 119 mg (0.36 mmol) 4-(4-isopropyl-phenyl)-6-propargyloxy-quinazoline-2-carbaldehyde and 27.3 mg (0.18 mmol) cesium fluoride in 2 ml THF is stirred for 3 days at 60 °C. After evaporation the dark residue is chromatographed (hexane / ethyl acetate). The yellow oil obtained (alcohol) slowly tronsforms into the desired ketone on standing, which is obtained pure after another chromatographic purification.
¹H-NMR (400 MHz, CDCl₃): 9.26 (s, 1H), 9.11 (s, 1H), 8.35 (d, 1H), 7.94 (d, 2H), 7.74-7.81 (m, 2H), 7.53 (d, 2H), 4.84 (d, 2H), 3.10 (hept., 1H), 2.68 (t, 1H), 1.39 (d, 6H).
MS: 414 (M+1)⁺

### Example 46: [4-(4-Isopropyl-phenyl)-6-propargyloxy-quinazolin-2-yl]-piperidin-1-yl-methanone

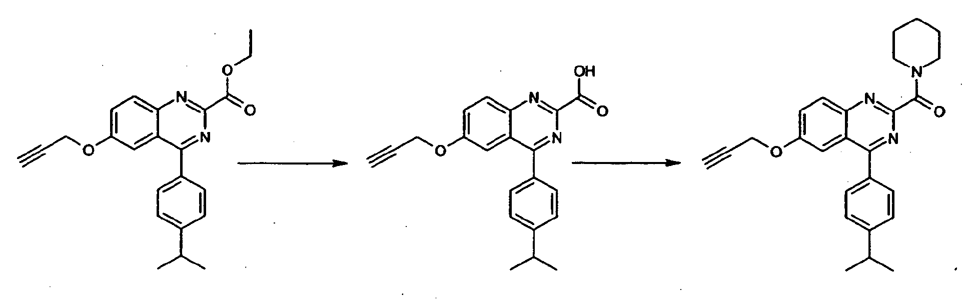

### A) 4-(4-lsopropyl-phenyl)-6-propargyloxy-quinazoline-2-carboxylic acid:

A solution of 1.7 g (4.5 mmol) 4-(4-isopropyl-phenyl)-6-propargyloxy-quinazoline-2-carboxylic acid ethyl ester in 50 ml ethanol is treated at RT with 15 ml aqueous 1 M NaOH. After 1.5 h the reaction mixture is acidified with 1 M hydrochloric acid and extracted with CH₂Cl₂. The organic layers are dried over MgSO₄ and evaporated. The free acid is obtained after chromatography (hexane / ethyl acetate).
¹H-NMR (300 MHz, CDCl₃): 8.28 (d, 1H), 7.81 (d, 2H), 7.73 (dd, 1H), 7.71 (s, 1H), 7.47 (d, 2H), 4.81 (d, 2H), 3.06 (hept, 1H), 2.63 (t, 1H), 1.36 (d, 6H).

### B) [4-(4-Isopropyl-phenyl)-6-propargyloxy-quinazolin-2-yl]-piperidin-1-yl-methanone:

A solution of 22 mg (64 µmol) of the acid prepared above, 6.3 µl (64 µmol) piperidine, 43 mg (96 µmol) BOP, and 16 µl (96 µmmol) Hünig's base in 0.5 ml THF is stirred overnight. The reaction mixture is acidified with 1 M hydrochloric acid and extracted with CH₂Cl₂. After drying over MgSO₄ and evaporation of the solvent the crude product is purified by preparative reversed phase HPLC.
¹H-NMR (300 MHz, DMSO-d₆): 8.04 (d, 1H), 7.80 (d, 2H), 7.74 (dd, 1 H), 7.61 (d, 1H), 7.49 (d, 2H), 4.94 (d, 2H), 3.74 (t, 1H), 3.63 (m, 2H), 3.17 (m, 2H), 3.02 (hept, 1 H), 1.60 (m, 4H), 1.47 (m, 2H), 1.28 (d, 6H).
MS: 414 (M+1)⁺

The compounds of the following examples are prepared in an analogous manner using the appropriate starting materials:

### Example 47: 4-(4-Isopropyl-phenyl)-6-prop-2-ynyloxy-quinazoline-2-carboxylic acid (3-chlorophenyl)-amide

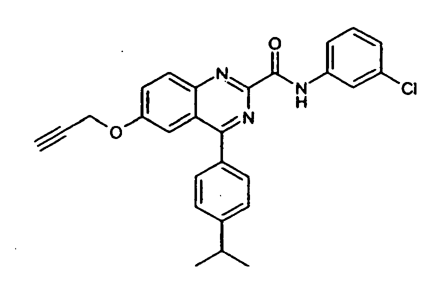

¹H-NMR (400 MHz, CDCl₃): 10.28 (s, 1 H), 8.31 (d, 1 H), 7.88 (s, 1 H), 7.84 (d, 2H), 7.78 (d, 1H), 7.71 - 7.67 (m, 2H), 7.49 (d, 2H), 7.32 (t, 1 H), 7.14 (d, 1H), 4.80 (d, 2H), 3.07 (hept, 1 H), 2.62 (t, 1 H), 1.37 (d, 6H).
MS: 456 (M+1)⁺ (isotope pattern for 1 Cl)

### Example 48: 4-(4-Isopropyl-phenyl)-6-prop-2-ynyloxy-quinazoline-2-carboxylic acid (3-methoxy-phenyl)-amide

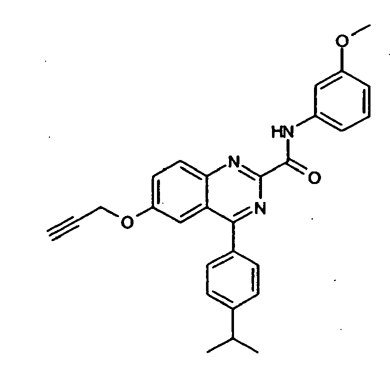

¹H-NMR (300 MHz, DMSO d₆): 10.66 (s, 1H), 8.23 (d, 1H), 7.95 (d, 2H), 7.84 (dd, 1H), 7.70 (d, 1 H), 7.57 (m, 1 H), 7.54 (d, 2H), 7.49 (dd, 1H), 7.29 (t, 1H), 6.73 (dd, 1H), 5.00 (d, 2H), 3.79 (t, 1 H), 3.78 (s, 3H), 3.06 (hept, 1H), 1.32 (d, 6H).
MS: 452 (M+1)⁺

### Example 49: 4-(4-Isopropyl-phenyl)-6-prop-2-ynyloxy-3,4-dihydro-quinazoline-2-carboxylic acid (3-methylsulfanyl-phenyl)-amide

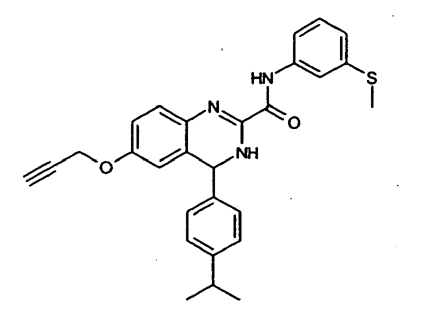

¹H-NMR (300 MHz, CDCl₃): 10.24 (s, 1H), 8.31 (d, 1 H), 7.90 (t, 1 H), 7.85 (d, 2H), 7.72 - 7.67 (m, 2H), 7.56 (dd, 1 H), 7.50 (d, 2H), 7.30 (t, 1H), 7.06 (dd, 1H), 4.81 (d, 2H), 3.09 (hept, 1H), 2.64 (t, 1H), 2.55 (s, 3H), 1.39 (d, 6H).
MS: 470 (M+1)⁺

### Example 50: 4-(4-Isopropyl-phenyl)-6-prop-2-ynyloxy-3,4-dihydro-quinazoline-2-carboxylic acid (3-methanesulfonyl-phenyl)-amide

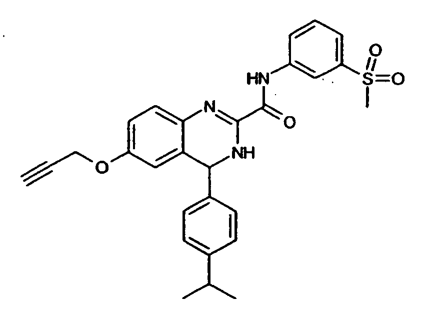

¹H-NMR (300 MHz, CDCl₃): 10.47 (s, 1H), 8.48 (d, broad, 1H), 8.32 (d, 1 H), 8.16 (t, 1H), 7.85 (d, 2H), 7.75 - 7.61 (m, 4H), 7.51 (d, 2H), 4.81 (d, 2H), 3.12 (s, 3H), 3.10 (hept, 1H), 2.65 (t, 1H), 1.40 (d, 6H).
MS: 502 (M+1)⁺

### Example 51: 4-(4-Isopropyl-phenyl)-6-prop-2-ynyloxy-quinazoline-2-carboxylic acid (3-trifluoromethylsulfanyl-phenyl)-amide

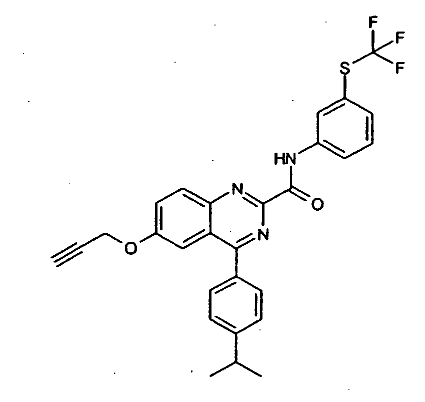

¹H-NMR (400 MHz, CDCl₃): 10.34 (s, 1H), 8.34 (d, 1H), 8.16 (dt, 1H), 8.05 (m, 1H), 7.86 (d, 2H), 7.72 (dd, 1H), 7.69 (d, 1 H), 7.51 (d, 2H), 7.48 - 7.45 (m, 2H), 4.81 (d, 2H), 3.09 (hept, 1 H), 2.64 (t, 1 H), 1.39 (d, 6H).
MS: 522 (M+1)⁺

### Example 52: 4-(4-Isopropyl-phenyl)-6-prop-2-ynyloxy-3,4-dihydro-quinazoline-2-carboxylic acid (3-sulfamoyl-phenyl)-amide

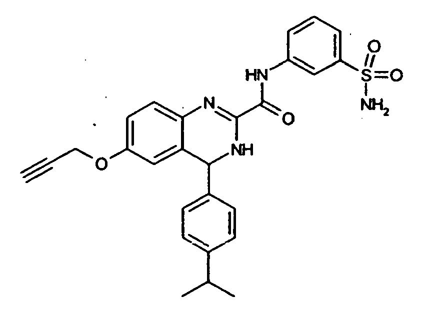

¹H-NMR (400 MHz, DMSO d₆): 8.49 (s, 1H), 8.25 (d, 1H), 8.08 (dt, 1H), 7.98 (d, 2H), 7.85 (dd, 1H), 7.72 (d, 1H), 7.62 - 7.58 (m, 2H), 7.56 (d, 2H), 5.01 (d, 2H), 3.80 (t, 1H), 3.06 hept, 1H), 1.32 (d, 6H).
MS: 503 (M+1)⁺

### Example 53: 4-(4-Isopropyl-phenyl)-6-prop-2-ynyloxy-3,4-dihydro-quinazoline-2-carboxylic acid [3-(2-hydroxy-ethanesulfonyl)-phenyl]-amide

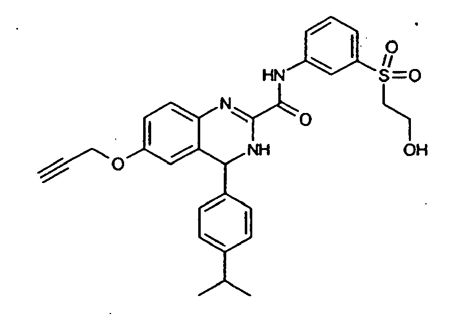

¹H-NMR (300 MHz, CDCl₃): 10.47 (s, 1H), 8.42 (d, broad, 1H), 8.33 (d, 1H), 8.21 (t, 1H), 7.85 (d, 2H), 7.75 - 7.62 (m, 4H), 7.52 (d, 2H), 4.82 (d, 2H), 4.07 (m, 1H), 3.43 (m, 1H), 3.10 (hept, 1H), 2.65 (t, 1H), 1.40 (d, 6H).
MS: 532 (M+1)⁺

### Example 54: 4-(4-Isopropyl-phenyl)-6-prop-2-ynyloxy-3,4-dihydro-quinazoline-2-carboxylic acid (5-ethanesulfonyl-2-hydroxy-phenyl)-amide

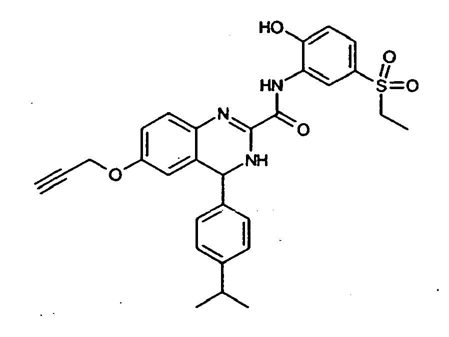

¹H-NMR (400 MHz, CDCl₃): 10.60 (s, 1H), 8.34 (d, 1H), 7.82 (d, 2H), 7.76 - 7.73 m, 2H), 7.71 (d, 1H), 7.67 (dd, 1H), 7.50 (d, 2H), 7.23 (d, H), 4.81 (d, 2H), 3.11 (q, 2H), 3.07 (hept, 1H), 2.63 (t, 1H), 1.37 (d, 6H), 1.28 (t, 3H).
MS: 532 (M+1)⁺

### Example 55: 4-(4-Isopropyl-phenyl)-6-prop-2-ynyloxy-quinazoline-2-carboxylic acid (3-nitrophenyl)-amide

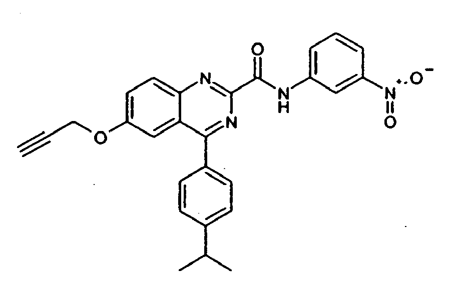

¹H-NMR (400 MHz, CDCl₃): 10.49 (s, 1 H), 8.54 (t, 1 H), 8.45 (d, 1H), 8.33 (d, 1 H), 8.02 (dd, 1H), 7.85 (d, 2H), 7.70 (d, 1 H), 7.73 (dd, 1H), 7.59 (t, 1H), 7.51 (d, 2H), 4.81 (d, 2H), 3.09 (hept, 1H), 2.63 (t, 1H), 1.38 (d, 6H).
MS: 467 (M+1)⁺

### Example 56: 4-(4-Isopropyl-phenyl)-6-prop-2-ynyloxy-quinazoline-2-carboxylic acid (3-cyanophenyl)-amide

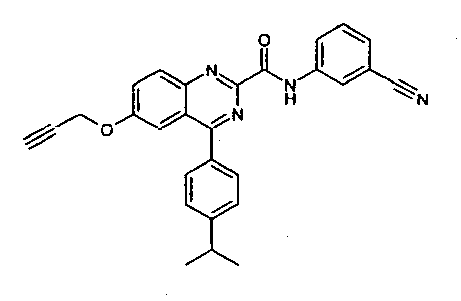

¹H-NMR (400 MHz, CDCl₃): 10.40 (s, 1H), 8.33 (d, 1H), 8.17 (m, 1H), 8.14 (m, 1H), 7.84 (d, 2H), 7.72 (dd, 1H), 7.69 (d, 1H), 7.53 - 7.49 (m, 3H), 7.45 (dt, 1H), 4.81 (d, 2H), 3.08 (hept, 1 H), 2.63 (t, 1 H), 1.38 (d, 6H).
MS: 447 (M+1)⁺

### Example 57: 3-{[4-(4-Isopropyl-phenyl)-6-prop-2-ynyloxy-quinazoline-2-carbonyl]-amino}-benzoic acid methyl ester

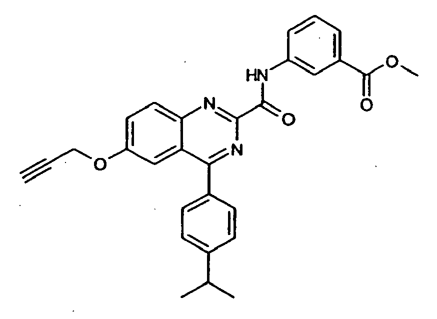

¹H-NMR (300 MHz, CDCl₃): 10.37 (s, 1 H), 8.41 (dd, 1H), 8.32 (d, 1H), 8.21 (t, 1 H), 7.86 (d, 2H), 7.83 (d, 1H), 7.72 - 7.68 (m, 2H), 7.51 (t, 1H), 7.51 (d, 2H), 4.81 (d, 2H), 3.96 (s, 3H), 3.10 (hept, 1H), 2.64 (t, 1H), 1.40 (d, 6H).
MS: 480 (M+1)⁺

### Example 58: 3-{[4-(4-Isopropyl-phenyt)-6-prop-2-ynyloxy-quinazoline-2-carbonyl]-amino}-benzoic acid ethyl ester

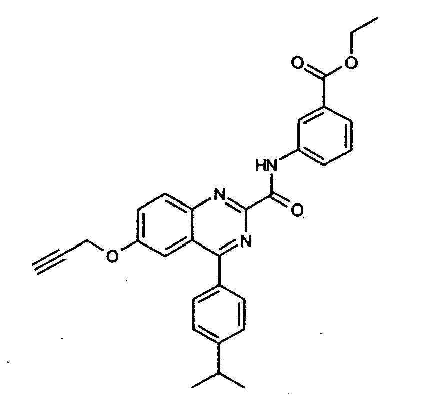

¹H-NMR (400 MHz, CDCl₃): 10.36 (s, 1H), 8.36 (dd, 1H), 8.28 (d, 1H), 8.22 (t, 1 H), 7.87 - 7.84 (m, 1H), 7.83 (d, 2H), 7.72 (dd, 1 H), 7.68 (d, 1 H), 7.50 (t, 1H), 7.49 (d, 2H), 4.80 (d, 2H), 4.41 (q, 2H), 3.08 (hept, 1H), 2.62 (t, 1H), 1.42 (t, 3H), 1.37 (d, 6H).
MS: 494 (M+1)⁺

### Example 59: 3-{[4-(4-Isopropyl-phenyl)-6-prop-2-ynyloxy-quinazoline-2-carbonyl]-amino}-benzoic acid isopropyl ester

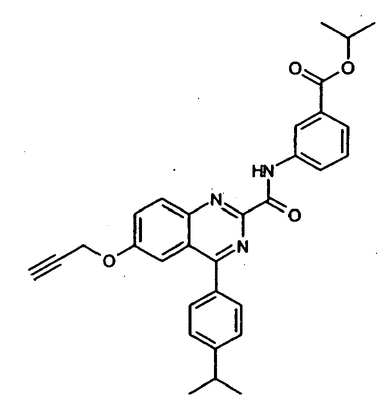

¹H-NMR (400 MHz, CDCl₃): 10.35 (s, 1H), 8.34 (ddd, 1H), 8.27 (d, 1H), 8.22 (t, 1H), 7.85 (dt, 1 H), 7.83 (d, 2H), 7.71 (dd, 1 H), 7.68 (d, 1H), 7.50 (t, 1H), 7.49 (d, 2H), 5.28 (hept, 1 H), 4.80 (d, 2H), 3.08 (hept, 1 H), 2.63 (t, 1H), 1.39 (d, 6H), 1.37 (d, 6H).
MS: 508 (M+1)⁺

### Example 60: 3-{[4-(4-Isopropyl-phenyl)-6-prop-2-ynyloxy-quinazoline-2-carbonyl]-amino}-benzoic acid tert-butyl ester

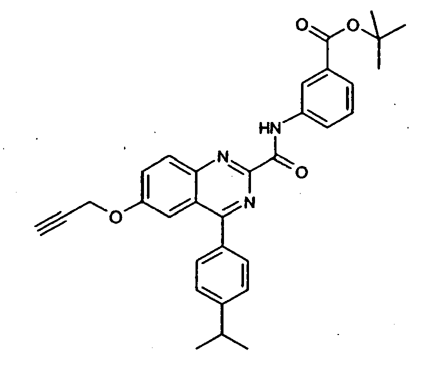

¹H-NMR (400 MHz, DMSO d₆): 10.94 (s, 1H), 8.50 (t, 1H), 8.24 (d, 1 H), 8.13 (d, broad, 1H), 7.97 (d, 2H), 7.85 (dd, 1H), 7.71 (d, 1H), 7.67 (d, 1H), 7.55 (d, 2H), 7.52 (t, 1H), 5.01 (d, 2H), 3.80 (t, 1H), 3.06 (hept, 1 H), 1.57 (s, 9H), 1.31 (d, 6H).
MS: 522 (M+1)⁺

### Example 61: 4-(4-Isopropyl-phenyl)-6-prop-2-ynyioxy-quinazoline-2-carboxylic acid (3-carbamoyl-phenyl)-amide

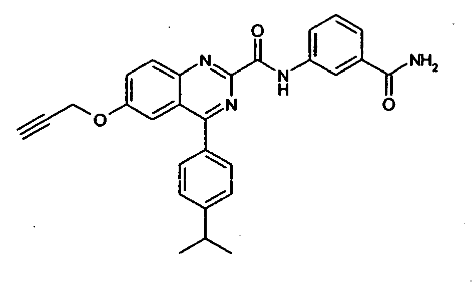

¹H-NMR (400 MHz, CDCl₃): 10.41 (s, 1H), 8.31 (d, 1H), 8.26 (t, 1H), 8.15 (d, 1H), 7.84 (d, 2H), 7.70 (dd, 1H), 7.68 (d, 1H), 7.62 (d, 1H), 7.50 (t, 1H), 7.49 (d, 2H), 6.32 (broad, 1H), 5.69 (broad, 1 H), 4.80 (d, 2H), 3.08 (hept, 1 H), 2.63 (t, 1 H), 1.38 (d, 6H).
MS: 465 (M+1)⁺

### Example 62: 4-(4-Isopropyl-phenyl)-6-prop-2-ynyloxy-quinazoline-2-carboxylic acid (3-acetylphenyl)-amide

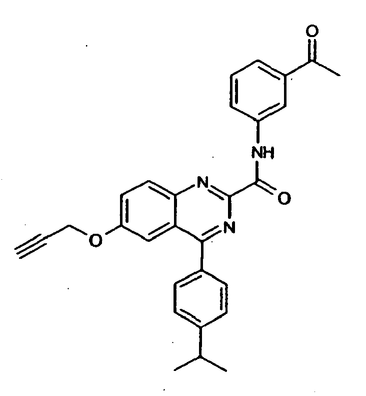

¹H-NMR (400 MHz, CDCl₃): 10.38 (s, 1 H), 8.30 - 8.26 (m, 3H), 7.84 (d, 2H), 7.76 (dt, 1H), 7.71 (dd, 1H), 7.68 (d, 1H), 7.52 (t, 1 H), 7.50 (d, 2H), 4.80 (d, 2H), 3.08 (hept, 1H), 2.66 (s, 3H), 2.62 (t, 1H), 1.38 (d, 6H).
MS: 464 (M+1)⁺

### Example 63: 3-{[4-(4-Isopropyl-phenyl)-6-prop-2-ynyloxy-quinazoline-2-carbonyl]-amino}-5-methoxy-benzoic acid methyl ester

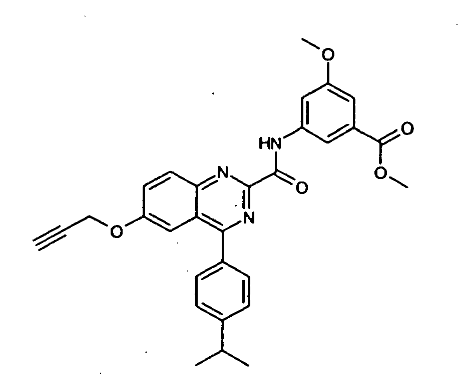

¹H-NMR (400 MHz, CDCl₃): 10.34 (s, 1H), 8.31 (d, 1H), 8.18 (s, 1H), 7.85 (d, 2H), 7.71 - 7.68 (m, 3H), 7.49 (d, 2H), 7.37 (m, 1 H), 4.80 (d, 2H), 3.93 (s, 3H), 3.90 (s, 3H), 3.08 (hept, 1 H), 2.62 (t, 1H), 1.38 (d, 6H).
MS: 510 (M+1)⁺

### Example 64: 4-(4-Isopropyl-phenyl)-6-prop-2-ynyloxy-quinazoline-2-carboxylic acid (3-methylcarbamoyl-phenyl)-amide

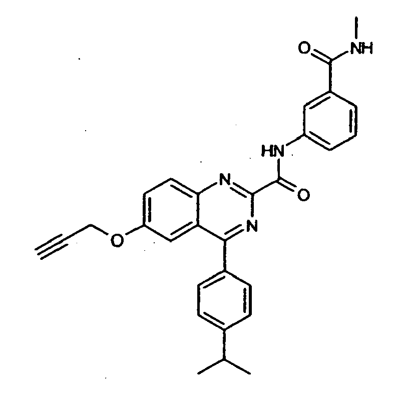

¹H-NMR (400 MHz, CDCl₃): 10.37 (s, 1H), 8.27 (d, 1H), 8.24 (s, 1H), 8.01 (d, 1H), 7.83 (d, 2H), 7.70 (dd, 1H), 7.68 (m, 1H), 7.57 (d, 1H), 7.48 (d, 2H), 7.44 (t, 1H), 6.62 (broad, 1H), 4.80 (d, 2H), 3.07 (hept, 1H), 3.05 (d, 3H), 2.63 (t, 1H), 1.37 (d, 6H).
MS: 479 (M+1)⁺

### Example 65: 4-(4-Isopropyl-phenyl)-6-prop-2-ynyloxy-quinazoline-2-carboxylic acid (3-tert-butylcarbamoyl-phenyl)-amide

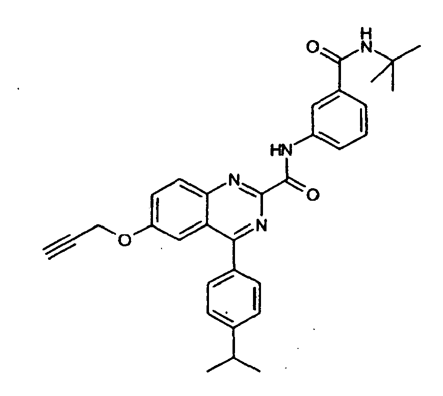

¹H-NMR (400 MHz, CDCl₃):1 0.37 (s, 1H), 8.29 (d, 1H), 8.20 (t, 1H), 8.04 (d, broad, 1H), 7.83 (d, 2H), 7.70 (dd, 1H), 7.68 (d, 1H), 7.53 (d, broad, 1H), 7.49 (d, 2H), 7.45 (t, 1H), 6.14 (s, 1H), 4.80 (d, 2H), 3.07 (hept, 1 H), 2.62 (t, 1H), 1.48 (s, 9H), 1.37 (d, 6H).
MS: 521 (M+1)⁺

### Example 66: 4-(4-Isopropyl-phenyl)-6-prop-2-ynyloxy-quinazoline-2-carboxylic acid (3-dimethylcarbamoyl-5-trifluoromethyl-phenyl)-amide

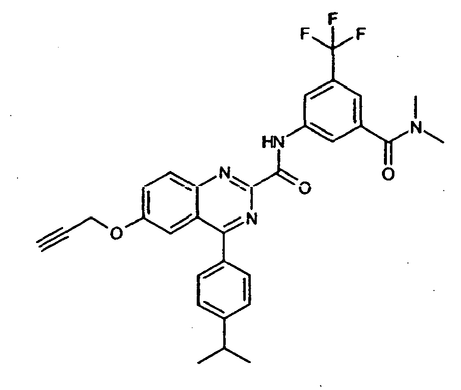

¹H-NMR (400 MHz, CDCl₃): 10.48 (s, 1H), 8.33 (d, 1H), 8.26 (s, 1H), 8.10 (s, 1H), 7.86 (d, 2H), 7.74 (dd, 1 H), 7.71 (d, 1H), 7.53 (s, 1H), 7.51 (d, 2H), 4.82 (d, 2H), 3.17 (s, 3H), 3.11 (s, 3H), 3.09 (hept, 1H), 2.64 (t, 1H), 1.38 (d, 6H).
MS: 561 (M+1)⁺

### Example 67: 3-{[4-(4-Isopropyl-phenyl)-6-prop-2-ynyloxy-quinazoline-2-carbonyl]-amino}-5-trifluoromethyl-benzoic acid methyl ester

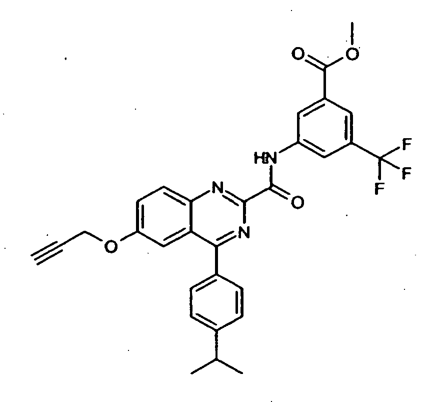

¹H-NMR (400 MHz, DMSO d₆):11.33 (s, 1H), 8.92 (s, 1 H), 8.68 (s, 1H), 8.27 (d, 1H), 7.99 - 7.97 (m, 3H), 7.87 (dd, 1H), 7.73 (d, 1H), 7.57 (d, 2H), 5.02 (d, 2H), 3.95 (s, 3H), 3.81 (t, 1H), 3.08 (hept, 1H), 1.33 (d, 6H).
MS: 548 (M+1)⁺

### Example 68: 3-{[4-(4-Isopropyl-phenyl)-6-prop-2-ynyloxy-quinazoline-2-carbonyl]-amino}-5-trifluoromethyl-benzoic acid isopropyl ester

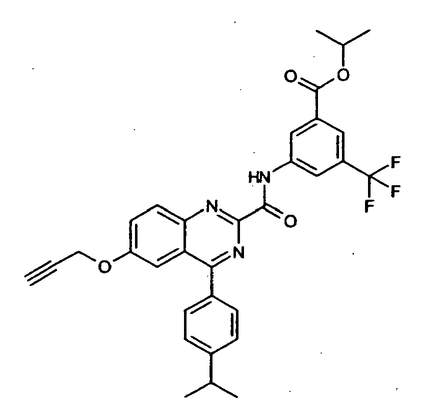

¹H-NMR (400 MHz, CDCl₃): 10.45 (s, 1H), 8.52 (m, 2H), 8.28 (d, 1H), 8.08 (m, 1H), 7.83 (d, 2H), 7.73 (dd, 1H), 7.69 (d, 1 H), 7.50 (d, 2H), 5.30 (hept, 1H), 4.81 (d, 2H), 3.08 (hept, 1H), 2.63 (t, 1H), 1.42 (d, 6H), 1.38 (d, 6H).
MS: 576 (M+1)⁺

### Example 69: 2-Fluoro-5-{[4-(4-isopropyl-phenyl)-6-prop-2-ynyloxy-quinazoline-2-carbonyl]-amino}-benzoic acid methyl ester

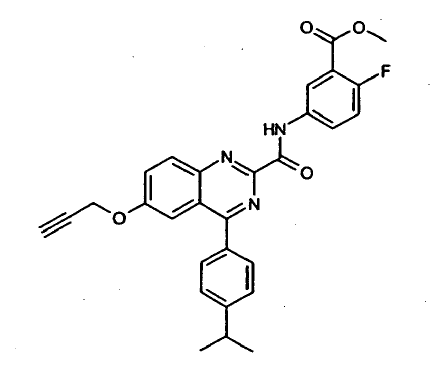

¹H-NMR (400 MHz, CDCl₃): 10.33 (s, 1H), 8.38 - 8.34 (m, 1H), 8.31 (d, 1H), 8.10 (dd, 1H), 7.83 (d, 2H), 7.71 (dd, 1 H), 7.68 (d, 1H), 7.49 (d, 2H), 7.21 (t, 1H), 4.80 (d, 2H), 3.96 (s, 3H), 3.08 (hept, 1 H), 2.62 (t, 1H), 1.37 (d, 6H).
MS: 498 (M+1)⁺

### Example 70: 2-Fluoro-5-{[4-(4-isopropyl-phenyl)-6-prop-2-ynyloxy-quinazoline-2-carbonyl]-amino}-benzoic acid isopropyl ester

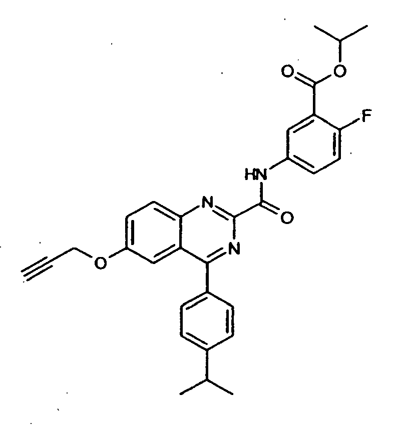

¹H-NMR (400 MHz, CDCl₃): 10.30 (s, 1H), 8.30 - 8.26 (m, 2H), 8.11 (dd, 1H), 7.82 (d, 2H), 7.72 (dd, 1H), 7.68 (d, 1H), 7.49 (d, 2H), 7.18 (t, 1H), 5.29 (hept, 1H), 4.80 (d, 2H), 3.07 (hept, 1 H), 2.63 (t, 1H), 1.40 (d, 6H), 1.37 (d, 6H).
MS: 526 (M+1)⁺

### Example 71: 2-Chloro-5-{[4-(4-isopropyl-phenyl)-6-prop-2-ynyloxy-quinazoline-2-carbonyl]-amino}-benzoic acid methyl ester

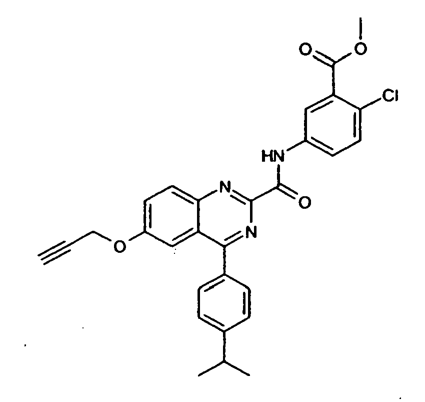

¹H-NMR (400 MHz, DMSO d₆): 11.06 (s, 1H), 8.46 (d, 1H), 8.25 (d; 1H), 8.16 (dd, 1H), 7.97 (d, 2H), 7.86 (dd, 1H), 7.72 (d, 1H), 7.63 (d, 1H), 7.56 (d, 2H), 5.02 (d, 2H), 3.91 (s, 3H), 3.80 (t, 1H)), 3.07 (hept, 1H), 1.32 (d, 6H).
MS: 514 (M+1)⁺ (isotope pattern for 1 Cl)

### Example 72: 2,5-Dichloro-3-{[4-(4-isopropyl-phenyl)-6-prop-2-ynyloxy-quinazoline-2-carbonyl]-amino}-benzoic acid methyl ester

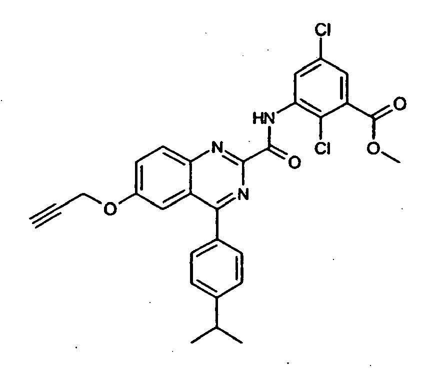

¹H-NMR (400 MHz, CDCl₃): 11.39 (s, 1H), 9.05 (d, 1 H), 8.32 (d, 1H), 7.91 (d, 2H), 7.76 (d, 1H), 7.71 (dd, 1H), 7.61 (d, 1H), 7.47 (d, 2H), 4.82 (d, 2H), 3.96 (s, 3H), 3.06 (hept, 1H), 2.64 (t, 1H), 1.36 (d, 6H).
MS: 548 (M+1)⁺ (isotope pattern for 2 Cl)

### Example 73: 2,5-Dichloro-3-{[4-(4-isopropyl-phenyl)-6-prop-2-ynyloxy-quinazoline-2-carbonyl]-amino}-benzoic acid isopropyl ester

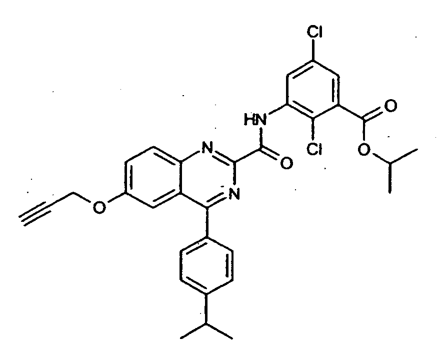

¹H-NMR (400 MHz, CDCl₃): 11.36 (s, 1 H), 9.03 (d, 1H), 8.34 (d, 1H), 7.91 (d, 2H), 7.76 (d, 1 H), 7.71 (dd, 1H), 7.54 (d, 1H), 7.47 (d, 2H), 5.29 (hept, 1H), 4.82 (d, 2H), 3.06 (hept, 1H), 2.64 (t, 1H), 1.41 (d, 6H), 1.36 (d, 6H).
MS: 548 (M+1)⁺ (isotope pattern for 2 Cl)

### Example 74: 4-(4-Isopropyl-phenyl)-6-prop-2-ynyloxy-quinazoline-2-carboxylic acid (3-cyano-5-fluoro-phenyl)-amide

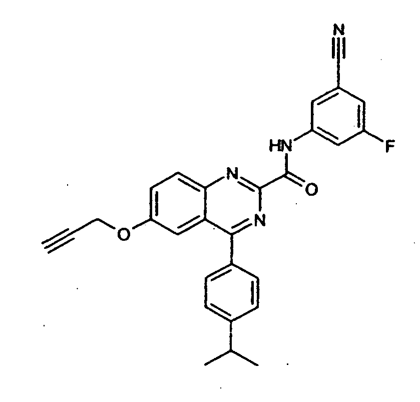

¹H-NMR (400 MHz, CDCl₃): 10.44 (s, 1H), 8.29 (d, 1H), 8.12 (dt, 1H), 7.83 - 7.81 (m, 3H), 7.73 (dd, 1H), 7.69 (d, 1H), 7.49 (d, 2H), 7.15 (ddd, 1H), 4.81 (d, 2H), 3.08 (hept, 1H), 2.63 (t, 1 H), 1.37 (d, 6H).
MS: 465 (M+1)⁺

### Example 75: 4-(4-Isopropyl-phenyl)-6-prop-2-ynyloxy-quinazoline-2-carboxylic acid (3,4-dicyano-phenyl)-amide

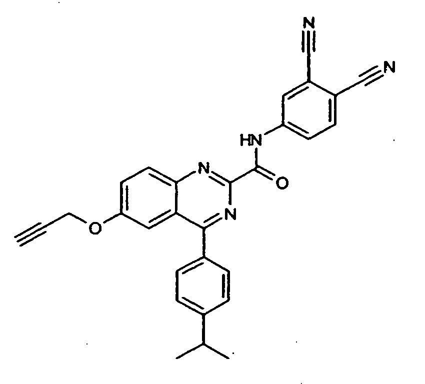

¹H-NMR (400 MHz, CDCl₃): 10.61 (s, 1H), 8.33 (d, 1 H), 8.28 (d, 1H), 8.25 (dd, 1H), 7.81 (d, 1 H), 7.81 (d, 2H), 7.74 (dd, 1H), 7.69 (d, 1H), 7.49 (d, 2H), 4.81 (d, 2H), 3.08 (hept, 1 H), 2.63 (t, 1 H), 1.37 (d, 6H).
MS: 472 (M+1)⁺

### Example 76: 4-(4-Isopropyl-phenyl)-6-prop-2-ynyloxy-quinazoline-2-carboxylic acid (4-cyano-3-trifluoromethyl-phenyl)-amide

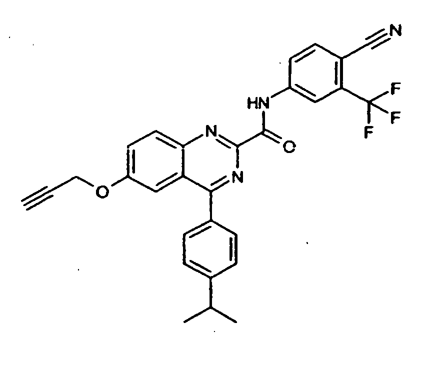

¹H-NMR (400 MHz, CDCl₃): 10.57 (s, 1H), 8.34 (dd, 1H), 8.28 (d, 1H), 8.16 (d, 1H), 7.87 (d, 1H), 7.81 (d, 2H), 7.75 (dd, 1H), 7.70 (d, 1H), 7.50 (d, 2H), 4.81 (d, 2H), 3.08 (hept, 1H), 2.63 (t, 1H), 1.37 (d, 6H).
MS: 515 (M+1)⁺

### Example 77: 4-(4-Isopropyl-phenyl)-6-prop-2-ynyloxy-quinazoline-2-carboxylic acid (3-trifluoromethyl-phenyl)-amide

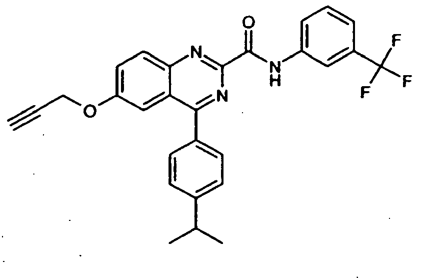

¹H-NMR (400 MHz, CDCl₃): 10.38 (s, 1 H), 8.32 (d, 1 H), 8.20 (d, 1 H), 8.01 (s, 1 H), 7.85 (d, 2H), 7.71 (dd, 1H), 7.69 (d, 1H), 7.54 (t, 1H), 7.50 (d, 2H), 7.42 (d, 1H), 4.80 (d, 2H), 3.08 (hept, 1H), 2.63 (t, 1H), 1.38 (d, 6H).
MS: 490 (M+1)⁺

### Example 78: 4-(4-Isopropyl-phenyl)-6-prop-2-ynyloxy-quinazoline-2-carboxylic acid (4-acetylamino-3-trifluoromethyl-phenyl)-amide

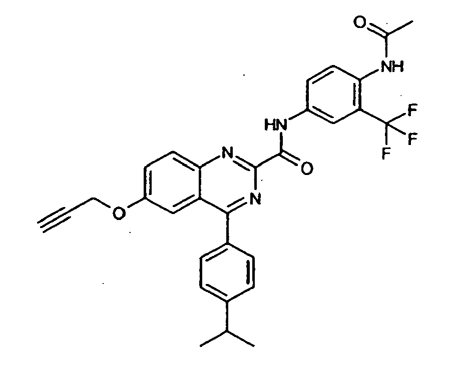

¹H-NMR (400 MHz, CDCl₃): 10.32 (s, 1H), 8.27 (d, 1H), 8.21 (s, 1H), 8.12 (d, 1 H), 7.97 (d, 1H), 7.83 (d, 2H), 7.71 (dd, 1 H), 7.67 (d, 1H), 7.49 (d, 2H), 7.44 (s, 1 H), 4.80 (d, 2H), 3.07 (hept, 1H), 2.62 (t, 1H), 2.25 (t, 1H), 1.37 (d, 6H).
MS: 547 (M+1)⁺

### Example 79: 4-(4-Isopropyl-phenyl)-6-prop-2-ynyloxy-quinazoline-2-carboxylic acid (3-methoxy-5₋trifluoromethyl-phenyl)-amide

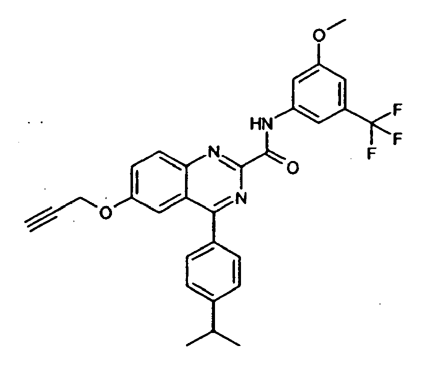

¹H-NMR (400 MHz, CDCl₃): 10.33 (s, 1H), 8.30 (d, 1H), 8.01 (t, 1 H), 7.84 (d, 2H),7.70 (dd, 1H), 7.68 (d, 1H), 7.49 (d, 2H), 7.41 (s, 1H), 6.94 (s, 1H), 4.80 (d, 2H), 3.90 (s, 3H), 3.08 (hept, 1H), 2.62 (t, 1 H), 1.37 (d, 6H).
MS: 520 (M+1)⁺

### Example 80: 4-(4-Isopropyl-phenyl)-6-prop-2-ynyloxy-quinazoline-2-carboxylic acid (3,5-bis-trifluoromethyl-phenyl)-amide

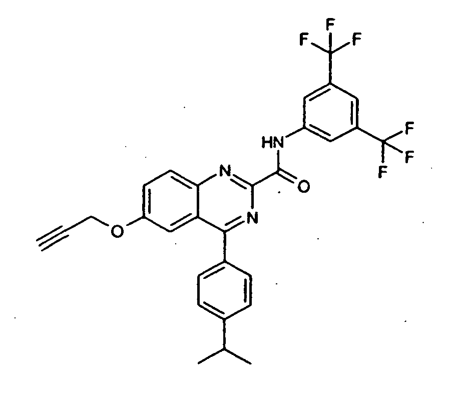

¹H-NMR (400 MHz, CDCl₃): 10.48 (s, 1H), 8.33 (s, 2H), 8.24 (d, 1H), 7.81 (d, 2H), 7.74 (dd, 1H), 7.69 - 7.68 (m, 2H), 7.50 (d, 2H), 4.81 (d, 2H), 3.08 (hept, 1 H), 2.63 (t, 1H), 1.37 (d, 6H).
MS: 558 (M+1)⁺

### Example 81: 4-(4-Isopropyl-phenyl)-6-prop-2-ynyloxy-quinazoline-2-carboxylic acid (3-fluoro-5-trifluoromethyl-phenyl)-amide

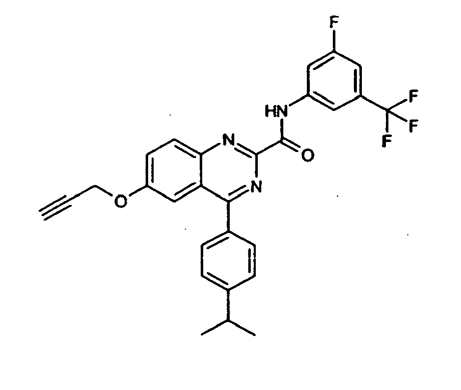

¹H-NMR (400 MHz, CDCl₃): 10.42 (s, 1 H), 8.27 (d, 1H), 8.14 (d, 1H), 7.82 (d, 2H), 7.72 (dd, 1H), 7.68 (d, 1 H), 7.66 (s, 1H), 7.50 (d, 2H), 7.13 (d, 1H), 4.80 (d, 2H), 3.08 (hept, 1 H), 2.63 (t, 1 H), 1.37 (d, 6H).
MS: 508 (M+1)⁺

### Example 82: 4-(4-Isopropyl-phenyl)-6-prop-2-ynyloxy-quinazoline-2-carboxylic acid (4-fluoro-3-trifluoromethyl-phenyl)-amide

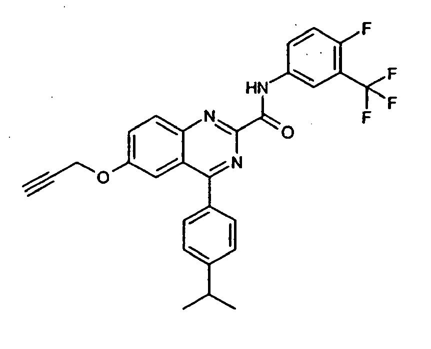

¹H-NMR (400 MHz, CDCl₃): 10.34 (s, 1 H), 8.33 (d, 1 H), 8.20 (dt, 1 H), 8.01 (dd, 1H), 7.85 (d, 2H), 7.72 (dd, 1H), 7.69 (d, 1H), 7.51 (d, 2H), 7.26 (t, 1H), 4.81 (d, 2H), 3.09 (hept, 1 H), 2.64 (t, 1H), 1.39 (d, 6H).
MS: 508 (M+1)⁺

### Example 83: 3-{[4-(4-Isopropyl-phenyl)-6-prop-2-ynyloxy-quinazoline-2-carbonyl]-amino}-2-methyl-benzoic acid methyl ester

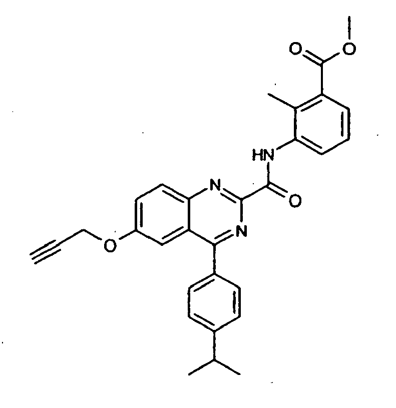

¹H-NMR (400 MHz, DMSO d₆): 10.65 (s, 1H), 8.24 (d, 1H), 8.00 (d, 2H), 7.93 (d, 1H), 7.85 (dd, 1 H), 7.74 (d, 1 H), 7.64 (d, 1H), 7.55 (d, 2H), 7.39 (t, 1H), 5.01 (d, 2H), 3.86 (s, 3H), 3.80 (t, 1H), 3.06 (hept, 1H), 2.47 (s, 3H), 1.31 (d, 6H).
MS: 494 (M+1)⁺

### Example 84: 3-{[4-(4-Isopropyl-phenyl)-6-prop-2-ynyloxy-quinazoline-2-carbonyl]-amino}-4-methyl-benzoic acid methyl ester

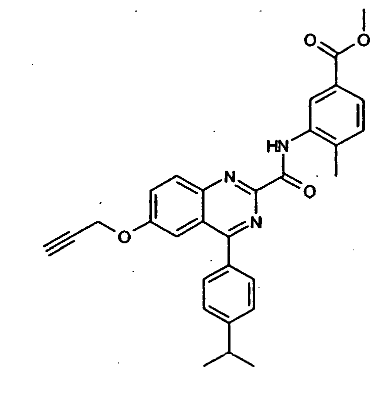

¹H-NMR (400 MHz, CDCl₃): 10.37 (s, 1H), 8.93 (s, 1H), 8.33 (d, 1H), 7.88 (d, 2H), 7.81 (dd, 1H), 7.72 - 7.69 (m, 2H), 7.49 (d, 2H), 7.32 (d, 1H), 4.82 d, 2H), 3.92 (s, 3H), 3.08 (hept, 1H), 2.64 (t, 1H), 2.50 (s, 1 H), 1.38 (d, 6H).
MS: 494 (M+1)⁺

### Example 85: 3-{[4-(4-Isopropyl-phenyl)-6-prop-2-ynyloxy-quinazoline-2-carbonyl]-amino}-4-methoxy-benzoic acid methyl ester

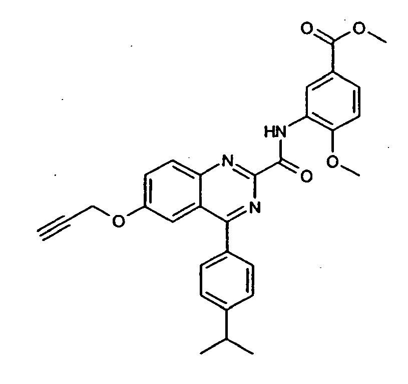

¹H-NMR (400 MHz, CDCl₃): 10.87 (s, 1H), 9.34 (d, 1H), 8.32 (d, 1H), 7.92 (d, 2H), 7.85 (dd, 1H), 7.72 (d, 1H), 7.68 (dd, 1H), 7.47 (d, 2H), 6.96 (d, 1H), 4.80 (t, 2H), 4.03 (s, 3H), 3.90 (s, 3H), 3.07 (hept, 1 H), 2.63 (t, 1H), 1.37 (d, 6H).
MS: 510 (M+1)⁺

### Example 86: 5-{[4-(4-Isopropyl-phenyl)-6-prop-2-ynyloxy-quinazoline-2-carbonyl]-amino}-isophthalic acid dimethyl ester

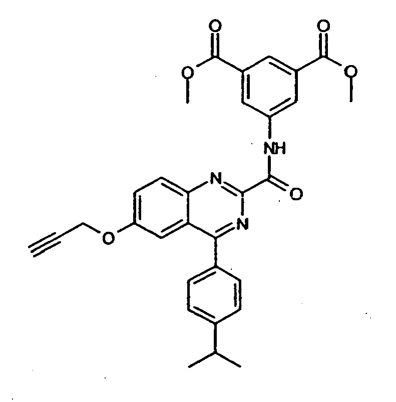

¹H-NMR (400 MHz, CDCl₃): 10.43 (s, 1H), 8.71 (d, 2H), 8.48 (t, 1H), 8.29 (d, 1H), 7.84 (d, 2H), 7.71 (dd, 1H), 7.68 (d, 1H), 7.50 (d, 2H), 4.80 (d, 2H), 3.97 (s, 6H), 3.08 (hept, 1H), 2.62 (t, 1H), 1.38 (d, 6H).
MS: 538 (M+1)⁺

### Example 87: 4-{[4-(4-Isopropyl-phenyl)-6-prop-2-ynyloxy-quinazoline-2-carbonyl]-amino}-phthalic acid dimethyl ester

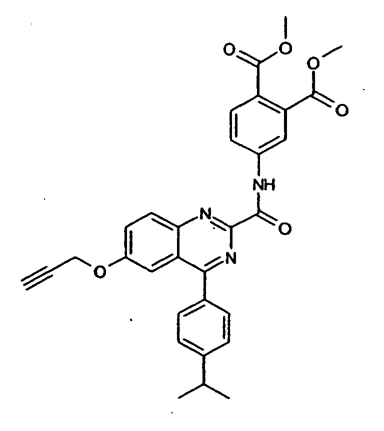

¹H-NMR (400 MHz, CDCl₃): 10.47 (s, 1H), 8.29 (d, 1H), 8.25 (dd, 1H), 7.92 (d, 1H), 7.88 (d, 1H), 7.83 (d, 2H), 7.70 (dd, 1H), 7.68 (d, 1H), 7.49 (d, 2H), 4.80 (d, 2H), 3.93 (s, 3H), 3.90 (s, 3H), 3.07 (hept, 1 H); 2.62 (t, 1 H), 1.37 (d, 6H).
MS: 538 (M+1)⁺

### Example 88: 4-(4-Isopropyl-phenyl)-6-prop-2-ynyloxy-quinazoline-2-carboxylic acid (3,5-dichloro-phenyl)-amide

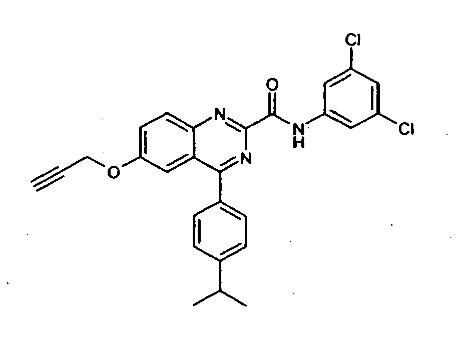

¹H-NMR (400 MHz, CDCl₃): 10.29 (s, 1H), 8.29 (d, 1H), 7.83 (d, 2H), 7.81 (d, 2H), 7.70 (dd, 1H), 7.67 (d, 1H), 7.49 (d, 2H), 7.15 (t, 1 H), 4.80 (d, 2H), 3.07 (hept, 1 H), 2.62 (t, 1H), 1.37 (d, 6H).
MS: 490 (M+1)⁺ (isotope pattern for 2 Cl)

### Example 89: 4-(4-Isopropyl-phenyl)-6-orop-2-ynyloxy-quinazoline-2-carboxylic acid (3,4-dichloro-phenyl)-amide

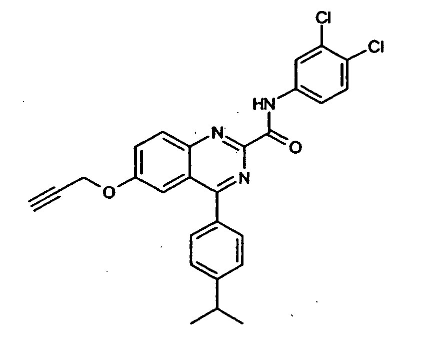

¹H-NMR (300 MHz, CDCl₃): 10.29 s, 1H), 8.32 (d, 1H), 8.02 (d, 1H), 7.85 (d, 2H), 7.77 (dd, 1H), 7.71 (dd, 1H), 7.68 (d, 1H), 7.50 (d, 2H), 7.46 (d, 1H), 4.81 (d, 2H), 3.10 (hept, 1H), 2.64 (t, 1 H), 1.40 (d, 6H).
MS: 490 (M+1)⁺ (isotope pattern for 2 Cl)

### Example 90: 4-(4-Isopropyl-phenyl)-6-prop-2-ynyloxy-quinazoline-2-cairboxylic acid (3-chloro-4-fluoro-phenyl)-amide

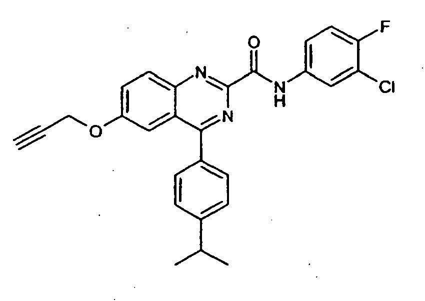

¹H-NMR (400 MHz, CDCl₃): 10.25 (s, 1H), 8.31 (d, 1H), 7.97 (d, 1H), 7.84 (d, 2H), 7.77 - 7.68 (m, 3H), 7.50 (d, 2H), 7.18 (t, 1H), 4.81 (d, 2H), 3.09 (hept, 1H), 2.63 (t, 1H), 1.38 (d, 6H).
MS: 474 (M+1)⁺ (isotope pattern for 1 Cl)

### Example 91: 4-(4-Isopropyl-phenyl)-6-prop-2-ynyloxy-quinazoline-2-carboxylic acid (4-chloro-3-trifluoromethyl-phenyl)-amide

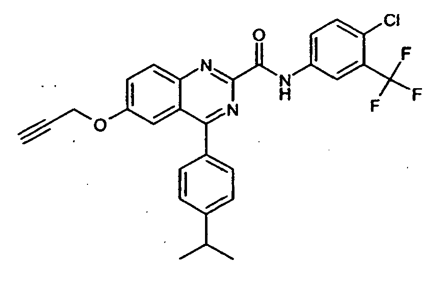

¹H-NMR (400 MHz, CDCl₃): 10.37 (s, 1 H), 8.32 (d, 1H), 8.18 (dd, 1H), 8.07 (d, 1H), 7.84 (d, 2H), 7.72 (dd, 1H), 7.68 (d, 1 H), 7.54 (d, 1 H), 7.50 (d, 2H), 4.80 (d, 2H), 3.08 (hept, 1 H), 2.63 (t, 1 H), 1.38 (d, 6H).
MS: 524 (M+1)⁺ (isotope pattern for 1 Cl)

### Example 92: 5-{[4-(4-Isopropyl-phenyl)-6-prop-2-ynyloxy-quinazoline-2-carbonyl]-amino}-pyridine-2-carboxylic acid methyl ester

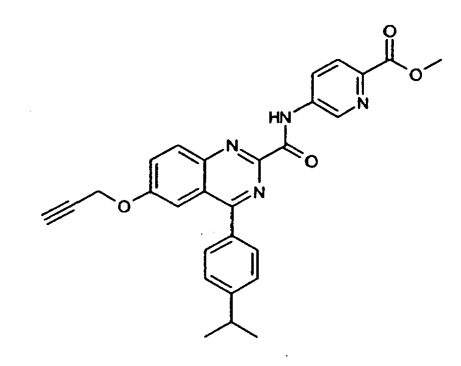

¹H-NMR (400 MHz, CDCl₃): 10.48 (s, 1 H), 8.91 (d, 1H), 8.75 (dd, 1 H), 8.31 (d, 1H), 8.22 (d, 1H), 7.84 (d, 2H), 7.72 (dd, 1H), 7.68 (d, 1H), 7.49 (d, 2H), 4.80 (d, 2H), 4.01 (s, 3H), 3.07 (hept, 1 H), 2.62 (t, 1 H), 1.37 (d, 6H).
MS: 481 (M+1)⁺

### Example 93: 5-{[4-(4-Isopropyl-phenyl)-6-prop-2-ynyloxy-quinazoline-2-carbonyl]-amino}-nicotinic acid methyl ester

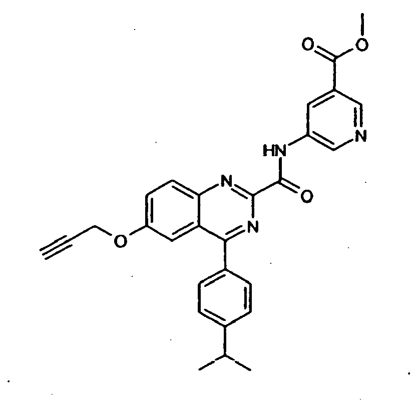

¹H-NMR (400 MHz, DMSO-d₆): 11.21 (s, 1 H), 9.32 (d, 1 H), 8.93 (t, 1H), 8.87 (d, 1 H), 8.26 (d, 1H), 7.97 (d, 2H), 7.86 (dd, 1H), 7.72 (d, 1H), 7.56 (d, 2H), 5.02 (d, 2H), 3.93 (s, 3H), 3.80 (t, 1H), 3.07 (hept, 1H), 1.32 (d, 6H).
MS: 481 (M+1)⁺

### Example 94: 5-{[4-(4-Isopropyl-phenyl)-6-prop-2-ynyloxy-quinazoline-2-carbonyl]-amino}-nicotinic acid isopropyl ester

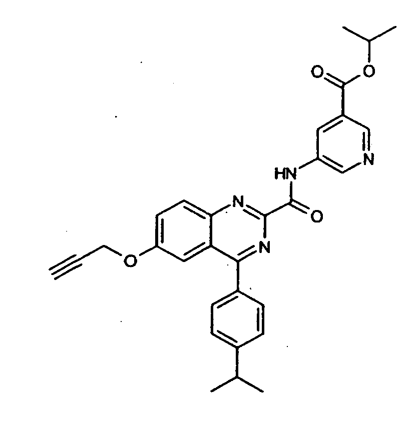

¹H-NMR (400 MHz, CDCl₃): 10.60 (s, 1H), 9.34 (d, 1H), 9.22 (t, 1H), 9.03 (d, 1H), 8.29 (d, 1H), 7.81 (d, 2H), 7.73 (dd, 1H), 7.69 (d, 1H), 7.48 (d, 2H), 5.33 (hept, 1H), 4.80 (d, 2H), 3.07 (hept, 1 H), 2.63 (t, 1 H), 1.42 (d, 6H), 1.37 (d, 6H).
MS: 509 (M+1)⁺

### Example 95: [4-(4-Isopropyl-phenyl)-6-prop-2-ynyloxy-quinazolin-2-yl]-pyrrol-1-yl-methanone

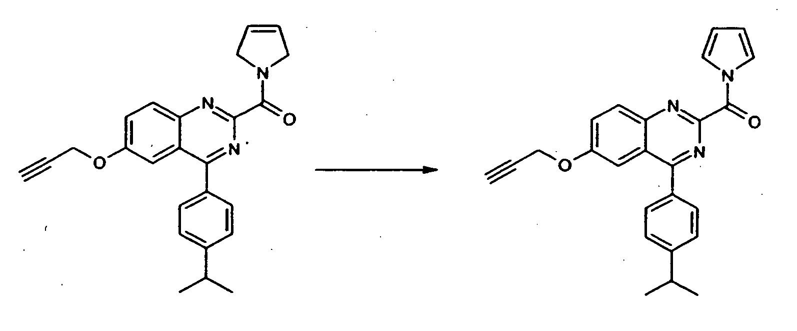

The intermediate (2,5-dihydro-pyrrol-1-yl)-[4-(4-isopropyl-phenyl)-6-prop-2-ynyloxy-quinazolin-2-yl]-methanone is prepared from 4-(4-isopropyl-phenyl)-6-propargyloxy-quinazoline-2-carboxylic acid and commercially available 3-pyrroline using the method described in example 46. A solution of 200 mg (0.50 mmol) of this intermediate and 150 mg (0.65 mmol) DDQ (2,3-dichloro-5,6-dicyano-p-benzoquinone) in 1 ml ethyl acetate is stirred for 18 h at RT. Water is added and the reaction mixture is extracted with ethyl acetate. The solvent is evaporated and the product is purified by flash chromatography using a ethyl acetate / hexane gradient.
¹H-NMR (400 MHz, CDCl₃): 8.22 (m, 1H), 7.86 (d, 2H), 7.72 - 7.69 (m, 2H), 7.64 (dd, 2H), 7.46 (d, 2H), 6.37 (dd, 1H), 4.83 (d, 2H), 3.06 (hept, 1H), 2.65 (t, 1H), 1.36 (d, 6H).
MS: 396 (M+1)⁺

### Example 96: 4-(4-Isopropyl-phenyl)-6-prop-2-ynyloxy-quinazoline-2-carboxylic acid (5-methyl-1 H-pyrazol-3-yl)-amide

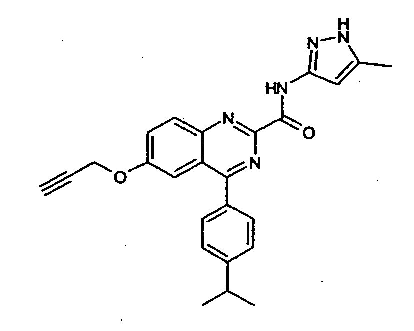

¹H-NMR (400 MHz, CDCl₃): 10.64 (s, 1 H), 8.24 (d, 1 H), 7.80 (d, 2H), 7.65 - 7.64 (m, 1H), 7.44 (d, 2H), 6.69 (broad, 1H), 4.78 (d, 2H), 3.05 hept, 1H), 2.62 (t, 1H), 2.34 (s, 3H), 1.35 (d, 6H).
MS: 426 (M+1)⁺

### Example 97: (2-{[4-(4-Isopropyl-phenyl)-6-prop-2-ynyloxy-quinazoline-2-carbonyl]-a mino}-thiazol-4-yl)-acetic acid ethyl ester

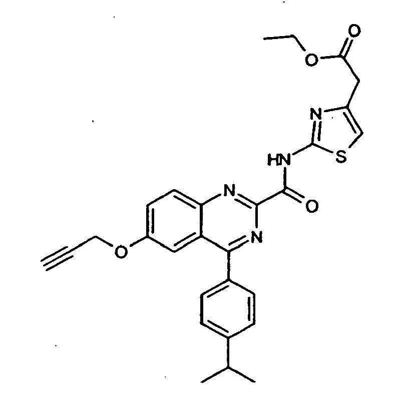

¹H-NMR (400 MHz, CDCl₃): 8.28 (d, 1H), 7.86 (d, 2H), 7.72 - 7.70 (m, 2H), 7.46 (d, 2H), 6.94 (s, 1H), 4.80 (d, 2H), 4.22 (q, 2H), 3.78 (s, 2H), 3.06 (hept, 1H), 2.62 (t, 1H), 1.36 (d, 6H), 1.29 (t, 3H).
MS: 515 (M+1)⁺

### Example 98: 4-(4-Isopropyl-phenyl)-6-prop-2-ynyloxy-quinazoline-2-carboxylic acid naphthalen-1-ylamide

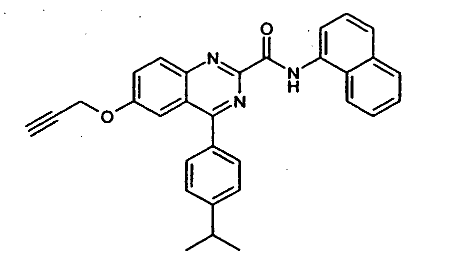

¹H-NMR (400 MHz, CDCl₃): 11.04 (s, 1H), 8.53 (d, 1H), 8.38 (d, 1H), 8.10 (d, 1H), 7.94 - 7.90 (m, 3H), 7.73 - 7.71 (m, 3H), 7.61 - 7.50 (m, 5H), 4.82 (d, 2H), 3.09 (hept, 1H), 2.64 (t, 1 H), 1.39 (d, 6H).
MS: 472 (M+1)⁺

### Example 99: 4-(4-Isopropyl-phenyl)-6-prop-2-ynyloxylquinazoline-2-carboxylic acid isoquinolin-8-ylamide

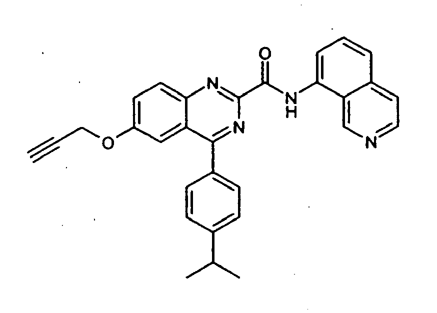

¹H-NMR (400 MHz, CDCl₃): 11.28 (s, 1H), 9.64 (s, 1H), 8.70 (d, 1H), 8.61 (d, 1H), 8.37 (d, 1H), 7.91 (d, 2H), 7.81 (t, 1H), 7.75 - 7.72 (m, 3H), 7.69 (d, 1H), 7.53 (d, 2H), 4.82 (d, 2H), 3.09 (hept, 1 H), 2.65 (t, 1 H), 1.39 (d, 6H).
MS: 473 (M+1)⁺

### Example 100: 4-(4-Isopropyl-phenyl)-6-prop-2-ynyloxy-quinazoline-2-carboxylic acid phthalazin-5-ylamide

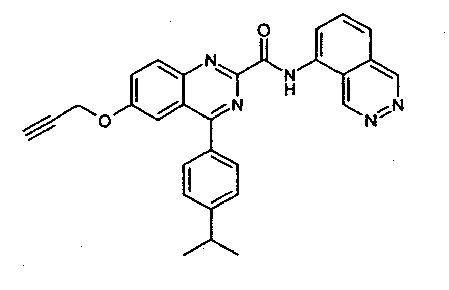

¹H-NMR (400 MHz, CDCl₃): 11.38 (s, 1H), 10.31 (s, 1H), 9.74 (s, 1H), 8.93 (d, 1H), 8.33 (d, 1H), 8.29 (t, 1H), 8.09 (d, 1H), 7.85 (d, 2H), 7.72 - 7.68 (m, 2H), 7.42 (d, 2H), 4.80 (s, 2H), 3.00 (hept, 1 H), 2.65 (s, 1H), 1.32 (d, 6H).
MS: 474 (M+1)⁺

### Example 101: 4-(4-Isopropyl-phenyl)-6-prop-2-ynyloxy-quinazoline-2-carboxylic acid quinolin-5-ylamide

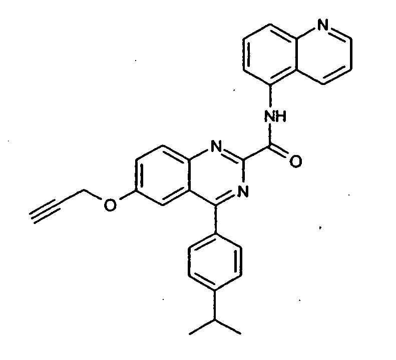

¹H-NMR (400 MHz, CDCl₃): 10.90 (s, 1H), 8.97 (dd, 1H), 8.44 (d, 2H), 8.34 (d, 1H), 8.02 (d, 1 H), 7.90 (d, 2H), 7.81 (t, 1 H), 7.73 - 7.70 (m, 2H), 7.51 - 7.48 (m, 3H), 4.81 (d, 2H), 3.08 (hept, 1H), 2.64 (t, 1H), 1.38 (d, 6H).
MS: 473 (M+1)⁺

### Example 102: 4-(4-Isopropyl-phenyl)-6-prop-2-ynyloxy-quinazoline-2-carboxylic acid quinolin-8-ylamide

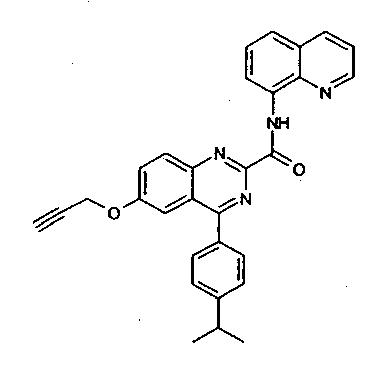

¹H-NMR (400 MHz, CDCl₃): 12.62 (s, 1H), 9.11 (dd, 1H), 8.95 (dd, 1H), 8.38 (d, 1 H), 8.21 (dd, 1H), 8.04 (d, 2H), 7.77 (d, 1H), 7.69 (dd, 1 H), 7.65 (t, 1H), 7.59 (dd, 1H), 7.52 - 7.49 (m, 3H), 4.81 (d, 2H), 3.08 (hept, 1 H), 2.64 (t, 1H), 1.38 (d, 6H).
MS: 473 (M+1)⁺

### Example 103: 4-(4-Isopropyl-phenyl)-6-prop-2-ynyloxy-quinazoline-2-carboxylic acid isoquinolin-4-ylamide

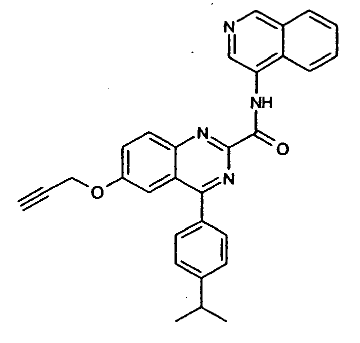

¹H-NMR (400 MHz, CDCl₃): 10.52 (s, 1 H), 9.12 (d, 1 H), 9.03 (d, 1H), 8.32 (d, 1H), 8.09 (d, 1 H), 7.90 (dd, 1H), 7.86 (d, 2H), 7.71 (dd, 1H), 7.68 (d, 1 H), 7.66 (ddd, 1H), 7.57 (ddd, 1 H), 7.50 (d, 2H), 4.80 (d, 2H), 3.08 (hept, 1H), 2.63 (t, 1 H), 1.38 (d, 6H).
MS: 473 (M+1)⁺

### Example 104: 4-(4-Isopropyl-phenyl)-6-prop-2-ynyloxy-quinazoline-2-carboxylic acid (5-acetyl-quinolin-8-yl)-amide

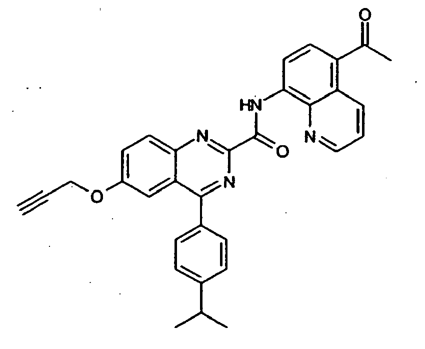

¹H-NMR (400 MHz, CDCl₃): 12.92 (s, 1H), 9.55 (dd, 1H), 9.13 (d, 1H); 8.98 (dd, 1H), 8.40 (d, 1 H), 8.29 (d, 1H), 8.05 (d, 2H), 7.79 (d, 1 H), 7.72 (dd, 1H), 7.64 (dd, 1H), 7.53 (d, 2H), 4.83 (d, 2H), 3.10 (hept, 1H), 2.78 (s, 3H), 2.66 (t, 1H), 1.40 (d, 6H).
MS: 515 (M+1)⁺

### Example 105: 4-(4-Isopropyl-phenyl)-6-prop-2-ynyloxy-quinazoline-2-carboxylic acid (3-bromo-6-methoxy-quinolin-8-yl)-amide

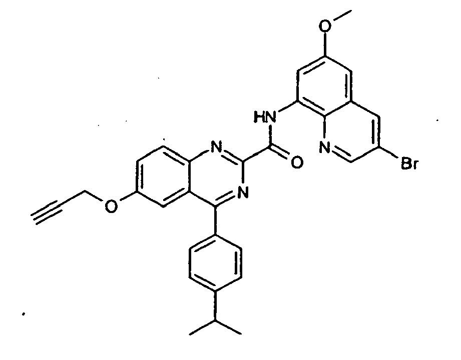

¹H-NMR (400 MHz, CDCl₃): 12.43 (s, 1H), 8.87 (d, 1H), 8.75 (d, 1H), 8.39 (d, 1 H), 8.22 (d, 1H), 8.02 (d, 2H), 7.78 (d, 1H), 7.71 (dd, 1H), 7.52 (d, 2H), 6.79 (d, 1H), 4.83 (d, 2H), 3.98 (s, 3H), 3.11 (hept, 1H), 2.65 (t, 1 H), 1.40 (d, 6H).
MS: 581 / 583 (M+1)⁺ (isotope pattern for 1 Br)

### Example 106: 4-(4-isopropyl-phenyl)-6-prop-2-ynyloxy-quinazoline-2-carboxylic acid quinolin-2-ylamide

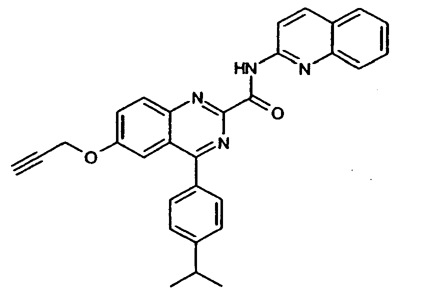

¹H-NMR (400 MHz, CDCl₃): 11.01 (broad, 1H), 8.82 (d, 1 H), 8.34 (d, 1H), 8.30 (d, 1H), 7.96 (d, 1H), 7.90 (d, 2H), 7.84 (d, 1H), 7.74 - 7.70 (m, 3H), 7.51 - 7.48 (m, 3H), 4.82 (d, 2H), 3.09 (hept, 1 H), 2.64 (t, 1H), 1.39 (d, 6H).
MS: 473 (M+1)⁺

### Example 107: 4-(4-Isopropyl-phenyl)-6-prop-2-ynyloxy-quinazoline-2-carboxylic acid quinolin-6-ylamide

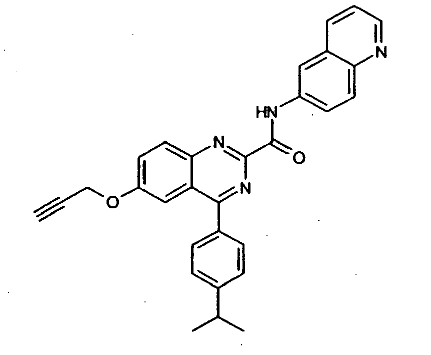

¹H-NMR (400 MHz, CDCl₃): 10.51 (s, 1H), 8.85 (dd, 1H), 8.71 (d, 1H), 8.31 (dd, 1 H), 8.23 (dd, 1H), 8.13 (d, 1 H), 7.88 (dd, 1H), 7.86 (d, 2H), 7.70 (dd, 1H), 7.67 (d, 1H), 7.50 (d, 2H), 7.42 (dd, 1H), 4.79 (d, 2H), 3.08 (hept, 1H), 2.62 (t, 1H), 1.38 (d, 6H).
MS: 473 (M+1)⁺

### Example 108: 4-(4-Isopropyl-phenyl)-6-prop-2-ynyloxy-quinazoline-2-carboxylic acid (2-methyl-quinolin-6-yl)-amide

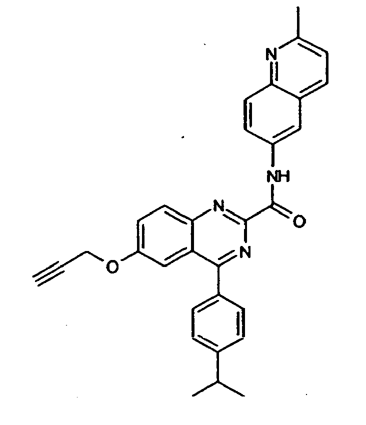

¹H-NMR (400 MHz, CDCl₃): 10.48 (s, 1 H), 8.67 (d, 1H), 8.31 (d, 1H), 8.12 (d, 1H), 8.05 (d, 1 H), 7.86 (d, 2H), 7.83 (dd, 1H), 7.69 (dd, 1H), 7.67 (d, 1H), 7.49 (d, 2H), 7.30 (d, 1H), 4.79 (d, 2H), 3.08 (hept, 1H), 2.75 (s, 3H), 2.62 (t, 1H), 1.38 (d, 6H).
MS: 487 (M+1)⁺

### Example 109: (6-{[4-(4-Isopropyl-phenyl)-6-prop-2-ynyloxy-quinazoline-2-carbonyl]-amino}-quinolin-8-yloxy)-acetic acid ethyl ester

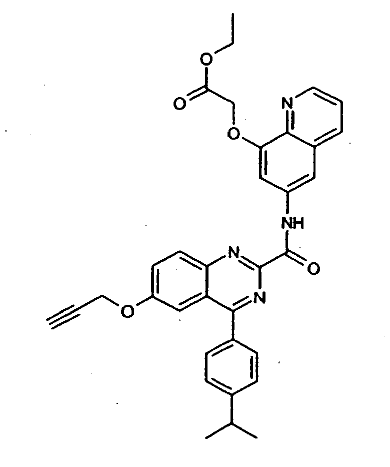

¹H-NMR (400 MHz, CDCl₃): 10.41 (s, 1 H), 8.88 (dd, 1H), 8.32 (d, 1H), 8.15 (dd, 1H), 7.97 (d, 1 H), 7.86 (d, 2H), 7.71 (dd, 1H), 7.66 (dd, 1H), 7.50 (d, 2H), 7.43 (dd, 1 H), 5.04 (s, 2H), 4.80 (d, 2H), 4.30 (q, 2H), 3.08 (hept, 1H), 2.62 (t, 1H), 1.38 (d, 6H), 1.29 (t, 1 H).
MS: 575 (M+1)⁺

### Example 110: 4-(4-Isopropyl-phenyl)-6-prop-2-ynyloxy-quinazoline-2-carboxylic acid (1 H-benzoimidazol-4-yl)-amide

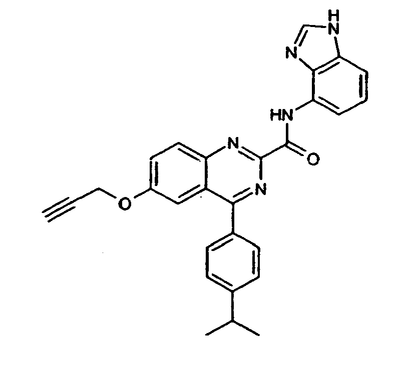

¹H-NMR (400 MHz, DMSO-d₆): 12.68 (s, 1H), 11.19 (s, 1H), 8.32 - 8.27 (m, 3H), 7.95 (d, 2H), 7.87 (dd, 1H), 7.72 (d, 1H), 7.59 (d, 2H), 7.34 (d, 1H), 7.27 (t, 1H), 5.02 (d, 2H), 3.81 (t, 1 H), 3.08 (hept, 1 H), 1.34 (d, 6H).
MS: 575 (M+1)⁺

### Example 111: 4-(4-Isopropyl-phenyl)-6-prop-2-ynyloxy-quinazoline-2-carboxylic acid benzothiazol-2-ylamide

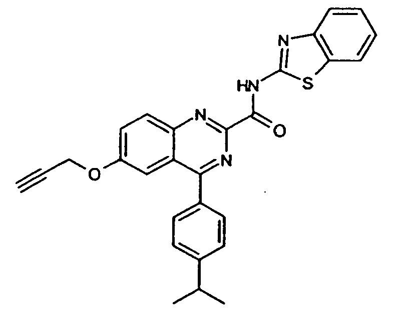

¹H-NMR (400 MHz, CDCl₃): 11.56 (broad, 1H), 8.30 (d, 1H), 7.90 - 7.84 (m, 4H), 7.73 (dd, 1H), 7.71 (d, 1H), 7.50 - 7.46 (m, 3H), 7.35 (td, 1H), 4.81 (d, 2H), 3.07 (hept, 1H), 2.63 (t, 1 H), 1.37 (d, 6H).
MS: 479 (M+1)⁺

### Example 112: 4-(4-Isopropyl-phenyl)-6-prop-2-ynyloxy-quinazoline-2-carboxylic acid (benzo[1,3]dioxol-5-ylmethyl)-amide

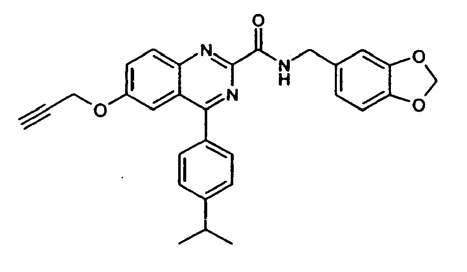

¹H-NMR (400 MHz, CDCl₃): 8.59 (broad, 1H), 8.29 (d, 1H), 7.78 (d, 2H), 7.68 - 7.63 (m, 2H), 7.44 (d, 2H), 6.92 (s, 1H), 6.87 (d, 1H), 6.77 (d, 1H), 5.94 (s, 2H), 4.78 (d, 2H), 4.68 (m, 2H), 3.04 (hept, 1H), 2.61 (t, 1H), 1.34 (d, 6H).
MS: 480 (M+1)⁺

### Example 113: 4-(4-Isopropyl-phenyl)-6-prop-2-ynyloxy-quinazoline-2-carboxylic acid (thiophen-2-ylmethyl)-amide

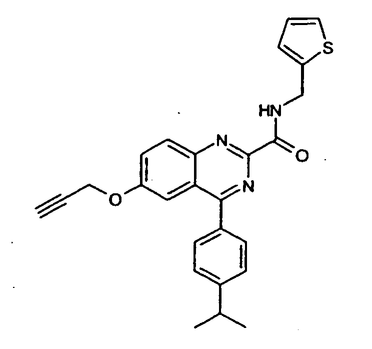

¹H-NMR (400 MHz, CDCl₃): 8.65 (t, 1 H), 8.24 (d, 1 H), 7.77 (d, 2H), 7.66 (dd, 1H), 7.63 (d, 1H), 7.43 (d, 2H), 7.24 (dd, 1H), 7.09 (dd, 1 H), 6.96 (dd, 1H), 4.93 (d, 2H), 4.77 (d, 2H), 3.03 (hept, 1H), 2.60 (t, 1H), 1.34 (d, 6H).
MS: 442 (M+1)⁺

### Example 114: 4-(4-Isopropyl-phenyl)-6-prop-2-ynyloxy-quinazoline-2-carboxylic acid 3-methoxy-phenyl ester

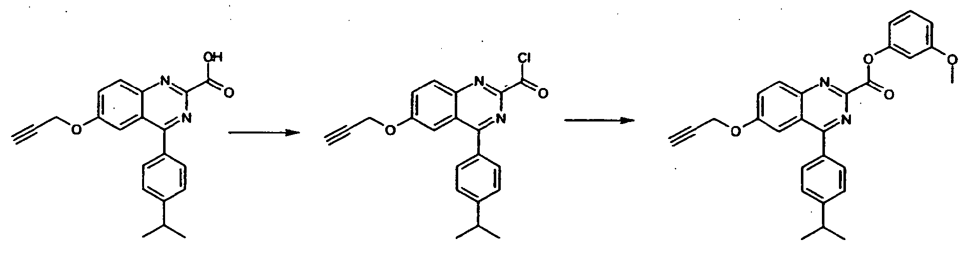

To a solution of 1.00 g (2.9 mmol)) 4-(4-isopropyl-phenyl)-6-propargyioxy-quinazoline-2-carboxylic acid in THF are added slowly 740 µl (8.7 mmol) oxalylic chloride. After 3 h at RT the solvent is evaporated and in order to remove residual oxalylic chloride, toluene is added and evaporated. A portion of the so prepared acid chloride [200 mg (0.55 mmol)] are dissolved in 0.5 ml dichloromethane before 94 µl (0.55 mmol) N-ethyldiisopropylamine and 68 mg (0.55 mmol) 3- methoxyphenol are added. After stirring overnight, 0.1 M hydrochloric acid is added and the reaction mixture is extracted with dichloromethane. The crude product is purified by flash chromatography using a ethyl acetate / hexane gradient.
¹H-NMR (400 MHz, CDCl₃): 8.33 (d, 1 H), 7.89 (d, 2H), 7.74 - 7.69 (m, 2H), 7.46 (d, 2H), 7.35 (t, 1 H), 6.95 (ddd, 1H), 6.92 (t, 1H), 6.86 (ddd, 1H), 4.82 (d, 2H), 3.84 (s, 3H), 3.06 (hept, 1 H), 2.65 (t, 1 H), 1.36 (d, 6H).
MS: 453 (M+1)⁺

### Example 115: 1-(4-Isopropyl-phenyl)-7-prop-2-ynyloxy-isoquinoline-3-carboxylic acid ethyl ester

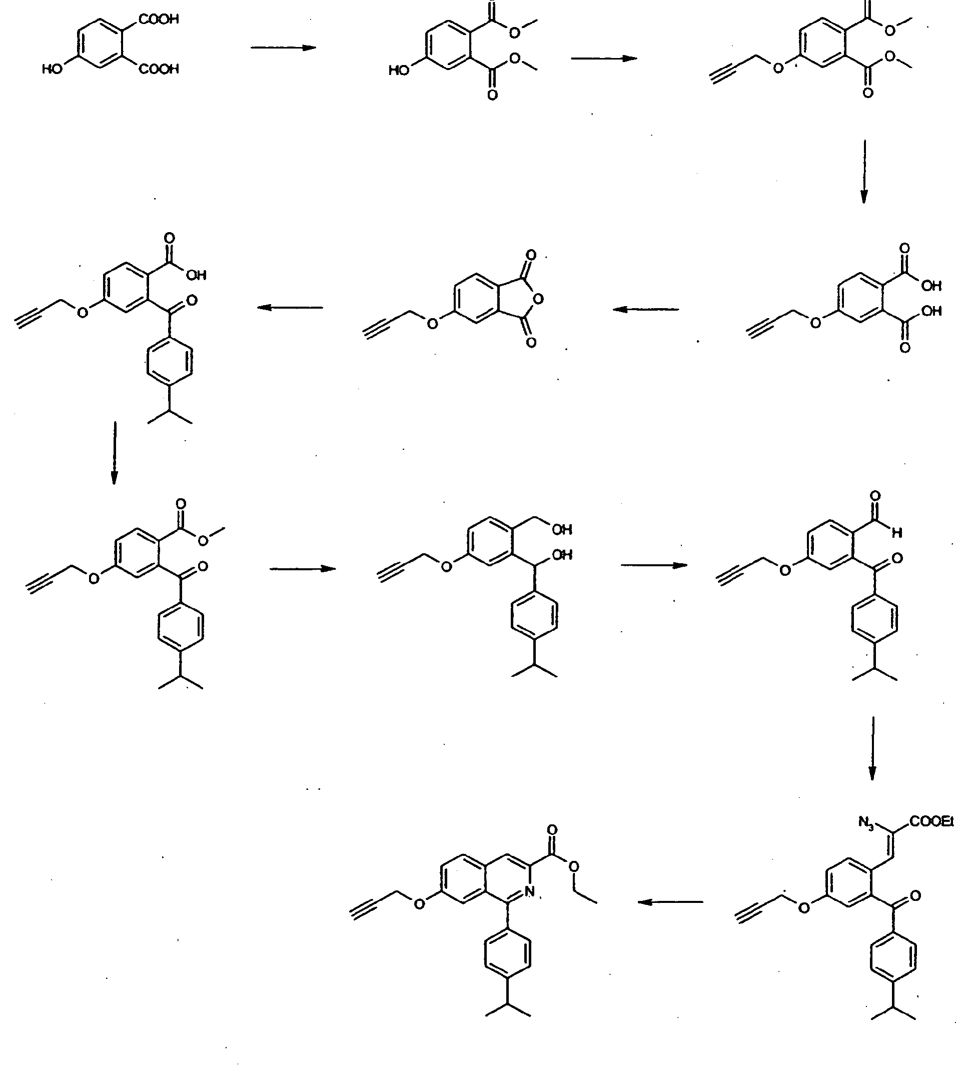

### A) Preparation of 4-hydroxy-phthalic acid dimethyl ester

A solution of 50 g (270 mmol) 4-hydroxy-phthalic acid and 7.4 ml concentrated sulfuric acid in 500 ml methanol is heated under reflux for 11 h. The methanol is evaporated and the residue dissolved in dichloromethane. Upon addition of hexane the dimethyl ester precipitates as white crystals.
Retention time: 1.60 min, MS: 211 (M+1)⁺

### B) Preparation of 4-prop-2-ynyloxy-phthalic acid dimethyl ester

To a solution of the whole amount of the product from above 105 g (1.1 mol) potassium carbonate (150 g, 1.1 mol) and 10 minutes later 43 ml (400 mmol) propargyl bromide (80% in toluene), are added. After stirring for 3 h at RT water is added and the reaction mixture is extracted with MTBE. After evaporation of the solvent the product is obtained that is used in the next step without purification.
Retention time: 2.15 min, MS: 249 (M+1)⁺

### C) Preparation of 4-prop-2-ynyloxy-phthalic acid

The product from above is dissolved in 500 ml methanol and treated with 31 g (780 mmol) sodium hydroxide dissolved in 100 ml water. After stirring overnight at RT the methanol is evaporated and the residues is taken up into water. Upon addition of concentrated hydrochloric acid at 0° C the free diacid precipitates which is dried in a vacuum oven at 70° C.
Retention time: 1.64 min, MS: 221 (M+1)⁺

### D) Preparation of 5-prop-2-ynyloxy-isobenzofuran-1,3-dione

The diacid from above (50 g, 230 mmol) is heated under reflux in 350 ml acetic anhydride for 24 h. The volatile components are evaporated and the remaining residue is dissolved in toluene and evaporated twice to remove residual acetic acid or anhydride.
A small sample is dissolved in MeOH and reacts to the corresponding mono methyl ester which is detected by HPLC-MS :
Retention time: 1.91 min, MS: 235 (M+1)⁺

### E) Preparation of 2-(4-isopropyl-benzoyl)-4-prop-2-ynyloxy-benzoic acid

A Grignard reagent is prepared in 400 ml THF from 66 g (330 mmol) 4-bromo-isopropylbenzene and 8.1 g Magnesium. Unreacted metallic magnesium is filtered off and the reagent solution is added dropwise at RT to a solution of 55.97 g (280 mmol) 5-prop-2-ynyloxy-isobenzofuran-1,3-dione in 400 ml THF. Cooling is applied to compensate for the exothermic reaction. Fifteen minutes after the end of the addition 500 ml saturated ammonium chloride solution are poured to the reaction mixture and THF is evaporated. The product is extracted with dichloromethane and purified by Flash chromatography using a ethyl acetate / hexane gradient.
¹H-NMR (300 MHz, CDCl₃): 10.52 (broad 1H), 8.04 (d, 1 H), 7.65 (d, 2H), 7.25 (d, 2H), 7.07 (dd, 1H), 6.86 (d, 1H), 4.73 (d, 2H), 2.53 (t, 1H), 1.26 (d, 6H).
Retention time: 2.42 min, MS: 323 (M+1)⁺

### F) Preparation of 2-(4-isopropyl-benzoyl)-4-prop-2-ynyloxy-benzoic acid methyl ester

A solution of 8.6 g (27 mmol) 2-(4-isopropyl-benzoyl)-4-prop-2-ynyloxy-benzoic acid and 710µl sulfuric acid in 50 ml methanol is heated at 60° C for 16 h. After evaporation of the solvent, water is added and the product is extracted with dichloromethane. HPLC retention time: 2.62 min, MS: 337 (M+1)⁺

### G) Preparation of (2-hydroxymethyl-5-prop-2-ynyloxy-phenyl)-(4-isopropyl-phenyl)-methanol

A solution of 9.14 g (27 mmol) 2-(4-isopropyl-benzoyl)-4-prop-2-ynyloxy-benzoic acid methyl ester in 60 ml THF is treated with 82 ml (82 mmol) of a solution of LiAlH₄ (1 M in THF). Cooling is applied to compensate for the exothermic reaction. Ten minutes after the end of the addition 3.37 ml water are dropped very slowly to the reaction mixture followed by 2.45 ml 20% NaOH. After addition of further 9.14 ml water and stirring for 1 h at RT a white powder can be filtered off. Water is added to the filtrate and the product is extracted with dichloromethane.
HPLC retention time: 2.36 min, MS: 293 (M-17)⁺

### H) Preparation of 2-(4-isopropyl-benzoyl)-4-prop-2-ynyloxy-benzaldehyde

To a solution of 14 g (63 mmol) pyridinium chloro chromate in 60 ml dichloromethane are added 6.5g (21 mmol) 2-hydroxymethyl-5-prop-2-ynyloxy-phenyl)-(4-isopropyl-phenyl)-methanol dissolved in 20 ml of the same solvent. After 30 minutes stirring at RT the reaction mixture is poured onto water and extracted with dichloromethane. The product is purified by flash chromatography using a ethyl acetate / hexane gradient followed by recrystallization from ethanol.
¹H-NMR (400 MHz, CDCl₃): 9.89 (s, 1H), 8.02 (d, 1 H), 7.75 (d, 2H), 7.31 (d, 2H), 7.22 (dd, 1H), 7.03 (d, 1H), 4.79 (d, 2H), 2.99 (hept, 1 H), 2.58 (t, 1H), 1.28 (d, 6H).
Retention time: 2.56 min, MS: 307 (M+1)⁺

### I) Preparation of (Z)-2-Azido-3-[2-(4-isopropyl-benzoyl)-4-prop-2-ynyloxy-phenyl]-acrylic acid ethyl ester

With 10 ml ethanol 4.8 ml of a sodium ethylate solution (21% in ethanol) is diluted. To this ethoxide solution is added at 0° C 1.00 g (3.3 mmol) 2-(4-isopropyl-benzoyl)-4-prop-2-ynyloxy-benzaldehyde dissolved in 7.8 ml (13 mmol) of a 25% solution of ethyl-azidoacetate in ethanol. After 1 h the temperature is allowed to reach RT and stirring is continued overnight. The crude product is obtained after addition of water and extraction with dichloromethane.
¹H-NMR (400 MHz, CDCl₃): 8.20 (d, 1H), 7.76 (d, 2H), 7.31 (d, 2H), 7.15 (dd, 1H), 7.00 (d, 1H), 4.72 (d, 2H), 4.20 (q, 2H), 2.98 (hept, 1H), 2.53 (t, 1H), 1.27 (d, 6H), 1.22 (t, 3H).
Retention time: 2.87 min, MS: 390 (M-28+1)⁺

### J) Preparation of 1-(4-Isopropyl-phenyl)-7-prop-2-ynyloxy-isoquinoline-3-carboxylic acid ethyl ester

A solution of the crude product from above in 50 ml toluene is treated with 2 ml (11 mmol) triethyl phosphite. After 2 h water is added and the reaction mixture is extracted with dichloromethane. The product is purified by flash chromatography using a ethyl acetate / hexane gradient.
¹H-NMR (400 MHz, CDCl₃): 8.47 (s, 1H), 7.94 (d, 1H), 7.70 (d, 2H), 7.45 (dd, 1 H), 7.64 (d, 1H), 7.37 (d, 2H), 4.72 (d, 2H), 4.50 (q, 2H), 3.00 (hept, 1H), 2.57 (t, 1H), 1.45 (t, 3H), 1.31 (d, 6H).
MS: 374 (M+1)⁺

### Example 116: 1-(4-Isopropyl-phenyl)-7-prop-2-ynyloxy-isoquinoline-3-carboxylic acid 1,2-dimethyl-propyl ester

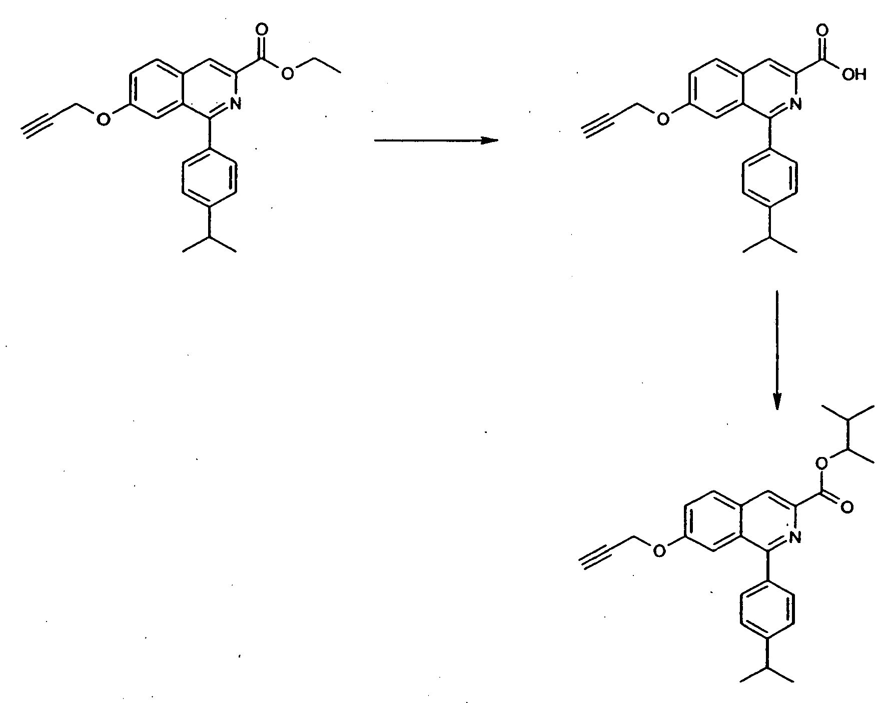

### A) 1-(4-Isopropyl-phenyl)-7-prop-2-ynyloxy-isoquinoline-3-carboxylic acid

To a solution of 1.5 g (4.0 mmol) 1-(4-isopropyl-phenyl)-7-prop-2-ynyloxy-isoquinoline-3-carboxylic acid ethyl ester in 10 ml ethanol are added 4 ml 2 M aqueous sodium hydroxide solution. After 1 h stirring at RT the reaction mixture is set acidic with 1 M hydrochloric acid and extracted with dichloromethane.
¹H-NMR (400 MHz, CDCl₃): 8.58 (s, 1H), 8.03 (d, 1H), 7.70 - 7.68 (m, 3H), 7.53 (dd, 1H), 7.45 (d, 2H), 4.77 (d, 2H), 3.06 (hept, 1H), 2.59 (t, 1H), 1.37 (d, 6H).
MS: 346 (M+1)⁺

### B) 1-(4-Isopropyl-phenyl)-7-prop-2-ynyloxy-isoquinoline-3-carboxylic acid 1,2-dimethyl-propyl ester

To a solution of 50 mg (0.14 mmol) of the acid from above in 1 ml dichloromethane are added 19 µl (0.22 mmol) oxalyl chloride. After 2 h stirring at RT 47 µl (0.43 mmol) 3-methyl-2-butanol are added. After completion of the reaction within 1 h, 1 ml DMSO is added and the reaction mixture is directly purified by preparative reversed phase HPLC.
¹H-NMR (400 MHz, CDCl₃): 8.40 (s, 1H), 7.94 (d, 1 H), 7.73 (d, 2H), 7.67 (d, 1H), 7.45 (dd, 1H), 7.37 (d, 2H), 5.09 (quint, 1H), 4.74 (d, 2H), 3.00 (hept, 1H), 2.58 (t, 1H), 2.01 (oct, 1H), 1.36 (d, 3H), 1.32 (d, 6H), 1.05 (d, 3H), 1.03 (d, 3H).
MS: 416 (M+1)⁺

The compounds of the following examples are prepared in an analogous manner using the appropriate starting materials:

### Example 117: 1-(4-Isopropyl-phenyl)-7-prop-2-ynyloxy-isoquinoline-3-carboxylic acid isobutyl ester

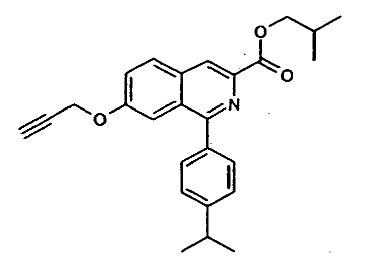

¹H-NMR (400 MHz, CDCl₃): 8.44 (s, 1H), 7.95 (d, 1H), 7.72 (d, 2H), 7.67 (d, 1H), 7.46 (dd, 1H), 7.38 (d, 2H), 4.74 (d, 2H), 4.23 (d, 2H), 3.01 (hept, 1H), 2.57 (t, 1H), 2.18 (non, 1H), 1.32 (d, 6H), 1.05 (d, 6H).
MS: 402 (M+1)⁺

### Example 118: 1-(4-Isopropyl-phenyl)-7-prop-2-ynyloxy-isoquinoline-3-carboxylic acid cyclopropylmethyl ester

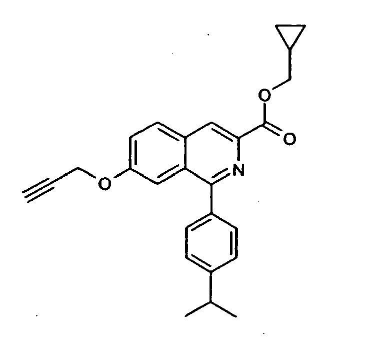

¹H-NMR (400 MHz, DMSO d₆): 8.59 (s, 1H), 8.27 (d, 1 H), 7.68 (d, 2H), 7.62 - 7.59 (m, 2H), 7.47 (d, 2H), 4.92 (d, 2H), 4.20 (d, 2H), 3.73 (t, 1H), 3.03 (hept, 1H), 1.30 (d, 6H), 0.61 - 0.56 (m, 2H), 0.41 - 0.37 (m, 2H).
MS: 400 (M+1)⁺

### Example 119: 1-(4-Isopropyl-phenyl)-7-prop-2-ynyloxy-isoquinoline-3-carboxylic acid benzyl ester

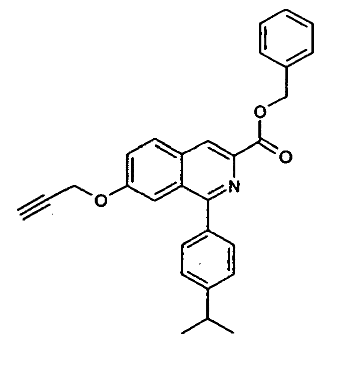

¹H-NMR (400 MHz, CDCl₃): 8.48 (s, 1 H), 7.94 (d, 1H), 7.71 (d, 2H), 7.66 (d, 1H), 7.53 (d, 2H), 7.47 (dd, 1H), 7.41 - 7.34 (m, 5H), 5.50 (s, 2H), 4.74 (d, 2H), 3.01 (hept, 1H), 2.57 (t, 1H), 1.32 (d, 6H).
MS: 436 (M+1)⁺

### Example 120: 1-(4-Isopropyl-phenyl)-7-prop-2-ynyloxy-isoquinoline-3-carboxylic acid 2-methoxy-benzyl ester

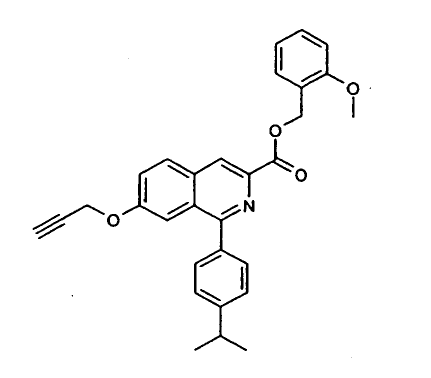

¹H-NMR (400 MHz, CDCl₃): 8.48 (s, 1H), 7.94 (d, 1H), 7.72 (d, 2H), 7.66 (d, 1H), 7.51 (dd, 1H), 7.47 (dd, 1H), 7.38 (d, 2H), 7.32 (ddd, 1H), 6.98 (td, 1 H), 6.92 (d, 1H), 5.55 (s, 2H), 4.74 (d, 2H), 3.87 (s, 3H), 3.01 (hept, 1H), 2.57 (t, 1H), 1.32 (d, 6H).
MS: 466 (M+1)⁺

### Example 121: 1-(4-Isopropyl-phenyl)-7-prop-2-ynyloxy-isoquinoline-3-carboxylic acid 3-methoxy-benzyl ester

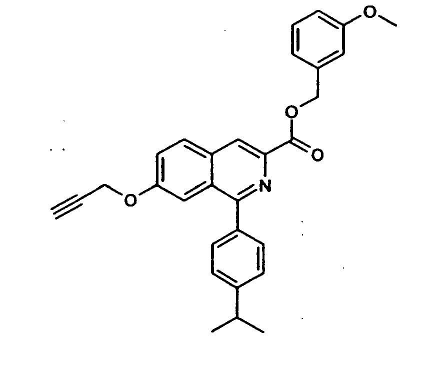

¹H-NMR (400 MHz, CDCl₃): 8.48 (s, 1 H), 7.94 (d, 1 H), 7.71 (d, 2H), 7.66 (d, 1 H), 7.46 (dd, 1H), 7.38 (d, 2H), 7.30 (t, 1H), 7.10 - 7.08 (m, 2H), 6.89 - 6.86 (m, 1H), 5.47 (s, 2H), 4.74 (d, 2H), 3.82 (s, 3H), 3.01 (hept, 1H), 2.57 (t, 1H), 1.32 (d, 6H).
MS: 466 (M+1)⁺

### Example. 122: 1-(4-Isopropyl-phenyl)-7-prop-2-ynyloxy-isoquinoline-3-carboxylic acid 4-methoxycarbonyl-benzyl ester

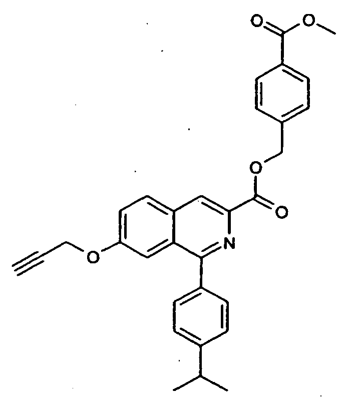

¹H-NMR (400 MHz, CDCl₃): 8.49 (s, 1H), 8.06 (d, 2H), 7.95 (d, 1 H), 7.71 (d, 2H), 7.67 (d, 1 H), 7.58 (d, 2H), 7.47 (dd, 1H), 7.39 (d, 2H), 5.54 (s, 2H), 4.75 (d, 2H), 3.92 (s, 3H), 3.02 (hept, 1 H), 2.58 (t, 1H), 1.33 (d, 6H).
MS: 494 (M+1)⁺

### Example 123: 1-(4-Isopropyl-phenyl)-7-prop-2-ynyloxy-isoquinoline-3-carboxylic acid phenethyl ester

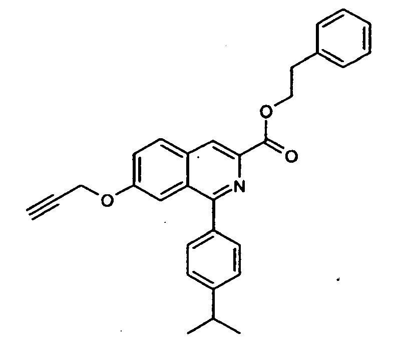

¹H-NMR (400 MHz, CDCl₃): 8.43 (s, 1H), 7.94 (d, 1H), 7.72 (d, 2H), 7.67 (d, 1H), 7.47 (dd, 1H), 7.39 (d, 2H), 7.37 - 7.30 (m, 4H), 7.26 - 7.22 (m, 1H), 4.75 (d, 2H), 4.64 (t, 2H), 3.16 (t, 2H), 3.02 (hept, 1H), 2.57 (t, 1H), 1.33 (d, 6H).
MS: 450 (M+1)⁺

### Example 124: 1-(4-Isopropyl-phenyl)-7-prop-2-ynyioxy-isoquinoline-3-carboxylic acid 1-phenyl-ethyl ester

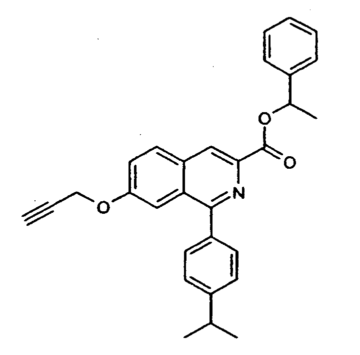

¹H-NMR (400 MHz, CDCl₃): 8.45 (s, 1H), 7.95 (d, 1 H), 7.73 (d, 2H), 7.67 (d, 1H), 7.53 (d, 2H), 7.46 (dd, 2H), 7.40 - 7.36 (m, 4H), 7.30 (tt, 1H), 6.26 (q, 1 H), 4.74 (d, 2H), 3.01 (hept, 1H), 2.58 (t, 1H), 1.75 (d, 3H), 1.32 (d, 6H).
MS: 450 (M+1)⁺

### Example 125: 1-(4-Isopropyl-phenyl)-7-prop-2-ynyloxy-isoquinoline-3-carboxylic acid [3-(2-hydroxy-ethanesulfonyl)-phenyl]-amide

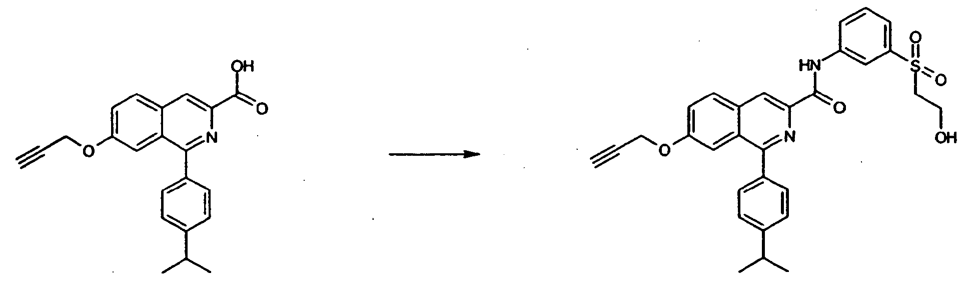

A solution of 75 mg (0.22 mmol) 1-(4-isopropyl-phenyl)-7-prop-2-ynyloxy-isoquinoline-3-carboxylic acid, 52 mg (0.22 mmol) 2-(3-aminophenylsulfonyl)ethanol 93 µl (0.54 mmol) N-ethyl-diisopropylamine and 140 mg (0.33 mmol) BOP in 1 ml THF is stirred for 1 h at RT. DMSO (1 ml) is added and the product is isolated by preparative reversed phase HPLC.
¹H-NMR (400 MHz, DMSO d₆): 10.80 (s, 1H), 8.66 (s, 1H), 8.49 (m, 1H), 8.31 (d, 1H), 8.29 - 8.27 (m, 1H), 7.90 (d, 2H), 7.69 (d, 1 H), 7.67 - 7.62 (m, 3H), 7.52 (d, 2H), 4.95 (d, 2H), 4.90 (t, 1H), 3.75 (t, 1H), 3.71 (q, 2H), 3.47 (t, 2H), 3.06 (hept, 1H), 1.33 (d, 6H).
MS: 529 (M+1)⁺

### Example 126: [1-(4-Isopropyl-phenyl)-7-prop-2-ynyloxy-isoquinolin-3-yl]-(3-methoxy-phenyl)-methanone

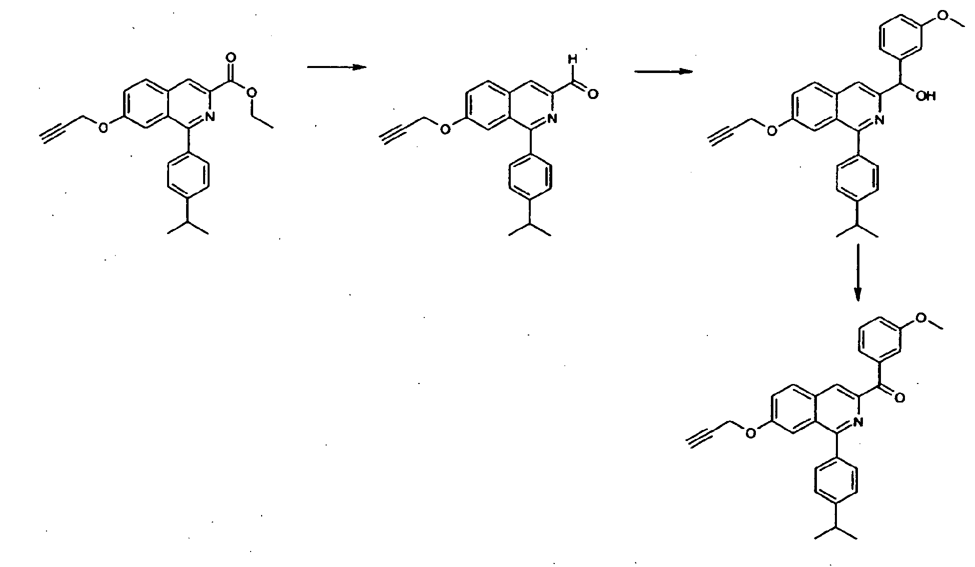

### A) 1-(4-Isopropyl-phenyl)-7-prop-2-ynyloxy-isoquinoline-3-carbaldehyde

To a solution of 1.1 g (2.9 mmol) 1-(4-Isopropyl-phenyl)-7-prop-2-ynyloxy-isoquinoline-3-carboxylic acid ethyl ester in 6 ml THF are added at -78° C 2.5 ml (2.9 mmol) 1.2 M DIBAH solution in toluene. The reaction mixture is allowed to reach RT and after the addition of water extracted with dichloromethane. Since a mixture of the corresponding alcohol and aldehyde is obtained the crude product dissolved in 5 ml dichloromethane is treated at RT with 1 g (4.6 mmol) pyridinium-chloro-chromate and stirred overnight. Water is added and the reaction mixture is extracted with dichloromethane. The product is purified by flash chromatography using a ethyl acetate / hexane gradient.

### B) [1-(4-Isopropyl-phenyl)-7-prop-2-ynyloxy-isoquinolin-3-yl]-(3-methoxy-phenyl)-methanol

The Grignard reagent prepared from 38 µl (0.3 mmol) 3-bromoanisole and 7.4 mg (0.3 mmol) magnesium in 0.2 ml THF is added at RT to a solution of 50 mg (0.15 mmol) of the aldehyde prepared above in 0.5 ml THF. After 10 minutes saturated ammonium chloride solution is added and the mixture is extracted with dichloromethane. The product is purified by preparative reversed phase HPLC.

### C) : [1-(4-Isopropyl-phenyl)-7-prop-2-ynyloxy-isoquinolin-3-yl]-(3-methoxy-phenyl)methanone

A solution of 25 mg (0.057 mmol) of the alcohol prepared above in 0.5 ml dichloromethane is treated with 22 µl (0.057 mmol) Jones reagent. After stirring overnight water is added and the product is extracted with dichloromethane and recrystallized from diethyl ether.
¹H-NMR (400 MHz, CDCl₃): 8.38 (s, 1H), 8.00 (d, 1H), 7.77 - 7.74 (m, 4H), 7.72 (d, 1H), 7.50 (dd, 1H), 7.41 - 7.37 (m, 3H), 7.14 (dd, 1H), 4.77 (d, 2H), 3.86 (s, 3H), 3.01 (hept, 1H), 2.59 (t, 1 H), 1.32 (d, 6H).
MS: 436 (M+1)⁺

The compound of the following examples are prepared in an analogous manner using the appropriate starting materials:

### Example 127: [1-(4-Isopropyl-phenyl)-7-prop-2-ynyloxy-isoquinolin-3-yl]-(4-methoxy-phenyl)-methanone

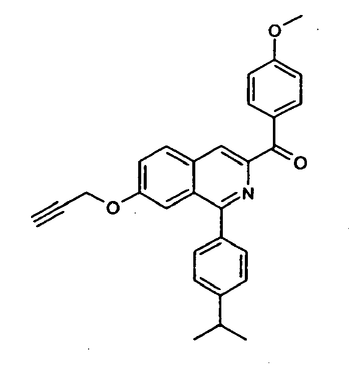

¹H-NMR (400 MHz, CDCl₃): 8.37 (s, 1H), 8.27 (d, 2H), 7.98 (d, 1H), 7.74 (d, 2H), 7.70 (d, 1 H), 7.47 (dd, 1H), 7.39 (d, 2H), 6.98 (d, 2H), 4.76 (d, 2H), 3.89 (s, 3H), 3.01 (hept, 1 H), 2.59 (t, 1H), 1.33 (d, 6H).
MS: 436 (M+1)⁺

The Agents of the Invention, as defined above, e.g., of formula (I), particularly as exemplified, in free or pharmaceutically acceptable acid addition salt form, exhibit pharmacological activity and are useful as pharmaceuticals, e.g. for therapy, in the treatment of diseases and conditions as hereinafter set forth.

### Assay for intracellular free calcium:

A method to determine antagonism at the PcaR consists in measuring the inhibition of intracellular calcium transients stimulated by extracellular calcium.

CCL39 fibroblasts stably transfected with human PcaR are seeded at 40'000 cells /well into 96-well Viewplates and incubated for 24 hours. Medium is then removed and replaced with fresh medium containing 2 µM Fluo-3 AM (Molecular Probes, Leiden, The Netherlands), In routine experiments, cells are incubated at 37°C, 5 % CO₂ for 1 h. Afterwards, plates are washed twice with mHBS and wells are refilled with 100 µl mHBS containing the test compounds. Incubation is continued at room temperature for 15 minutes. To record changes of intracellular free calcium, plates are transferred to fluorescence-imaging plate reader (Molecular Devices, Sunnyvale, CA, USA). A baseline consisting in 5 measurements of 0.4 seconds each (laser excitation 488 nm) is recorded. Cells are then stimulated with calcium (2.5 mM final), and fluorescence changes recorded over a period of 3 minutes.

When measured in the above assays, Agents of the Invention typically have IC₅₀s in the range from about 1000 nM down to about 1 nM or less.

It is now well established that controlled treatment of patients with parathyroid hormone (PTH) and analogues and fragments thereof can have a pronounced anabolic effect on bone formation. Thus compounds which promote PTH release, such as the Agents of the Invention may be used for preventing or treating conditions of bone which are associated with increased calcium depletion or resorption or in which stimulation of bone formation and calcium fixation in the bone is desirable.

The invention allows for a method for preventing or treating bone conditions which are associated with increased calcium depletion or resorption or in which stimulation of bone formation and calcium fixation in the bone is desirable in which an effective amount of an Agent of the Invention is administered to a patient in need of such treatment.

In a further aspect the invention includes a pharmaceutical composition for preventing or treating bone conditions which are associated with increased calcium depletion or resorption or in which stimulation of bone formation and calcium fixation in the bone is desirable comprising an Agent of the Invention in admixture with a pharmaceutically acceptable excipient, diluent or carrier.

Agents of the Invention are accordingly indicated for preventing or treating all bone conditions which are associated with increased calcium depletion or resorption or in which stimulation of bone formation and calcium fixation in the bone is desirable, e.g. osteoporosis of various genesis (e.g. juvenile, menopausal, post-menopausal, post-traumatic, caused by old age or by corticosteroid therapy or inactivity), fractures, osteopathy, including acute and chronic states associated with skeletal demineralisation, osteo-malacia, periodontal bone loss or bone loss due to arthritis or osteoarthritis or for treating hypoparathyroidism.

Further diseases and disorders which might be prevented or treated include e.g. seizures, stroke, head trauma, spinal cord injury, hypoxia-induced nerve cell damage such as in cardiac arrest or neonatal distress, epilepsy, neurodegenerative diseases such as Alzheimer's disease, Huntington's disease and Parkinson's disease, dementia, muscle tension, depression, anxiety, panic disorder, obsessive-compulsive disorder, post-traumatic stress disorder, schizophrenia, neuroleptic malignant syndrome, congestive heart failure; hypertension; gut motility disorders such as diarrhoea, and spastic colon and dermatological disorders, e.g. in tissue healing, for example burns, ulcerations and wounds.

The Agents of the Invention are particularly indicated for preventing or treating osteoporosis of various genesis.

For all the above uses, an indicated daily dosage is in the range from about 0.03 to about 300 mg preferably 0.03 to 30, more preferably 0.1 to 10 mg of a compound of the invention. Agents of the Invention may be administered twice a day or up to twice a week.

The Agents of the Invention may be administered in free form or in pharmaceutically acceptable salt form. Such salts may be prepared in conventional manner and exhibit the same order of activity as the free compounds. The present invention also provides a pharmaceutical composition comprising an Agent of the Invention in free base form or in pharmaceutically acceptable salt form in association with a pharmaceutically acceptable diluent or carrier. Such compositions may be formulated in conventional manner. The Agents of the Invention may be administered by any conventional route, for example parenterally e.g. in form of injectable solutions, microemulsions or suspensions, enterally, e.g. orally, for example in the form of tablets or capsules or in a transdermal, nasal or a suppository form.

According to a further embodiment of the invention, the Agents of the Invention may be employed as adjunct or adjuvant to other therapy, e.g. a therapy using a bone resorption inhibitor, for example as in osteoporosis therapy, in particular a therapy employing calcium, a calcitonin or an analogue or derivative thereof, e.g. salmon, eel or human calcitonin, a steroid hormone, e.g. an estrogen, a partial estrogen agonist or estrogen-gestagen combination, a SERM (Selective Estrogen Receptor Modulator) e.g. raloxifene, lasofoxifene, bazedoxifene, arzoxifene, FC1271, Tibolone (Livial ®), a RANKL antibody, e.g. denosumab, a cathepsin K inhibitor, vitamin D or an analogue thereof or PTH, a PTH fragment or a PTH derivative e.g. PTH (1-84), PTH (1-34), PTH (1-36), PTH (1-38), PTH (1-31)NH₂ or PTS 893.

When the Agents of the Invention are administered in conjunction with, e.g. as an adjuvant to bone resorption inhibition therapy, dosages for the co-administered inhibitor will of course vary depending on the type of inhibitor drug employed, e.g. whether it is a steroid or a calcitonin, on the condition to be treated, whether it is a curative or preventive therapy, on the regimen and so forth.

In accordance with the foregoing the present invention further provides:
a) an Agent of the Invention or a pharmaceutically acceptable salt thereof for use as a pharmaceutical;
b) an Agent of the Invention or a pharmaceutically acceptable salt thereof for use in the preparation of a pharmaceutical composition e.g. for use in the method for preventing or treating above mentioned disorders and diseases in a subject in need of such treatment, which method comprises administering to said subject an effective amount of an Agent of the Invention or a pharmaceutically acceptable salt thereof.

According to a further embodiment of the invention, the Agents of the Invention may be employed as adjunct or adjuvant to other therapy, e.g. a therapy using a bone resorption inhibitor, for example as in osteoporosis therapy, in particular a therapy employing calcium, a calcitonin or an analogue or derivative thereof, e.g. salmon, eel or human calcitonin, a steroid hormone, e.g. an estrogen, a partial estrogen agonist or estrogen-gestagen combination, a SERM (Selective Estrogen Receptor Modulator) e.g. raloxifene, lasofoxifene, TSE-424, FC1271, Tibolone (Livial ®), vitamin D or an analogue thereof or PTH, a PTH fragment or a PTH derivative e.g. PTH (1-84), PTH (1-34), PTH (1-36), PTH (1-38), PTH (1-31)NH₂ or PTS 893.

When the Agents of the Invention are administered in conjunction with, e.g. as an adjuvant to bone resorption inhibition therapy, dosages for the co-administered inhibitor will of course vary depending on the type of inhibitor drug employed, e.g. whether it is a steroid or a calcitonin, on the condition to be treated, whether it is a curative or preventive therapy, on the regimen and so forth.

## Claims

1. A compound of formula (I) or a pharmaceutically acceptable salt or prodrug ester thereof: 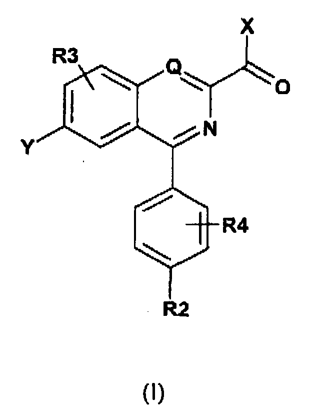 wherein: "
Q is CH or N;
R2 is C₁-C₄ alkyl;
Y is R5-O-;
where R5 is ethynyl or propynyl;
X is selected from the group consisting of aryl, heteroaryl, C₁-C₁₀ alkyl, C₁-C₁₀ alkyloxy, cycloalkyl, heterocycloalkyl, aryl C₁-C₄ alkyl, heteroaryl C₁-C₄ alkyl, cycloalkyl C₁-C₄ alkyl, heterocycloalkyl C₁-C₄ alkyl, arylamino, heteroarylamino, aryl C₁-C₄ alkylamino, heteroaryl C₁-C₄ alkylamino, C₁-C₆ alkylamino, C₁-C₆ dialkylamino, aryloxy, heteroaryloxy, aryl C₁-C₄ alkyloxy, heteroaryl C₁-C₄ alkyloxy, cycloalkyl C₁-C₄ alkylamino, heterocycloalkyl C₁-C₄ alkylamino, cycloalkyl C₁-C₄ alkyloxy or heterocycloalkyl C₁-C₄ alkyloxy each of which is optionally substituted once or more,
the optional substituent or substituents on X being independently selected from the group consisting of halo, cyano, trifluoromethyl, nitro, hydroxy and optionally substituted (C₁-C₄ alkyl, C₁-C₄ alkyloxy, amino, sulfanyl, sulfonyl, oxycarbonyl, hydroxyl, sulfinyl, aminosulfonyl, sulfonylamino, carbonyl, carbonyloxy, carbonyl amino, carboxyl, acyl, acylamino, or carbamoyl), the optional substituent or substituents being selected from C₁-C₆ alkyl, C₁-C₆ alkyloxy, carboxyl, hydroxyl, hydroxy C₁-C₄ alkyl; each of which in turn may be optionally substituted by C₁-C₆ alkyloxy, C₁-C₆ alkyl, C₁-C₃ fluorinated alkyl, C₁-C₆ alkyloxy, carboxyl, hydroxyl, hydroxy C₁-C₄ alkyl, halo, cyano, nitro; and
R3 and R4 each represent one or more substituents independently selected from: H, halo, C₁-C₄ alkyl, C₁-C₄alkyloxy and CF₃,
the optional substituent or substituents on R3 or R4 being independently selected from the group consisting of C₁-C₄ alkyl, halo, C₁-C₄ alkyloxy, cyano, sulfanyl, sulfonyl, amino, oxycarbonyl and hydroxyl, which may in turn be optionally substituted once or more by C₁-C₄ alkyl, halo, C₁-C₄ alkyloxy, cyano, sulfanyl, sulfonyl, amino, oxycarbonyl or hydroxyl.

2. A compound of formula (I') according to claim 1 or a pharmaceutically acceptable salt or prodrug ester thereof: 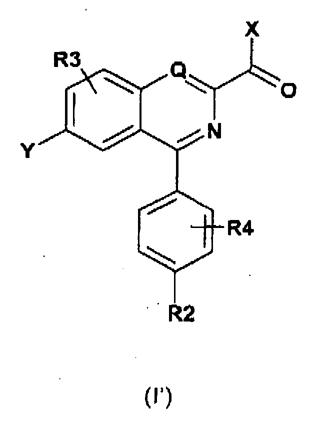 wherein:
Q is CH or N;
R2 is C₁-C₄ alkyl;
Y is R5-O-,
where R5 is ethynyl or propargyl;
X is selected from the group consisting of aryl, heteroaryl, C₁-C₆ alkyl, cycloalkyl, heterocycloalkyl, aryl C₁-C₄ alkyl, heteroaryl C₁-C₄ alkyl, arylamino, aryl C₁-C₄ alkylamino, heteroaryl C₁-C₄ alkylamino, C₁-C₆ alkylamino, C₁-C₆ dialkylamino amino, aryloxy, heteroaryloxy, aryl C₁-C₄ alkyloxy, or heteroaryl C₁-C₄ alkyloxy, each of which is optionally substituted once or more,
the optional substituent or substituents on X being independently selected from the group consisting of C₁-C₄ alkyl, halo, C₁-C₄ alkyloxy, cyano, trifluoromethyl, hydroxy, amino, nitro and C₁-C₄alkyl substituted (sulfanyl, sulfonyl, amino, oxycarbonyl, hydroxyl, sulfinyl, carbonyl, carboxyl or carbamoyl); and
R3 and R4 each represent one or more substituents independently selected from: H, halo, optionally substituted C₁-C₄ alkyl and optionally substituted C₁-C₄ alkyloxy,
the optional substituent or substituents on R3 or R4 being independently selected from the group consisting of C₁-C₄ alkyl, halo, C₁-C₄ alkyloxy, cyano, sulfanyl, sulfonyl, amino, oxycarbonyl and hydroxyl.

3. A compound according to claim 1 having the formula (II) or a pharmaceutically acceptable salt or prodrug ester thereof: 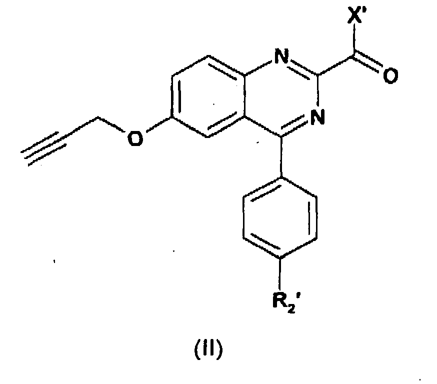 wherein:
X' is selected from the group consisting of aryl, heteroaryl, cycloalkyl, heterocycloalkyl, -C₁-C₄ alkylaryl, -C₁-C₄ alkylheteroaryl, arylamino, heteroarylamino, aryl C₁-C₄ alkylamino, heteroaryl C₁-C₄ alkylamino, aryloxy, heteroaryloxy, aryl C₁-C₄ alkyloxy, heteroaryl C₁-C₄ alkyloxy, aryl C₁-C₄ alkyl, heteroaryl C₁-C₄ alkyl, C₁-C₆ alkyl, -C₁-C₄ alkylamino or amino, each of which is optionally substituted once or more,
the optional substituent or substituents on X' being independently selected from the group consisting of halo, cyano, trifluoromethyl, nitro, hydroxy and optionally substituted (C₁-C₄ alkyl, C₁-C₄ alkyloxy, amino, sulfanyl, sulfonyl, amino, oxycarbonyl, hydroxyl, sulfinyl, carbonyl, carboxyl, acyl, acylamino or carbamoyl); the optional substituent or substituents being selected from C₁-C₆ alkyl, C₁-C₆ alkyloxy, carboxyl, hydroxyl and hydroxy C₁-C₄ alkyl; each of which in turn may be optionally substituted by C₁-C₆ alkyloxy, C₁-C₆ alkyl, C₁-C₆ alkyloxy, carboxyl, hydroxyl, hydroxy C₁-C₄ alkyl, halo, cyano, nitro, and
R₂' is C₁-C₄ alkyl.

4. A compound according to claim 3 wherein R₂' is isopropyl.

5. A compound according to claim 2 or 3 wherein X' is optionally substituted (aryl, heteroaryl, heterocycloalkyl, arylamino, heteroarylamino, aryl C₁-C₄ alkylamino, heteroaryl C₁-C₄ alkylamino, aryloxy, heteroaryloxy, C₁-C₆ alkyloxy, aryl C₁-C₄ alkyloxy, heteroaryl C₁-C₄ alkyloxy), wherein the substituents are as defined in claim 2 or claim 3, respectively.

6. A compound according to any one of the preceding claims selected from the following:
(4-tert-Butyl-phenyl)-[4-(4-isopropyl-phenyl)-6-propargyloxy-quinazolin-2-yl]-methanone
[4-(4-Isopropyl-phenyl)-6-propargyloxy-quinazolin-2-yl]-phenyl-methanone
(2-Methoxy-phenyl)-[4-(4-Isopropyl-phenyl)-6- propargyloxy -quinazolin-2-yl]-methanone
(3-Methoxy-phenyl)-[4-(4-Isopropyl-phenyl)-6- propargyloxy -quinazolin-2-yl]-methanone
(4-Methoxy-phenyl)-[4-(4-Isopropyl-phenyl)-6- propargyloxy -quinazolin-2-yl]-methanone
(4-F'uoro-phenyl)-[4-(4-isopropyl-phenyl)-6-propargyloxy-quinazolin-2-yl]-methanone
(3-Fluoro-phenyl)-[4-(4-isopropyl-phenyl)-6-propargyloxy-quinazolin-2-yl]-methanone
(3-Chloro-phenyl)-[4-(4-isopropyl-phenyl)-6-propargyloxy-quinazolin-2-yl]-methanone
(4-Chloro-phenyl)-[4-(4-isopropyl-phenyl)-6-propargyloxy-quinazolin-2-yl]-methanone
(4-Fluoro-phenyl)-[4-(4-tert.butyl-phenyl)-6-propargyloxy-quinazolin-2-yl]-methanone
(3-Fluoro-phenyl)-[4-(4-tert.butyl-phenyl)-6-propargyloxy-quinazolin-2-yl]-methanone
(3-Bromo-phenyl)-[4-(4-isopropyl-phenyl)-6-propargyloxy-quinazolin-2-yl]-methanone
(4-Bromo-phenyl)-[4-(4-isopropyl-phenyl)-6-propargyloxy-quinazolin-2-yl]-methanone
(4-Methyl-phenyl)-[4-(4-isopropyl-phenyl)-6-propargyloxy-quinazolin-2-yl]-methanone
(4-Isopropyl-phenyl)-[4-(4-isopropyl-phenyl)-6-propargyloxy-quinazolin-2-yl]-methanone
(4-Ethyl-phenyl)-[4-(4-isopropyl-phenyl)-6-propargyloxy-quinazolin-2-yl]-methanone
(4-Propyl-phenyl)-[4-(4-isopropyl-phenyl)-6-propargyloxy-quinazolin-2-yl]-methanone
(4-Cyano-phenyl)-[4-(4-isopropyl-phenyl)-6-propargyloxy-quinazolin-2-yl]-methanone
(4-Methylthio-phenyl)-[4-(4-isopropyl-phenyl)-6-propargyloxy-quinazolin-2-yl]-methanone
(4-Methansulfonyl-phenyl)-[4-(4-isopropyl-phenyl)-6-propargyloxy-quinazolin-2-yl]-methanone
(4-Dimethylamino-phenyl)-[4-(4-isopropyl-phenyl)-6-propargyloxy-quinazolin-2-yl]-methanone
(4-Ethoxy-phenyl)-[4-(4-isopropyl-phenyl)-6-propargyloxy-quinazolin-2-yl]-methanone
4-[4-(4-Isopropyl-phenyl)-6-propargyloxy-quinazoline-2-carbonyl]-benzoic acid methyl ester
(4-Dimethylamino-phenyl)-[4-(4-tert.butyl-phenyl)-6-propargyloxy-quinazolin-2-yl]-methanone
4-[4-(4-Isopropyl-phenyl)-6-propargyloxy-quinazoline-2-carbonyl]-benzoic acid ethyl ester
(4-Methoxy-phenyl)-[4-(4-tert.butyl-phenyl)-6-propargyloxy-quinazolin-2-yl]-methanone
(3-Ethoxy-4-methoxy-phenyl)-[4-(4-isopropyl-phenyl)-6-propargyloxy-quinazolin-2-yl]-methanone
(4-tert.Butyloxy-phenyl)-[4-(4-isopropyl-phenyl)-6-propargyloxy-quinazolin-2-yl]-methanone
(4-Hydroxy)-[4-(4-isopropyl-phenyl)-6-propargyloxy-quinazolin-2-yl]-methanone
(4-Butyloxy)-[4-(4-isopropyl-phenyl)-6-propargyloxy-quinazolin-2-yl]-methanone
Furan-2-yl-[4-(4-isopropyl-phenyl)-6-propargyloxy-quinazolin-2-yl]-methanone
Furan-3-yl-[4-(4-tert.butyl-phenyl)-6-propargyloxy-quinazolin-2-yl]-methanone
Furan-3-yl-[4-(4-isopropyl-phenyl)-6-propargyloxy-quinazolin-2-yl]-methanone
Thiophen-2-yl-[4-(4-isopropyl-phenyl)-6-propargyloxy-quinazolin-2-yl]-methanone
(3-Methyl-thiophen-2-yl-[4-(4-isopropyl-phenyl)-6-propargylaxy-quinazolin-2-yl]-methanone
Benz[b]thiophen-2-yl-[4-(4-isopropyl-phenyl)-6-propargyloxy-quinazolin-2-yl]-methanone
Thiophen-3-yl-[4-(4-isopropyl-phenyl)-6-propargyloxy-quinazolin-2-yl]-methanone
(1-Methyl-1H-pyrrol)-2-yl-[4-(4-isopropyl-phenyl)-6-propargyloxy-quinazolin-2-yl]-methanone
4-(4-Isopropyl-phenyl)-6-propargyloxy-quinazoline-2-carboxylic acid ethyl ester
[4-(4-isopropyl-phenyl)-6-propargyloxy-quinazolin-2-yl]-pyridine-3-yl-methanone
[4-(4-Isopropyl-phenyl)-6-propargyloxy-quinazolin-2-yl]-naphthalen-1-yl-methanone
[4-(4-Isopropyl-phenyl)-6-propargyloxy-naphathalen-2-yl]-methanone
Benzothiazol-2-yl-[4-(4-isopropyl-phenyl)-6-propargyloxy-quinazolin-2-yl]-methanone
[4-(4-Isopropyl-phenyl)-6-propargyloxy-quinazolin-2-yl]-thiazol-5-yl-methanone
[4-(4-Isopropyl-phenyl)-6-propargyloxy-quinazolin-2-yl]-piperidin-1-yl-methanone
4-(4-Isopropyl-phenyl)-6-prop-2-ynyloxy-quinazoline-2-carboxylic acid (3-chloro-phenyl)-amide
4-(4-Isopropyl-phenyl)-6-prop-2-ynyloxy-quinazoline-2-carboxylic acid (3-methoxy-phenyl)-amide
4-(4-Isopropyl-phenyl)-6-prop-2-ynyloxy-3,4-dihydro-quinazoline-2-carboxylic acid (3-methylsulfanyl-phenyl)-amide
4-(4-Isopropyl-phenyl)-6-prop-2-ynyloxy-3,4-dihydro-quinazoline-2-carboxylic acid (3-methanesulfonyl-phenyl)-amide
4-(4-lsopropyl-phenyl)-6-prop-2-ynyloxy-quinazoline-2-carboxylic acid (3-trifluoromethylsulfanyl-phenyl)-amide
4-(4-Isopropyl-phenyl)-6-prop-2-ynyloxy-3,4-dihydro-quinazoline-2-carboxylic acid (3-sulfamoyl-phenyl)-amide
4-(4-Isopropyl-phenyl)-6-prop-2-ynyioxy-3,4-dihydro-quinazoline-2-carboxylic acid [3-(2-hydroxy-ethanesulfonyl)-phenyl]-amide
4-(4-Isopropyl-phenyl)-6-prop-2-ynyloxy-3,4-dihydro-quinazoline-2-carboxylic acid (5-ethanesulfonyl-2-hydroxy-phenyl)-amide
4-(4-Isopropyl-phenyl)-6-prop-2-ynyloxy-quinazoline-2-carboxylic acid (3-nitro-phenyl)-amide
4-(4-Isopropyl-phenyl)-6-prop-2-ynyloxy-quinazoline-2-carboxylic acid (3-cyano-phenyl)-amide
3-{[4-(4-Isopropyl-phenyl)-6-prop-2-ynyloxy-quinazoline-2-carbonyl]-amino}-benzoic acid methyl ester
3-{[4-(4-Isopropyl-phenyl)-6-prop-2-ynyloxy-quinazoline-2-carbonyl]-amino}-benzoic acid ethyl ester
3-{[4-(4-Isopropyl-phenyl)-6-prop-2-ynyloxy-quinazoline-2-carbonyl]-amino}-benzoic acid isopropyl ester
3-{[4-(4-Isopropyl-phenyl)-6-prop-2-ynyloxy-quinazoline-2-carbonyl]-amino}-benzoic acid tert-butyl ester
4-(4-Isopropyl-phenyl)-6-prop-2-ynyloxy-quinazoline-2-carboxylic acid (3-carbamoyl-phenyl)-amide
4-(4-Isopropyl-phenyl)-6-prop-2-ynyloxy-quinazoline-2-carboxylic acid (3-acetyl-phenyl)-amide
3-{[4-(4-Isopropyl-phenyl)-6-prop-2-ynyloxy-quinazoline-2-carbonyl]-amino}-5-methoxybenzoic acid methyl ester
4-(4-Isopropyl-phenyl)-6-prop-2-ynyloxy-quinazoline-2-carboxylic acid (3-methylcarbamoylphenyl)-amide
4-(4-Isopropyl-phenyl)-6-prop-2-ynyloxy-quinazoline-2-carboxylic acid (3-tert-butylcarbamoylphenyl)-amide
4-(4-Isopropyl-phenyl)-6-prop-2-ynyloxy-quinazoline-2-carboxylic acid (3-dimethylcarbamoyl-5-trifluoromethyl-phenyl)-amide
3-{[4-(4-Isopropyl-phenyl)-6-prop-2-ynyloxy-quinazoline-2-carbonyl]-amino}-5-trifluoromethylbenzoic acid methyl ester
3-{[4-(4-Isopropyl-phenyl)-6-prop-2-ynyloxy-quinazoline-2-carbonyl]-amino}-5-trifluoromethylbenzoic acid isopropyl ester
2-Fluoro-5-{[4-(4-isopropyl-phenyl)-6-prop-2-ynyloxy-quinazoline-2-carbonyl]-amino}-benzoic acid methyl ester
2-Fluoro-5-{[4-(4-isopropyl-phenyl)-6-prop-2-ynyloxy-quinazoline-2-carbonyl]-amino}-benzoic acid isopropyl ester
2-Chloro-5-{[4-(4-isopropyl-phenyl)-6-prop-2-ynyloxy-quinazoline-2-carbonyl]-amino}-benzoic acid methyl ester
2,5-Dichloro-3-{[4-(4-isopropyl-phenyl)-6-prop-2-ynyloxy-quinazoline-2-carbonyl]-amino}-benzoic acid methyl ester
2,5-Dichloro-3-{[4-(4-isopropyl-phenyl)-6-prop-2-ynyloxy-quinazoline-2-carbonyl]-amino}-benzoic acid isopropyl ester
4-(4-Isopropyl-phenyl)-6-prop-2-ynyloxy-quinazoline-2-carboxylic acid (3-cyano-5-fluorophenyl)-amide
4-(4-Isopropyl-phenyl)-6-prop-2-ynyloxy-quinazoline-2-carboxylic acid (3,4-dicyano-phenyl)-amide
4-(4-Isopropyl-phenyl)-6-prop-2-ynyloxy-quinazoline-2-carboxylic acid (4-cyano-3-trifluoromethyl-phenyl)-amide
4-(4-Isopropyl-phenyl)-6-prop-2-ynyloxy-quinazoline-2-carboxylic acid (3-trifluoromethylphenyl)-amide
4-(4-Isopropyl-phenyl)-6-prop-2-ynyloxy-quinazoline-2-carboxylic acid (4-acetylamino-3-trifluoromethyl-phenyl)-amide
4-(4-Isopropyl-phenyl)-6-prop-2-ynyloxy-quinazoline-2-carboxylic acid (3-methoxy-5-trifluoromethyl-phenyl)-amide
4-(4-Isopropyl-phenyl)-6-prop-2-ynyloxy-quinazoline-2-carboxylic acid (3,5-bis-trifluoromethyl-phenyl)-amide
4-(4-Isopropyl-phenyl)-6-prop-2-ynyloxy-quinazoline-2-carboxylic acid (3-fluoro-5-trifluoromethyl-phenyl)-amide
4-(4-Isopropyl-phenyl)-6-prop-2-ynyloxy-quinazoline-2-carboxylic acid (4-fluoro-3-trifluoromethyl-phenyl)-amide
3-{[4-(4-Isopropyl-phenyl)-6-prop-2-ynyloxy-quinazoline-2-carbonyl]-amino}-2-methyl-benzoic acid methyl ester
3-{[4-(4-Isopropyl-phenyl)-6-prop-2-ynyloxy-quinazoline-2-carbonyl]-amino}-4-methyl-benzoic acid methyl ester
3-{[4-(4-Isopropyl-phenyl)-6-prop-2-ynyloxy-quinazoline-2-carbonyl]-amino}-4-methoxybenzoic acid methyl ester
5-{[4-(4-Isopropyl-phenyl)-6-prop-2-ynyloxy-quinazoline-2-carbonyl]-amino}-isophthalic acid dimethyl ester
4-{[4-(4-Isopropyl-phenyl)-6-prop-2-ynyloxy-quinazoline-2-carbonyl]-amino}-phthalic acid dimethyl ester
4-(4-Isopropyl-phenyl)-6-prop-2-ynyloxy-quinazoline-2-carboxylic acid (3,5-dichloro-phenyl)-amide
4-(4-Isopropyl-phenyl)-6-prop-2-ynyloxy-quinazoline-2-carboxylic acid (3,4-dichloro-phenyl)-amide
4-(4-Isopropyl-phenyl)-6-prop-2-ynyloxy-quinazoline-2-carboxylic acid (3-chloro-4-fluorophenyl)-amide
4-(4-Isopropyl-phenyl)-6-prop-2-ynyloxy-quinazoline-2-carboxylic acid (4-chloro-3-trifluoromethyl-phenyl)-amide
5-{[4-(4-Isopropyl-phenyl)-6-prop-2-ynyloxy-quinazoline-2-carbonyl]-amino}-pyridine-2-carboxylic acid methyl ester
5-{[4-(4-Isopropyl-phenyl)-6-prop-2-ynyloxy-quinazoline-2-carbonyl]-amino}-nicotinic acid methyl ester
5-{[4-(4-Isopropyl-phenyl)-5-prop-2-ynyloxy-quinazoline-2-carbonyl]-amino}-nicotinic acid isopropyl ester
[4-(4-Isopropyl-phenyl)-6-prop-2-ynyloxy-quinazolin-2-yl]-pyrrol-1-yl-methanone
4-(4-Isopropyl-phenyl)-6-prop-2-ynyloxy-quinazoline-2-carboxylic acid (5-methyl-1H-pyrazol-3-yl)-amide
(2-{[4-(4-Isopropyl-phenyl)-6-prop-2-ynyloxy-quinazoline-2-carbonyl]-a mino}-thiazol-4-yl)-acetic acid ethyl ester
4-(4-Isopropyl-phenyl)-6-prop-2-ynyloxy-quinazoline-2-carboxylic acid naphthalen-1-ylamide
4-(4-Isopropyl-phenyl)-6-prop-2-ynyloxy-quinazoline-2-carboxylic acid isoquinolin-8-ylamide
4-(4-Isopropyl-phenyl)-6-prop-2-ynyloxy-quinazoline-2-carboxylic acid phthalazin-5-ylamide
4-(4-Isopropyl-phenyl)-6-prop-2-ynyloxy-quinazoline-2-carboxylic acid quinolin-5-ylamide
4-(4-Isopropyl-phenyl)-6-prop-2-ynyloxy-quinazoline-2-carboxylic acid quinolin-8-ylamide
4-(4-Isopropyl-phenyl)-6-prop-2-ynyloxy-quinazoline-2-carboxylic acid isoquinolin-4-ylamide
4-(4-Isopropyl-phenyl)-6-prop-2-ynyloxy-quinazoline-2-carboxylic acid (5-acetyl-quinolin-8-yl)-amide
4-(4-Isopropyl-phenyl)-6-prop-2-ynyloxy-quinazoline-2-carboxylic acid (3-bromo-6-methoxy-quinolin-8-yl)-amide
4-(4-Isopropyl-phenyl)-6-prop-2-ynyloxy-quinazoline-2-carboxylic acid quinolin-2-ylamide
4-(4-Isopropyl-phenyl)-6-prop-2-ynyloxy-quinazoline-2-carboxylic acid quinolin-6-ylamide
4-(4-Isopropyl-phenyl)-6-prop-2-ynyloxy-quinazoline-2-carboxylic acid (2-methyl-quinolin-6-yl)-amide
(6-{[4-(4-Isopropyl-phenyl)-6-prop-2-ynyloxy-quinazoline-2-carbonyl]-amino}-quinolin-8-yloxy)-acetic acid ethyl ester
4-(4-Isopropyl-phenyl)-6-prop-2-ynyloxy-quinazoline-2-carboxylic acid (1H-benzoimidazol-4-yl)-amide
4-(4-Isopropyl-phenyl)-6-prop-2-ynyloxy-quinazoline-2-carboxylic acid benzothiazol-2-ylamide
4-(4-Isopropyl-phenyl)-6-prop-2-ynyloxy-quinazoline-2-carboxylic acid (benzo[1,3]dioxol-5-ylmethyl)-amide
4-(4-Isopropyl-phenyl)-6-prop-2-ynyloxy-quinazoline-2-carboxylic acid (thiophen-2-ylmethyl)-amide
4-(4-Isopropyl-phenyl)-6-prop-2-ynyloxy-quinazoline-2-carboxylic acid 3-methoxy-phenyl ester
1-(4-Isopropyl-phenyl)-7-prop-2-ynyloxy-isoquinoline-3-carboxylic acid ethyl ester
1-(4-Isopropyl-phenyl)-7-prop-2-ynyloxy-isoquinoline-3-carboxylic acid 1,2-dimethyl-propyl ester
1-(4-Isopropyl-phenyl)-7-prop-2-ynyloxy-isoquinoline-3-carboxylic acid isobutyl ester
1-(4-Isopropyl-phenyl)-7-prop-2-ynyloxy-isoquinoline-3-carboxylic acid cyclopropylmethyl ester
1-(4-Isopropyl-phenyl)-7-prop-2-ynyloxy-isoquinoline-3-carboxylic acid benzyl ester
1-(4-Isopropyl-phenyl)-7-prop-2-ynyloxy-isoquinoline-3-carboxylic acid 2-methoxy-benzyl ester
1-(4-Isopropyl-phenyl)-7-prop-2-ynyloxy-isoquinoline-3-carboxylic acid 3-methoxy-benzyl ester
1-(4-Isopropyl-phenyl)-7-prop-2-ynyloxy-isoquinoline-3-carboxylic acid 4-methoxycarbonylbenzyl ester
1-(4-Isopropyl-phenyl)-7-prop-2-ynyloxy-isoquinoline-3-carboxylic acid phenethyl ester
1-(4-Isopropyl-phenyl)-7-prop-2-ynyloxy-isoquinoline-3-carboxylic acid 1-phenyl-ethyl ester
1-(4-Isopropyl-phenyl)-7-prop-2-ynyloxy-isoquinoline-3-carboxylic acid [3-(2-hydroxy-ethanesulfonyl)-phenyl]-amide
[1-(4-Isopropyl-phenyl)-7-prop-2-ynyloxy-isoquinolin-3-yl]-(3-methoxy-phenyl)-methanone
[1-(4-Isopropyl-phenyl)-7-prop-2-ynyloxy-isoquinolin-3-yl]-(4-methoxy-phenyl)-methanone.

7. A compound selected from the group consisting of
(4-Dimethylamino-phenyl)-[4-(4-cyclopropyl-phenyl)-6-propargyloxy-quinazolin-2-yl]-methanone
and
(4-Ethoxy-phenyl)-[4-(4-cyclopropyl-phenyl)-6-propargyloxy-quinazolin-2-yl]-methanone

8. A pharmaceutical composition comprising a compound of formula (I) in association with a pharmaceutically acceptable excipient, diluent or carrier.

9. A compound of formula (I) for promoting the release of parathyroid hormone.

10. A process for preparation of a compound of formula (I) according to claim 1 or claim 7 in free or salt form, comprising the step of:
(i) for cases where Q is N, reacting a compound of formula (III) with a compound of formula (IV) and an ammonium salt in the presence of a suitable solvent: 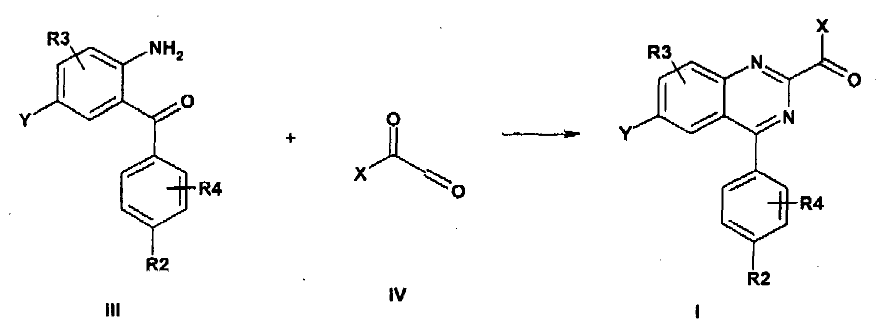 or
(ii) reacting a compound of formula V 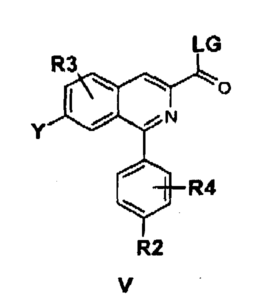
wherein LG represents a suitable leaving group;
with an organometallic reagent of formula VI:
X-Met VI
under suitable anhydrous conditions; or
(iii) reacting a compound of formula Va 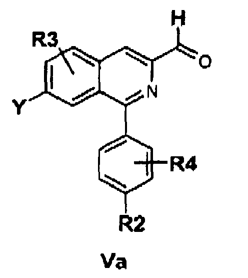 with an organometallic reagent of formula VI:
X-Met VI
under suitable anhydrous conditions followed by oxidation to the carbonyl compound by an appropriate oxidation agent; or
(iv) reacting a compound of formula VII 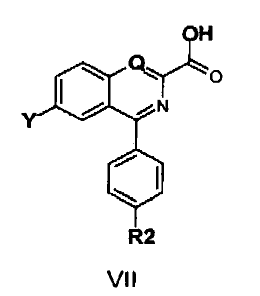 with a compound X-H wherein the H forms part of an amino or hydroxy group, the reaction being carried out in the presence of a coupling reagent; or
(v) reacting a compound of formula VIII 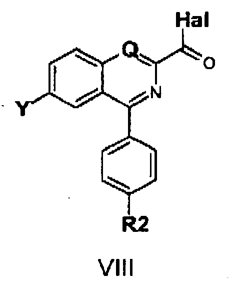
wherein Hal is halogen or a leaving group
with a compound X-H wherein the H forms part of an amino or hydroxy group, the reaction being carried out in the presence of a coupling reagent.

11. Use of a compound of formula (I) according to claim 1 or 7 in the manufacture of a medicament for preventing or treating bone conditions which are associated with increased calcium depletion or resorption or in which stimulation of bone formation and calcium fixation in the bone is desirable.

12. A combination comprising a therapeutically effective amount of a compound according to any one of claims 1 to 7 and a second drug substance selected from: calcium, a calcitonin or an analogue or derivative thereof, a steroid hormone, a partial estrogen agonist or estrogen-gestagen combination, a SERM (Selective Estrogen Receptor Modulator), a RANKL antibody, a cathepsin K inhibitor, vitamin D or an analogue thereof or PTH, a PTH fragment or a PTH derivative for simultaneous, separate or sequential treatment.

## Patentansprüche

1. Verbindung der Formel (I) oder ein pharmazeutisch unbedenkliches Salz oder ein pharmazeutisch unbedenklicher Prodrug-Ester davon: 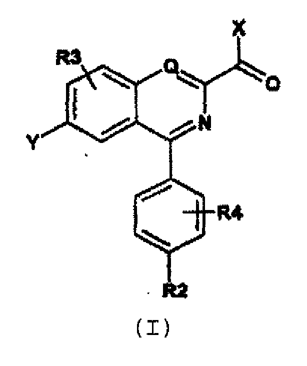 worin:
Q für CH oder N steht;
R2 für C₁-C₄-Alkyl steht;
Y für R5-O- steht;
wobei R5 für Ethinyl oder Propinyl steht;
X aus der Gruppe bestehend aus Aryl, Heteroaryl, C₁-C₁₀-Alkyl, C₁-C₁₀-Alkyloxy, Cycloalkyl, Heterocycloalkyl, Aryl-C₁-C₄-alkyl, Heteroaryl-C₁-C₄-alkyl, Cycloalkyl-C₁-C₄-alkyl, Heterocycloalkyl-C₁-C₄-alkyl, Arylamino, Heteroarylamino, Aryl-C₁-C₄-alkylamino, Heteroaryl-C₁-C₄-alkylamino, C₁-C₆-Alkylamino, C₁-C₆-Dialkylamino, Acyloxy, Heteroaryloxy, Aryl-C₁-C₄-alkyloxy, Heteroaryl-C₁-C₄-alkyloxy, Cycloalkyl-C₁-C₄-alkylamino, Heterocycloalkyl-C₁-C₄-alkylamino, Cycloalkyl-C₁-C₄-alkyloxy oder Heterocycloalkyl-C₁-C₄-alkyloxy ausgewählt ist, wobei jede dieser Gruppen gegebenenfalls ein- oder mehrfach substituiert ist,
der fakultative Substituent bzw. die fakultativen Substituenten an X unabhängig voneinander aus der Gruppe bestehend aus Halogen, Cyano, Trifluormethyl, Nitro, Hydroxy und gegebenenfalls substituiertem (C₁-C₄-Alkyl, C₁-C₄-Alkyloxy, Amino, Sulfanyl, Sulfonyl, Oxycarbonyl, Hydroxyl, Sulfinyl, Aminosulfonyl, Sulfonylamino, Carbonyl, Carbonyloxy, Carbonylamino, Carboxyl, Acyl, Acylamino oder Carbamoyl) ausgewählt sind, wobei der fakultative Substituent bzw. die fakultativen Substituenten aus C₁-C₆-Alkyl, C₁-C₆-Alkyloxy, Carboxyl, Hydroxyl, Hydroxy-C₁-C₄-alkyl ausgewählt sind, wobei jede dieser Gruppen wiederum gegebenenfalls durch C₁-C₆-Alkyloxy, C₁-C₆-Alkyl, C₁-C₃-Fluoralkyl, C₁-C₆-Alkyloxy, Carboxyl, Hydroxyl, Hydroxy-C₁-C₄-alkyl, Halogen, Cyano, Nitro substituiert sein kann; und
R3 und R4 jeweils für einen oder mehrere unabhängig voneinander aus H, Halogen, C₁-C₄-Alkyl, C₁-C₄-Alkyloxy und CF₃ ausgewählte Substituenten stehen,
wobei der fakultative Substituent bzw. die fakultativen Substituenten an R3 oder R4 unabhängig voneinander aus der Gruppe bestehend aus C₁-C₄-Alkyl, Halogen, C₁-C₄-Alkyloxy, Cyano, Sulfanyl, Sulfonyl, Amino, Oxycarbonyl und Hydroxyl ausgewählt sind, die wiederum gegebenenfalls ein- oder mehrfach durch C₁-C₄-Alkyl, Halogen, C₁-C₄-Alkyloxy, Cyano, Sulfanyl, Sulfonyl, Amino, Oxycarbonyl oder Hydroxyl substituiert sein können.

2. Verbindung der Formel (I') nach Anspruch 1 oder ein pharmazeutisch unbedenkliches Salz oder ein pharmazeutisch unbedenklicher Prodrug-Ester davon: 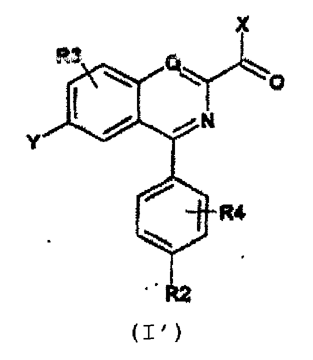 worin:
Q für CH oder N steht;
R2 für C₁-C₄-Alkyl steht;
Y für R5-O- steht;
wobei R5 für Ethinyl oder Propargyl steht;
X aus der Gruppe bestehend aus Aryl, Heteroaryl, C₁-C₆-Alkyl, Cycloalkyl, Heterocycloalkyl, Aryl-C₁-C₄-alkyl, Heteroaryl-C₁-C₄-alkyl, Arylamino, Aryl-C₁-C₄-alkylamino, Heteroaryl-C₁-C₄-alkylamino, C₁-C₆-Alkylamino, C₁-C₆-Dialkylaminoamino, Aryloxy, Heteroaryloxy, Aryl-C₁-C₄-alkyloxy oder Heteroaryl-C₁-C₄-alkyloxy ausgewählt ist, wobei jede dieser Gruppen gegebenenfalls ein- oder mehrfach substituiert ist,
wobei der fakultative Substituent bzw. die fakultativen Substituenten an X unabhängig voneinander aus der Gruppe bestehend aus C₁-C₄-Alkyl, Halogen, C₁-C₄-Alkyloxy, Cyano, Trifluormethyl, Hydroxy, Amino, Nitro und substituiertem C₁-C₄-Alkyl (Sulfanyl, Sulfonyl, Amino, Oxycarbonyl, Hydroxyl, Sulfinyl, Carbonyl, Carboxyl oder Carbamoyl) ausgewählt sind; und
R3 und R4 jeweils für einen oder mehrere unabhängig voneinander aus H, Halogen, gegebenenfalls substituiertem C₁-C₄-Alkyl und gegebenenfalls substituiertem C₁-C₄-Alkyloxy ausgewählt sind,
wobei der fakultative Substituent bzw. die fakultativen Substituenten an R3 oder R4 unabhängig voneinander aus der Gruppe bestehend aus C₁-C₄-Alkyl, Halogen, C₁-C₄-Alkyloxy, Cyano, Sulfanyl, Sulfonyl, Amino, Oxycarbonyl und Hydroxyl ausgewählt sind.

3. Verbindung nach Anspruch 1 der Formel (II) oder ein pharmazeutisch unbedenkliches Salz oder ein pharmazeutisch unbedenklicher Prodrug-Ester davon: 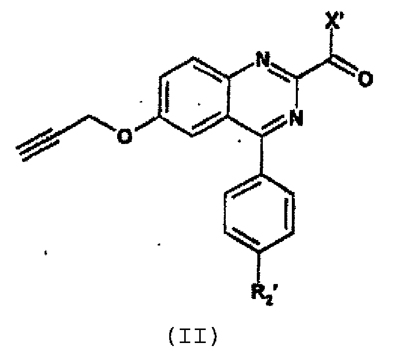 worin:
X' aus der Gruppe bestehend aus Aryl, Heteroaryl, Cycloalkyl, Heterocycloalkyl, -C₁-C₄-Alkylaryl, -C₁-C₄-Alkylheteroaryl, Arylamino, Heteroarylamino, Aryl-C₁-C₄-alkylamino, Heteroaryl-C₁-C₄-alkylamino, Aryloxy, Heteroaryloxy, Aryl-C₁-C₄-alkyloxy, Heteroaryl-C₁-C₄-alkyloxy, Aryl-C₁-C₄-alkyl, Heteroaryl-C₁-C₄-alkyl, C₁-C₆-Alkyl, -C₁-C₄-Alkylamino oder Amino ausgewählt ist, wobei jede dieser Gruppen gegebenenfalls ein- oder mehrfach substituiert ist,
wobei der fakultative Substituent bzw. die fakultativen Substituenten an X' unabhängig voneinander aus der Gruppe bestehend aus Halogen, Cyano, Trifluormethyl, Nitro, Hydroxy und gegebenenfalls substituiertem (C₁-C₄-Alkyl, C₁-C₄-Alkyloxy, Amino, Sulfanyl, Sulfonyl, Amino, Oxycarbonyl, Hydroxyl, Sulfinyl, Carbonyl, Carboxyl, Acyl, Acylamino oder Carbamoyl) ausgewählt sind; wobei der fakultative Substituent bzw. die fakultativen Substituenten aus C₁-C₆-Alkyl, C₁-C₆-Alkyloxy, Carboxyl, Hydroxyl und Hydroxy-C₁-C₄-alkyl ausgewählt sind; wobei jede dieser Gruppen wiederum gegebenenfalls durch C₁-C₆-Alkyloxy, C₁-C₆-Alkyl, C₁-C₆-Alkyloxy, Carboxyl, Hydroxyl, Hydroxy-C₁-C₄-alkyl, Halogen, Cyano, Nitro substituiert sein kann, und
R₂' für C₁-C₄-Alkyl steht.

4. Verbindung nach Anspruch 3, worin R₂' für Isopropyl steht.

5. Verbindung nach Anspruch 2 oder 3, worin X' für gegebenenfalls substituiertes (Aryl, Heteroaryl, Heterocycloalkyl, Arylamino, Heteroarylamino, Aryl-C₁-C₄-alkylamino, Heteroaryl-C₁-C₄-alkylamino, Aryloxy, Heteroaryloxy, C₁-C₆-Alkyloxy, Aryl-C₁-C₄-alkyloxy, Heteroaryl-C₁-C₄-alkyloxy) steht, wobei die Substituenten die in Anspruch 2 bzw. Anspruch 3 angegebene Bedeutung besitzen.

6. Verbindung nach einem der vorhergehenden Ansprüche, ausgewählt aus den folgenden:
(4-tert-Butylphenyl)-[4-(4-isopropylphenyl)-6-propargyloxychinazolin-2-yl]methanon
[4-(4-Isopropylphenyl)-6-propargyloxychinazolin-2-yl]phenylmethanon
(2-Methoxyphenyl)-[4-(4-isopropylphenyl)-6-propargyloxychinazolin-2-yl]methanon
(3-Methoxyphenyl)-[4-(4-isopropylphenyl)-6-propargyloxychinazolin-2-yl]methanon
(4-Methoxyphenyl)-[4-(4-isopropylphenyl)-6-propargyloxychinazolin-2-yl]methanon
(4-Fluorphenyl)-[4-(4-isopropylphenyl)-6-propargyloxychinazolin-2-yl]methanon
(3-Fluorphenyl)-[4-(4-isopropylphenyl)-6-propargyloxychinazolin-2-yl]methanon
(3-Chlorphenyl)-[4-(4-isopropylphenyl)-6-propargyloxychinazolin-2-yl]methanon
(4-Chlorphenyl)-[4-(4-isopropylphenyl)-6-propargyloxychinazolin-2-yl]methanon
(4-Fluorphenyl)-[4-(4-tert.-butylphenyl)-6-propargyloxychinazolin-2-yl]methanon
(3-Fluorphenyl)-[4-(4-tert.-butylphenyl)-6-propargyloxychinazolin-2-yl]methanon
(3-Bromphenyl)-[4-(4-isopropylphenyl)-6-propargyloxychinazolin-2-yl]methanon
(4-Bromphenyl)-[4-(4-isopropylphenyl)-5-propargyloxychinazolin-2-yl]methanon
(4-Methylphenyl)-[4-(4-isopropylphenyl)-6-propargyloxychinazolin-2-yl]methanon
(4-Isopropylphenyl)-[4-(4-isopropylphenyl)-6-propargyloxychinazolin-2-yl]methanon
(4-Ethylphenyl)-[4-(4-isopropylphenyl)-6-propargyloxychinazolin-2-yl]methanon
(4-Propylphenyl)-[4-(4-isopropylphenyl)-6-propargyloxychinazolin-2-yl]methanon
(4-Cyanophenyl)-[4-(4-isopropylphenyl)-6-propargyloxychinazolin-2-yl]methanon
(4-Methylthiophenyl)-[4-(4-isopropylphenyl)-6-propargyloxychinazolin-2-yl]methanon
(4-Methansulfonylphenyl)-[4-(4-isopropylphenyl)-6-propargyloxychinazolin-2-yl]methanon
(4-Dimethylaminophenyl)-[4-(4-isopropylphenyl)-6-propargyloxychinazolin-2-yl]methanon
(4-Ethoxyphenyl)-[4-(4-isopropylphenyl)-6-propargyloxychinazolin-2-yl]methanon
4-[4-(4-Isopropylphenyl)-6-propargyloxychinazolin-2-carbonyl]benzoesäuremethylester
(4-Dimethylaminophenyl)-[4-(4-tert.-butylphenyl)-6-propargyloxychinazolin-2-yl]methanon
4-[4-(4-Isopropylphenyl)-6-propargyloxychinazolin-2-carbonyl]benzoesäureethylester
(4-Methoxyphenyl)-[4-(4-tert.-butylphenyl)-6-propargyloxychinazolin-2-yl]methanon
(3-Ethoxy-4-methoxyphenyl)-[4-(4-isopropylphenyl)-6-propargyloxychinazolin-2-yl]methanon
(4-tert.-Beutyloxyphenyl)-[4-(4-isopropylphenyl)-6-propargyloxychinazolin-2-yl]methanon
(4-Hydroxy)-[4-(4-isopropylphenyl)-6-propargyloxychinazolin-2-yl]methanon
(4-Butyloxy)-[4-(4-isopropylphenyl)-6-propargyloxychinazolin-2-yl]methanon
Furan-2-yl-[4-(4-isopropylphenyl)-6-propargyloxychinazolin-2-yl]methanon
Furan-3-yl-[4-(4-tert.-butylphenyl)-6-propargyloxychinazolin-2-yl]methanon
Furan-3-yl-[4-(4-isopropylphenyl)-6-propargyloxychinazolin-2-yl]methanon
Thiophen-2-yl-[4-(4-isopropylphenyl)-6-propargyloxychinazolin-2-yl]methanon
(3-Methylthiophen-2-y1-[4-(4-isopropylphenyl)-6-propargyloxychinazolin-2-yl]methanon
Benz[b]thiophen-2-yl-[4-(4-isopropylphenyl)-6-propargyloxychinazolin-2-yl]methanon
Thiophen-3-yl-[4-(4-isopropylphenyl)-6-propargyloxychinazolin-2-yl]methanon
(1-Methyl-1H-pyrrol)-2-yl-[4-(4-isopropylphenyl)-6-propargyloxychinazolin-2-yl]methanon
4-(4-Isopropylphenyl)-6-propargyloxychinazolin-2-carbonsäureethylester
[4-(4-Isopropylphenyl)-6-propargarloxychinazolin-2-yl]pyridin-3-ylmethanon
[4-(4-Isopropylphenyl)-6-propargyloxychinazolin-2-yl]naphthalin-1-ylmethanon
[4-(4-Isopropylphenyl)-6-propargyloxynaphthalin-2-yl]methanon
Benzothiazol-2-yl-[4-(4-isopropylphenyl)-6-propargyloxychinazolin-2-yl]methanon
[4-(4-Isopropylphenyl)-5-propargyloxychinazolin-2-yl]thiazol-5-ylmethanon
[4-(4-Isopropylphenyl)-6-propargyloxychinazolin-2-yl]piperidin-1-ylmethanon
4-(4-Isopropylphenyl)-6-prop-2-inyloxychinazolin-2-carbonsäure-(3-chlorphenyl)amid
4-(4-Isopropylphenyl)-6-prop-2-inyloxychinazolin-2-carbonsäure-(3-methoxyphenyl)amid
4-(4-Isopropylphenyl)-6-prop-2-inyloxy-3,4-dihydrochinazolin-2-carbonsäure-(3-methylsulfanylphenyl)amid
4-(4-Isopropylphenyl)-6-prop-2-inyloxy-3,4-dihydrochinazolin-2-carbonsäure-(3-methansulfonylphenyl)amid
4-(4-Isopropylphenyl)-6-prop-2-inyloxychinazolin-2-carbonsäure-(3-trifluormethylsulfanylphenyl)amid
4-(4-Isopropylphenyl)-6-prop-2-inyloxy-3,4-dihydrochinazolin-2-carbonsäure-(3-sulfamoylphenyl)amid
4-(4-Isopropylphenyl)-6-prop-2-inyloxy-3,4-dihydrochinazolin-2-carbonsäure-[3-(2-hydroxyethansulfonyl)phenyl]amid
4-(4-Isopropylphenyl)-6-prop-2-inyloxy-3,4-dihydrochinazolin-2-carbonsäure-(5-ethansulfonyl-2-hydroxyphenyl)amid
4-(4-Isopropylphenyl)-6-prop-2-inyloxychinazolin-2-carbonsäure-(3-nitrophenyl)amid
4-(4-Isopropylphenyl)-6-prop-2-inyloxychinazolin-2-carbonsäure-(3-cyanophenyl)amid
3-{[4-(4-Isopropylphenyl)-6-prop-2-inyloxy-chinazolin-2-carbonyl]amino}benzoesäuremethylester
3-{[4-(4-Isopropylphenyl)-6-prop-2-inyloxy-chinazolin-2-carbonyl]amino}benzoesäureethylester
3-{[4-(4-Isopropylphenyl)-6-prop-2-inyloxy-chinazolin-2-carbonyl]amino}benzoesäureisopropylester
3-{[4-(4-Isopropylphenyl)-6-prop-2-inyloxy-chinazolin-2-carbonyl]amino}benzoesäure-tert-butylester
4-(4-Isopropylphenyl)-6-prop-2-inyloxychinazolin-2-carbonsäure-(3-carbamoylphenyl)amid
4-(4-Isopropylphenyl)-6-prop-2-inyloxychinazolin-2-carbonsäure-(3-acetylphenyl)amid
3-{[4-(4-Isopropylphenyl)-6-prop-2-inyloxy-chinazolin-2-carbonyl]amino}-5-methoxybenzoesäuremethylester
4-(4-Isopropylphenyl)-6-prop-2-inyloxychinazolin-2-carbonsäure(3-methylcarbamoylphenyl)amid
4-(4-Isopropylphenyl)-6-prop-2-inyloxychinazolin-2-carbonsäure(3-tert-butylcarbamoylphenyl)amid
4-(4-Isopropylphenyl)-6-prop-2-inyloxychinazolin-2-carbonsäure(3-dimethylcarbamoyl-5-trifluormethylphenyl)amid
3-{[4-(4-Isopropylphenyl)-6-prop-2-inyloxy-chinazolin-2-carbonyl]amino}-5-trifluormethylbenzoesäuremethylester
3-{[4-(4-Isopropylphenyl)-6-prop-2-inyloxy-chinazolin-2-carbonyl]amino}-5-trifluormethylbenzoesäureisopropylester
2-Fluor-5-{[4-(4-isopropylphenyl)-6-prop-2-inyl-oxychinazolin-2-carbonyl]amino}benzoesäure-methylester
2-Fluor-5-{[4-(4-isopropylphenyl)-6-prop-2-inyl-oxychinazolin-2-carbonyl]amino}benzoesäure-isopropylester
2-Chlor-5-{[4-(4-isopropylphenyl)-6-prop-2-inyl-oxychinazolin-2-carbonyl]amino}benzoesäure-methylester
2,5-Dichlor-3-{[4-(4-isopropylphenyl)-6-prop-2-inyloxychinazolin-2-carbonyl]amino}benzoesäure-methylester
2,5-Dichlor-3-{[4-(4-isopropylphenyl)-6-prop-2-inyloxychinazolin-2-carbonyl]amino}benzoesäure-isopropylester
4-(4-Isopropylphenyl)-6-prop-2-inyloxychinazolin-2-carbonsäure(3-cyano-5-fluorphenyl)amid
4-(4-Isopropylphenyl)-6-prop-2-inyloxychinazolin-2-carbonsäure(3,4-dicyanophenyl)amid
4-(4-Isopropylphenyl)-6-prop-2-inyloxychinazolin-2-carbonsäure(4-cyano-3-trifluormethylphenyl)amid
4-(4-Isopropylphenyl)-6-prop-2-inyloxychinazolin-2-carbonsäure(3-trifluormethylphenyl)amid
4-(4-Isopropylphenyl)-6-prop-2-inyloxychinazolin-2-carbonsäure(4-acetylamino-3-trifluormethylphenyl)amid
4-(4-Isopropylphenyl)-6-prop-2-inyloxychinazolin-2-carbonsäure(3-methoxy-5-trifluormethylphenyl)amid
4-(4-Isopropylphenyl)-6-prop-2-inyloxychinazolin-2-carbonsäure(3,5-bis-trifluormethylphenyl)amid
4-(4-Isopropylphenyl)-6-prop-2-inyloxychinazolin-2-carbonsäure(3-fluor-5-trifluormethylphenyl)amid
4-(4-Isopropylphenyl)-6-prop-2-inyloxychinazolin-2-carbonsäure(4-fluor-3-trifluormethylphenyl)amid
3-{[4-(4-Isopropylphenyl)-6-prop-2-inyloxy-chinazolin-2-carbonyl]amino}-2-methylbenzoesäure-methylester
3-{[4-(4-Isopropylphenyl)-6-prop-2-inyloxy-chinazolin-2-carbonyl]amino}-4-methylbenzoesäure-methylester
3-{[4-(4-Isopropylphenyl)-6-prop-2-inyloxy-chinazolin-2-carbonyl]amino}-4-methoxybenzoesäuremethylester
5-{[4-(4-Isopropylphenyl)-6-prop-2-inyloxy-chinazolin-2-carbonyl]amino}isophthalsäure-diethylester
4-{[4-(4-Isopropylphenyl)-6-prop-2-inyloxy-chinazolin-2-carbonyl]amino}phthalsäure-dimethylester
4-(4-Isopropylphenyl)-6-prop-2-inyloxychinazolin-2-carbonsäure(3,5-dichlorphenyl)amid
4-(4-Isopropylphenyl)-6-prop-2-inyloxychinazolin-2-carbonsäure(3,4-dichlorphenyl)amid
4-(4-Isopropylphenyl)-6-prop-2-inyloxychinazolin-2-carbonsäure(3-chlor-4-fluorphenyl)amid
4-(4-Isopropylphenyl)-6-prop-2-inyloxychinazolin-2-carbonsäure(4-chlor-3-trifluormethylphenyl)amid
5-{[4-(4-Isopropylphenyl)-6-prop-2-inyloxy-chinazolin-2-carbonyl]amino}pyridin-2-carbonsäuremethylester
5-{[4-(4-Isopropylphenyl)-6-prop-2-inyloxy-chinazolin-2-carbonyl]amino}nicotinsäure-methylester
5-{[4-(4-Isopropylphenyl)-6-prop-2-inyloxy-chinazolin-2-carbonyl]amino}nicotinsäure-isopropylester
[4-(4-Isopropylphenyl)-6-prop-2-inyloxychinazolin-2-yl]pyrrol-1-ylmethanon
4-(4-Isopropylphenyl)-6-prop-2-inyloxychinazolin-2-carbonsäure(5-methyl-1H-pyrazol-3-yl)amid
(2-{[4-(4-Isopropylphenyl)-6-prop-2-inyloxy-chinazolin-2-carbonyl]amino}thiazol-4-yl)essigsäureethylester
4-(4-Isopropylphenyl)-6-prop-2-inyloxychinazolin-2-carbonsäurenaphthalin-1-ylamid
4-(4-Isopropylphenyl)-6-prop-2-inyloxychinazolin-2-carbonsäureisochinolin-8-ylamid
4-(4-Isopropylphenyl)-6-prop-2-inyloxychinazolin-2-carbonsäurephthalazin-5-ylamid
4-(4-Isopropylphenyl)-6-prop-2-inyloxychinazolin-2-carbonsäurechinolin-5-ylamid
4-(4-Isopropylphenyl)-6-prop-2-inyloxychinazolin-2-carbonsäurechinolin-8-ylamid
4-(4-Isopropylphenyl)-6-prop-2-inyloxychinazolin-2-carbonsäureisochinolin-4-ylamid
4-(4-Isopropylphenyl)-6-prop-2-inyloxychinazolin-2-carbonsäure(5-acetylchinolin-8-yl)amid
4-(4-Isopropylphenyl)-6-prop-2-inyloxychinazolin-2-carbonsäure(3-brom-6-methoxychinolin-8-yl)amid
4-(4-Isopropylphenyl)-6-prop-2-inyloxychinazolin-2-carbonsäurechinolin-2-ylamid
4-(4-Isopropylphenyl)-6-prop-2-inyloxychinazolin-2-carbonsäurechinolin-6-ylamid
4-(4-Isopropylphenyl)-6-prop-2-inyloxychinazolin-2-carbonsäure(2-methylchinolin-6-yl)amid
(6-{[4-(4-Isopropylphenyl)-6-prop-2-inyloxy-chinazolin-2-carbonyl]amino}chinolin-8-yloxy)essigsäureethylester
4-(4-Isopropylphenyl)-6-prop-2-inyloxychinazolin-2-carhonsäure(1H-benzimidazol-4-yl)amid
4-(4-Isopropylphenyl)-6-prop-2-inyloxychinazolin-2-carbonsäurebenzothiazol-2-ylamid
4-(4-Isopropylphenyl)-6-prop-2-inyloxychinazolin-2-carbonsäure(benzo[1,3]dioxol-5-ylmethyl)amid
4-(4-Isopropylphenyl)-6-prop-2-inyloxychinazolin-2-carbonsäure(thiophen-2-ylmethyl)amid
4-(4-Isopropylphenyl)-6-prop-2-inyloxychinazolin-2-carbonsäure-3-methoxyphenylester
1-(4-Isopropylphenyl)-7-prop-2-inyloxyisochinolin-3-carbonsäureethylester
1-(4-Isopropylphenyl)-7-prop-2-inyloxyisochinolin-3-carbonsäure-1,2-dimethylpropylester
1-(4-Isopropylphenyl)-7-prop-2-inyloxyisochinolin-3-carbonsäureisobutylester
1-(4-Isopropylphenyl)-7-prop-2-inyloxyisochinolin-3-carbonsäurecyclopropylmethylester
1-(4-Isopropylphenyl)-7-prop-2-inyloxyisochinolin-3-carbonsäurebenzylester
1-(4-Isopropylphenyl)-7-prop-2-inyloxyisochinolin-3-carbonsäure-2-methoxybenzylester
1-(4-Isopropylphenyl)-7-prop-2-inyloxyisochinolin-3-carbonsäure-3-methoxybenzylester
1-(4-Isopropylphenyl)-7-prop-2-inyloxyisochinolin-3-carbonsäure-4-methoxycarbonylbenzylester
1-(4-Isopropylphenyl)-7-prop-2-inyloxyisochinolin-3-carbonsäurephenethylester
1-(4-Isopropylphenyl)-7-prop-2-inyloxyisochinolin-3-carbonsäure-1-phenylethylester
1-(4-Isopropylphenyl)-7-prop-2-inyloxyisochinolin-3-carbonsäure[3-(2-hydroxyethan-sulfonyl)phenyl]amid
[1-(4-1sopropylphenyl)-7-prop-2-inyloxy-isochinolin-3-yl]-(3-methoxyphenyl)methanon
[1-(4-Isopropylphenyl)-7-prop-2-inyloxy-isochinolin-3-yl]-(4-methoxyphenyl)methanon.

7. Verbindung, ausgewählt aus der Gruppe bestehend aus
(4-Dimethylaminophenyl)-[4-(4-cyclopropylphenyl)-6-propargyloxychinazolin-2-yl]methanon und
(4-Ethoxyphenyl)-[4-(4-cyclopropylphenyl)-6-propargyloxychinazolin-2-yl]methanon.

8. Pharmazeutische Zusammensetzung, enthaltend eine Verbindung der Formel (I) zusammen mit einem pharmazeutisch unbedenklichen Hilfsstoff, Verdünnungsmittel oder Träger.

9. Verbindung der Formel (I) zur Förderung der Freisetzung von Parathormon.

10. Verfahren zur Herstellung einer Verbindung der Formel (I) nach Anspruch 1 oder Anspruch 7 in freier Form oder Salzform, bei dem man:
(i) für Fälle, in denen Q für N steht, eine Verbindung der Formel (III) in Gegenwart eines geeigneten Lösungsmittels mit einer Verbindung der Formel (IV) und einem Ammoniumsalz umsetzt: 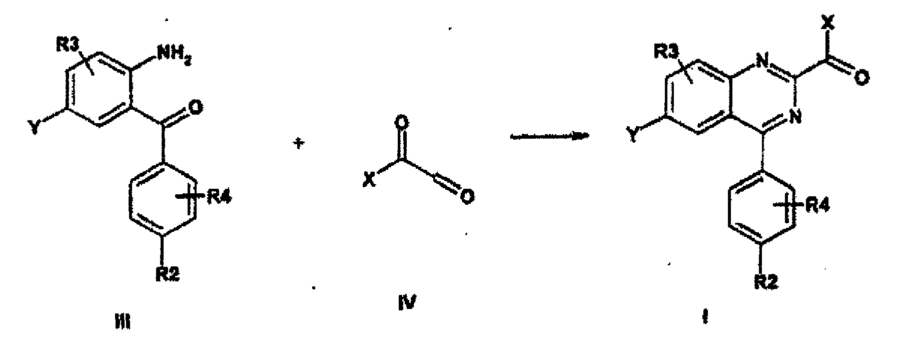 oder
(ii) eine Verbindung der Formel (V) 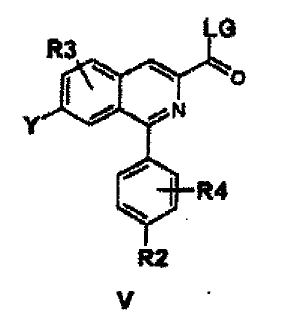 worin LG für eine geeignete Abgangsgruppe steht,
unter geeigneten wasserfreien Bedingungen mit einem metallorganischen Reagens der Formel VI:
X-Met VI
umsetzt; oder
(iii)eine Verbindung der Formel Va 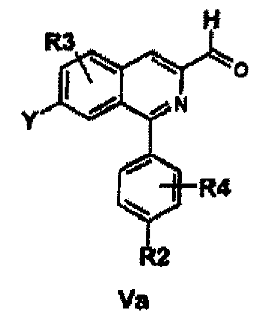 unter geeigneten wasserfreien Bedingungen mit einem metallorganischen Reagens der Formel VI:
X-Met VI
umsetzt und danach eine Oxidation zur Carbonylverbindung mit einem geeigneten Oxidationsmittel durchführt; oder
(iv) eine Verbindung der Formel VII 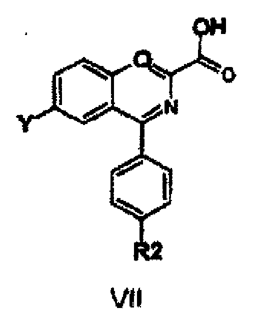 mit einer Verbindung X-H, worin das H Teil einer Amino- oder Hydroxygruppe ist, umsetzt, wobei die Umsetzung in Gegenwart eines Kupplungsmittels durchgeführt wird; oder
(v) eine Verbindung der Formel VIII 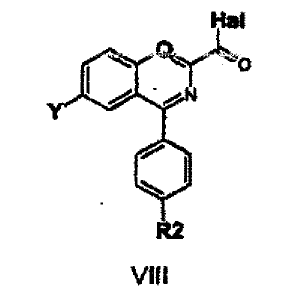
worin Hal für Halogen oder eine Abgangsgruppe steht,
mit einer Verbindung X-H, worin das H Teil einer Amino- oder Hydroxygruppe ist, umsetzt, wobei die Umsetzung in Gegenwart eines Kupplungsmittels durchgeführt wird.

11. Verwendung einer Verbindung der Formel (I) nach Anspruch 1 oder 7 bei der Herstellung eines Arzneimittels zur Prävention oder Behandlung von Knochenleiden, die mit erhöhter Calciumdepletion oder -resorption assoziiert sind oder bei denen die Stimulierung von Knochenbildung und Calciumfixierung im Knochen wünschenswert ist.

12. Kombination, enthaltend eine therapeutisch wirksame Menge einer Verbindung nach einem der Ansprüche 1 bis 7 und einen zweiten Arzneistoff, ausgewählt aus: Calcium, einem Calcitonin oder einem Analog oder Derivat davon, einem Steroidhormon, einem partiellen Östrogenagonisten oder einer Östrogen-Gestagen-Kombination, einem SERM (Selective Estrogen Receptor Modulator), einem RANKL-Antikörper, einem Cathepsin-K-Inhibitor, Vitamin D oder einem Analog davon oder PTH, einem PTH-Fragment oder einem PTH-Derivat, zur gleichzeitigen, separaten oder aufeinander folgenden Behandlung.

## Revendications

1. Composé de formule (I) ou sel ou ester promédicament pharmaceutiquement acceptable de celui-ci : 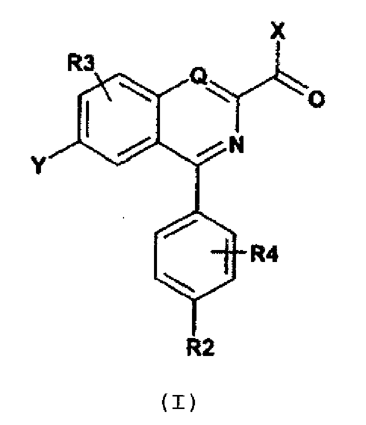 dans laquelle :
Q est CH ou N ;
R2 est un alkyle en C₁-C₄ ;
Y est R5-O- ;
où R5 est un éthynyle ou un propynyle ;
X est choisi dans le groupe constitué d'un aryle, un hétéroaryle, un alkyle en C₁-C₁₀, un alkyloxy en C₁-C₁₀, un cycloalkyle, un hétérocycloalkyle, un aryl(alkyle en C₁-C₄), un hétéroaryl(alkyle en C₁-C₄), un cycloalkyl(alkyle en C₁-C₄), un hétérocycloalkyl(alkyle en C₁-C₄), un arylamino, un hétéroarylamino, un aryl(alkyle en C₁-C₄)amino, un hétéroaryl (alkyle en C₁-C₄)amino, un (alkyle en C₁-C₆)amino, un di(alkyle en C₁-C₆)amino, un aryloxy, un hétéroaryloxy, un aryl(alkyloxy en C₁-C₄), un hétéroaryl(alkyloxy en C₁-C₄), un cycloalkyl(alkyle en C₁-C₄)amino, un hétérocycloalkyl(alkyle en C₁-C₄)amino, un cycloalkyl(alkyloxy en C₁-C₄) ou un hétérocycloalkyl(alkyloxy en C₁-C₄) dont chacun est facultativement substitué une ou plusieurs fois,
le substituant ou les substituants facultatif(s) sur X étant indépendamment choisis dans le groupe constitué d'un halogéno, un cyano, un trifluorométhyle, un nitro, un hydroxy et un (alkyle en C₁-C₄, alkyloxy, en C₁-C₄, amino, sulfanyle, sulfonyle, amino, oxycarbonyle, hydroxyle, sulfinyle, aminosulfonyle, sulfonylamino, carbonyle, carbonyloxy, carbonylamino, carboxyle, acyle, acylamino, ou carbamoyle) facultativement substitué, le substituant ou les substituants facultatif(s) étant choisis parmi un alkyle en C₁-C₆, un alkyloxy en C₁-C₆, un carboxyle, un hydroxyle, un hydroxy(alkyle en C₁-C₄) ; dont chacun peut lui-même être facultativement substitué par un alkyloxy, en C₁-C₆, un alkyle en C₁-C₆, un alkyle en C₁-C₃ fluoré, un alkyloxy en C₁-C₆, un carboxyle, un hydroxyle, un hydroxy(alkyle en C₁-C₄), un halogéno, un cyano, un nitro, et
R3 et R4 représentent chacun un ou plusieurs substituants indépendamment choisis parmi : H, un halogéno, un alkyle en C₁-C₄, un alkyloxy en C₁-C₄ et CF₃,
le substituant ou les substituants facultatif(s) sur R3 ou R4 étant indépendamment choisis dans le groupe constitué d'un alkyle en C₁-C₄, un halogéno, un alkyloxy en C₁-C₄, un cyano, un sulfanyle, un sulfonyle, un amino, un oxycarbonyle, un hydroxyle dont chacun peut lui-même être facultativement substitué une ou plusieurs fois par un alkyle en C₁-C₄, un halogéno, un alkyloxy en C₁-C₄, un cyano, un sulfanyle, un sulfonyle, un amino, un oxycarbonyle ou un hydroxyle.

2. Composé de formule (I') selon la revendication 1 ou un sel ou ester promédicament pharmaceutiquement acceptable de celui-ci : 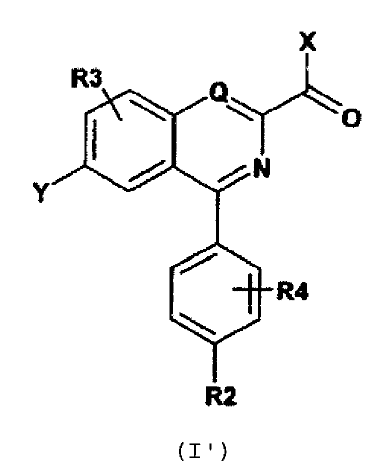 dans laquelle :
Q est CH ou N,
R2 est un alkyle en C₁-C₄,
Y est R5-O-,
où R5 est un éthynyle ou un propargyle,
X est choisi dans le groupe constitué d'un aryle, un hétéroaryle, un alkyle en C₁-C₆, un cycloalkyle, un hétérocycloalkyle, un aryl(alkyle en C₁-C₄), un hétéroaryl(alkyle en C₁-C₄), un arylamino, un hétéroarylamino, un aryl(alkyle en C₁-C₄)amino, un hétéroaryl(alkyle en C₁-C₄)amino, un (alkyle en C₁-C₆)amino, un di (alkyle en C₁-C₆)amino, un aryloxy, un hétéroaryloxy, un aryl(alkyloxy en C₁-C₄), ou un hétéroaryl(alkyloxy en C₁-C₄), dont chacun est facultativement substitué une ou plusieurs fois,
le substituant ou les substituants facultatif(s) sur X étant indépendamment choisis dans le groupe constitué d'un alkyle en C₁-C₄, un halogéno, un alkyloxy en C₁-C₄, un cyano, un trifluorométhyle, un hydroxy, un amino, un nitro et un alkyle en C₁-C₄ et un (sulfanyle, sulfonyle, amino, oxycarbonyle, hydroxyle, sulfinyle, aminosulfonyle, sulfonylamino, carbonyle, carbonyloxy, carbonylamino, carboxyle, acyle, acylamino, ou carbamoyle) substitué,
R3 et R4 représentent chacun un ou plusieurs substituants indépendamment choisis parmi : H, un halogéno, un alkyle en C₁-C₄ facultativement substitué et un alkyloxy en C₁-C₄ facultativement substitué,
le substituant ou les substituants facultatif(s) sur R3 ou R4 étant indépendamment choisis dans le groupe constitué d'un alkyle en C₁-C₄, un halogéno, un alkyloxy en C₁-C₄, un cyano, un sulfanyle, un sulfonyle, un amino, un oxycarbonyle et un hydroxyle.

3. Composé selon la revendication 1 ayant la formule (II) ou un sel ou ester promédicament pharmaceutiquement acceptable de celui-ci : 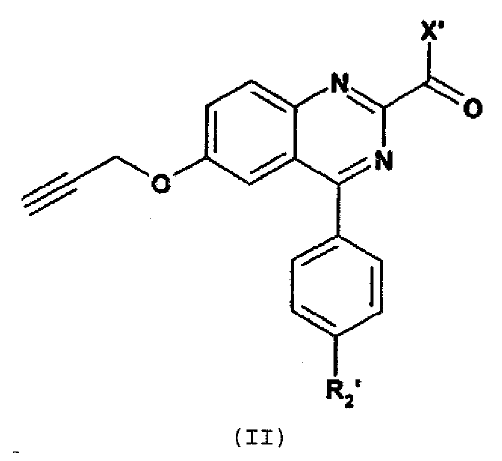 dans laquelle :
X' est choisi dans le groupe constitué d'un aryle, un hétéroaryle, un cycloalkyle, un hétérocycloalkyle, un (alkyle en C₁-C₄)aryle, un (alkyle en C₁-C₄)hétéroaryle, un arylamino, un hétéroarylamino, un aryl(alkyle en C₁-C₄)amino, un hétéroaryl (alkyle en C₁-C₄)amino, un aryloxy, un hétéroaryloxy, un aryl(alkyloxy en C₁-C₄), un hétéroaryl(alkyloxy en C₁-C₄), un aryl(alkyle en C₁-C₄), un hétéroaryl (alkyle en C₁-C₄), un alkyle en C₁-C₆, un (alkyle en C₁-C₄)amino ou un amino, dont chacun est facultativement substitué une ou plusieurs fois,
le substituant ou les substituants facultatif(s) sur X' étant indépendamment choisis dans le groupe constitué d'un halogéno, un cyano, un trifluorométhyle, un nitro, un hydroxy et un (alkyle en C₁-C₄, alkyloxy en C₁-C₄, amino, sulfanyle, sulfonyle, amino, oxycarbonyle, hydroxyle, sulfinyle, carbonyle, carboxyle, acyle, arylamino, ou carbamoyle) facultativement substitué ;
le substituant ou les substituants facultatif(s) étant choisis parmi un alkyle en C₁-C₆, un alkyloxy en C₁-C₆, un carboxyle, un hydroxyle et un hydroxy(alkyle en C₁-C₄) ; dont chacun peut lui-même être facultativement substitué par un alkyloxy en C₁-C₆, un alkyle en C₁-C₆, un alkyloxy en C₁-C₆, un carboxyle, un hydroxyle, un hydroxy(alkyle en C₁-C₄), un halogéno, un cyano, un nitro, et
R₂' est un alkyle en C₁-C₄.

4. Composé selon la revendication 3 **caractérisé en ce que** R₂' est un isopropyle.

5. Composé selon la revendication 2 ou 3 dans laquelle X' est un (aryle, hétéroaryle, hétérocycloalkyle, arylamino, hétéroarylamino, aryl(alkyle en C₁-C₄)amino, hétéroaryl(alkyle en C₁-C₄)amino, aryloxy, hétéroaryloxy, alkyloxy en C₁-C₆, aryl(alkyloxy en C₁-C₄) ou hétéroaryl(alkyloxy en C₁-C₄)) facultativement substitué où les substituants sont comme défini dans la revendication 2 ou la revendication 3, respectivement.

6. Composé selon l'une quelconque des revendications précédentes choisi parmi les suivants :
(4-tert-Butyl-phényl)-[4-(4-isopropyl-phényl)-6-propargyloxy-quinazolin-2-yl]-méthanone
[4-(4-Isopropyl-phényl)-6-propargyloxy-quinazolin-2-yl]-phényl-méthanone
(2-Méthoxy-phényl)-[4-(4-isopropyl-phényl)-6-propargyloxy-quinazolin-2-yl]-méthanone
(3-Méthoxy-phényl)-[4-(4-isopropyl-phényl)-6-propargyloxy-quinazolin-2-yl]-méthanone
(4-Méthoxy-phényl)-[4-(4-isopropyl-phényl)-6-propargyloxy-quinazolin-2-yl]-méthanone
(4-Fluoro-phényl)-[4-(4-isopropyl-phényl)-6-propargyloxy-quinazolin-2-yl]-méthanone
(3-Fluoro-phényl)-[4-(4-isopropyl-phényl)-6-propargyloxy-quinazolin-2-yl]-méthanone
(3-Chloro-phényl)-[4-(4-isopropyl-phényl)-6-propargyloxy-quinazolin-2-yl]-méthanone
(4-Chloro-phényl)-[4-(4-isopropyl-phényl)-6-propargyloxy-quinazolin-2-yl]-méthanone
(4-Fluoro-phényl)-[4-(4-tert-butyl-phényl)-6-propargyloxy-quinazolin-2-yl]-méthanone
(3-Fluoro-phényl)-[4-(4-tert-butyl-phényl)-6-propargyloxy-quinazolin-2-yl]-méthanone
(3-Bromo-phényl)-[4-(4-isopropyl-phényl)-6-propargyloxy-quinazolin-2-yl]-méthanone
(4-Bromo-phényl)-[4-(4-isopropyl-phényl)-6-propargyloxy-quinazolin-2-yl]-méthanone
(4-Méthyl-phényl)-[4-(4-isopropyl-phényl)-6-propargyloxy-quinazolin-2-yl]-méthanone
(4-Isopropyl-phényl)-[4-(4-isopropyl-phényl)-6-propargyloxy-quinazolin-2-yl]-méthanone
(4-Éthyl-phényl)-[4-(4-isopropyl-phényl)-6-propargyloxy-quinazolin-2-yl]-méthanone
(4-Propyl-phényl)-[4-(4-isopropyl-phényl)-6-propargyloxy-quinazolin-2-yl]-méthanone
(4-Cyano-phényl)-[4-(4-isopropyl-phényl)-6-propargyloxy-quinazolin-2-yl]-méthanone
(4-Méthylthio-phényl)-[4-(4-isopropyl-phényl)-6-propargyloxy-quinazolin-2-yl]-méthanone
(4-Méthanesulfonyl-phényl)-[4-(4-isopropyl-phényl)-6-propargyloxy-quinazolin-2-yl]-méthanone
(4-Diméthylamino-phényl)-[4-(4-isopropyl-phényl)-6-propargyloxy-quinazolin-2-yl]-méthanone
(4-Éthoxy-phényl)-[4-(4-isopropyl-phényl)-6-propargyloxy-quinazolin-2-yl]-méthanone
Ester méthylique d'acide 4-[4-(4-isopropyl-pnényl)-6-propargyloxy-quinazoline-2-carbonyl]-benzoïque
(4-Diméthylamino-phényl)-[4-(4-tert-butyl-phényl)-6-propargyloxy-quinazolin-2-yl]-méthanone
Ester éthylique d'acide 4-[4-(4-isopropyl-phényl)-6-propargyloxy-quinazoline-2-carbonyl]-benzoïque
(4-Méthoxy-phényl)-[4-(4-tert-butyl-phényl)-6-propargyloxy-quinazolin-2-yl]-méthanone
(3-Éthoxy-4-méthoxy-phényl)-[4-(4-isopropyl-phényl)-6-propargyloxy-quinazolin-2-yl]-méthanone
(4-tert-Butyloxy-phényl)-[4-(4-isopropyl-phényl)-6-propargyloxy-quinazolin-2-yl]-méthanone
(4-Hydroxy)-[4-(4-isopropyl-phényl)-6-propargyloxy-quinazolin-2-yl]-méthanone
(4-Butyloxy)-[4-(4-isopropyl-phényl)-6-propargyloxy-quinazolin-2-yl]-méthanone
Furan-2-yl-[4-(4-isopropyl-phényl)-6-propargyloxy-quinazolin-2-yl]-méthanone
Furan-3-yl-[4-(4-tert-butyl-phényl)-6-propargyloxy-quinazolin-2-yl]-méthanone
Furan-3-yl-[4-(4-isopropyl-phényl)-6-propargyloxy-quinazolin-2-yl]-méthanone
Thiophén-2-yl-[4-(4-isopropyl-phényl)-6-propargyloxy-quinazolin-2-yl]-méthanone (3-Méthyl-thiophén-2-yl-[4-(4-isopropyl-phényl)-6-propargyloxy-quinazolin-2-yl]-méthanone
Benz[b]thiophén-2-yl-[4-(4-isopropyl-phényl)-6-propargyloxy-quinazolin-2-yl]-méthanone
Thiophén-3-yl-[4-(4-isopropyl-phényl)-6-propargyloxy-quinazolin-2-yl]-méthanone
(1-Méthyl-1H-pyrrol)-2-yl-[4-(4-isopropyl-phényl)-6-propargyloxy-quinazolin-2-yl]-méthanone
Ester éthylique d'acide 4-(4-isopropyl-phényl)-6-propargyloxy-quinazoline-2-carboxylique
[4-(4-isopropyl-phényl)-6-propargyloxy-quinazolin-2-yl]-pyridine-3-yl-méthanone
[4-(4-isopropyl-phényl)-6-propargyloxy-quinazolin-2-yl]-naphtalén-1-yl-méthanone
[4-(4-isopropyl-phényl)-6-propargyloxy-naphathalén-2-yl]-méthanone
Benzothiazol-2-yl-[4-(4-isopropyl-phényl)-6-propargyloxy-quinazolin-2-yl]-méthanone
[4-(4-isopropyl-phényl)-6-propargyloxy-quinazolin-yl]-thiazol-5-yl-méthanone
[4-(4-isopropyl-phényl)-6-propargyloxy-quinazolin-2-yl]-pipéridin-1-yl-méthanone
(3-Chloro-phényl)-amide d'acide 4-(4-isopropyl-phényl)-6-prop-2-ynyloxy-quinazoline-2-carboxylique
(3-Méthoxy-phényl)-amide d'acide 4-(4-isopropyl-phényl)-6-prop-2-ynyloxy-quinazoline-2-carboxylique
(3-Méthylsulfanyl-phényl)-amide d'acide 4-(4-isopropyl-phényl)-6-prop-2-ynyloxy-3,4-dihydro-quinazoline-2-carboxylique
(3-Méthanesulfonyl-phényl)-amide d'acide 4-(4-isopropyl-phényl)-6-prop-2-ynyloxy-3,4-dihydro-quinazoline-2-carboxylique
(3-Trifluorométhylsulfanyl-phényl)-amide d'acide 4-(4-isopropyl-phényl)-6-prop-2-ynyloxy-quinazoline-2-carboxylique
(3-Sulfamoyl-phényl)-amide d'acide 4-(4-isopropyl-phényl)-6-prop-2-ynyloxy-3,4-dihydro-quinazoline-2-carboxylique
[3-(2-Hydroxy-éthanesulfonyl)-phényl]-amide d'acide 4-(4-isopropyl-phényl)-6-prop-2-ynyloxy-3,4-dihydro-quinazoline-2-carboxylique
(5-Éthanesulfonyl-2-hydroxy-phényl)-amide d'acide 4-(4-isopropyl-phényl)-6-prop-2-ynyloxy-3,4-dihydro-quinazoline-2-carboxylique
(3-Nitro-phényl)-amide d'acide 4-(4-isopropyl-phényl)-6-prop-2-ynyloxy-quinazoline-2-carboxylique
(3-Cyano-phényl)-amide d'acide 4-(4-isopropyl-phényl)-6-prop-2-ynyloxy-quinazoline-2-carboxylique
Ester méthylique d'acide 3-{[4-(4-isopropyl-phényl)-6-prop-2-ynyloxy-quinazoline-2-carbonyl]-amino}-benzoïque
Ester éthylique d'acide 3-{[4-(4-isopropyl-phényl)-6-prop-2-ynyloxy-quinazoline-2-carbonyl]-amino}-benzoïque
Ester isopropylique d'acide 3-{[4-(4-isopropyl-phényl)-6-prop-2-ynyloxy-quinazoline-2-carbonyl]-amino}-benzoïque
Ester tert-butylique d'acide 3-{[4-(4-isopropyl-phényl)-6-prop-2-ynyloxy-quinazoline-2-carhonyl]-amino}-benzoïque
(3-Carbamoyl-phényl)-amide d'acide 4-(4-isopropyl-phényl)-6-prop-2-ynyloxy-quinazoline-2-carboxylique
(3-Acétyl-phényl)-amide d'acide 4-(4-isopropyl-phényl)-6-prop-2-ynyloxy-quinazoline-2-carboxylique
Ester méthylique d'acide 3-{[4-(4-isopropyl-phényl)-6-prop-2-ynyloxy-quinazoline-2-carbonyl]-amino}-5-méthoxy-benzoïque
(3-Méthylcarbamoyl-phényl)-amide d'acide 4-(4-isopropyl-phényl)-6-prop-2-ynyloxy-quinazoline-2-carboxylique
(3-tert-Butylcarbamoyl-phényl)-amide d'acide 4-(4-isopropyl-phényl)-6-prop-2-ynyloxy-quinazoline-2-carboxylique
(3-Diméthylcarbamoyl-5-trifluorométhyl-phényl)-amide d'acide 4-(4-isopropyl-phényl)-6-prop-2-ynyloxy-quinazoline-2-carboxylique
Ester méthylique d'acide 3-{[4-(4-isopropyl-phényl)-6-prop-2-ynyloxy-quinazoline-2-carbonyl]-amino}-5-trifluorométhyl-benzoïque
Ester isopropylique d'acide 3-{[4-(4-isopropyl-phényl)-6-prop-2-ynyloxy-quinazoline-2-carbonyl]-amino}-5-trifluorométhyl-benzoïque
Ester méthylique d'acide 2-fluoro-5-{[4-(4-isopropyl-phényl)-6-prop-2-ynyloxy-quinazoline-2-carbonyl]-amino}-benzoïque
Ester isopropylique d'acide 2-fluoro-5-{[4-(4-isopropyl-phényl)-6-prop-2-ynyloxy-quinazoline-2-carbonyl]-amino}-benzoïque
Ester méthylique d'acide 2-chloro-5-{[4-(4-isopropyl-phényl)-6-prop-2-ynyloxy-quinazoline-2-carbonyl]-amino}-benzoïque
Ester méthylique d'acide 2,5-dichloro-3-{[4-(4-isopropyl-phényl)-6-prop-2-ynyloxy-quinazoline-2-carbonyl]-amino}-benzoïque
Ester isopropylique d'acide 2,5-dichloro-3-{[4-(4-isopropyl-phényl)-5-prop-2-ynyloxy-quinazoline-2-carbonyl]-amino}-benzoïque
(3-Cyano-5-fluoro-phényl)-amide d'acide 4-(4-isopropyl-phényl)-6-prop-2-ynyloxy-quinazoline-2-carboxylique
(3,4-Dicyano-phényl)-amide d'acide 4-(4-isopropyl-phényl)-6-prop-2-ynyloxy-quinazoline-2-carboxylique
(4-Cyano-3-trifluorométhyl-phényl)-amide d'acide 4-(4-isopropyl-phényl)-6-prop-2-ynyloxy-quinazoline-2-carboxylique
(3-Trifluorométhyl-phényl)-amide d'acide 4-(4-isopropyl-phényl)-6-prop-2-ynyloxy-quinazoline-2-carboxylique
(4-Acétylamino-3-trifluorométhyl-phényl)-amide d'acide 4-(4-isopropyl-phényl)-5-prop-2-ynyloxy-quinazoline-2-carboxylique
(3-Méthoxy-5-trifluorométhyl-phényl)-amide d'acide 4-(4-isopropyl-phényl)-6-prop-2-ynyloxy-quinazoline-2-carboxylique
(3,5-bis-Trifluorométhyl-phényl)-amide d'acide 4-(4-isopropyl-phényl)-6-prop-2-ynyloxy-quinazoline-2-carboxylique
(3-Fluoro-5-trifluorométhyl-phényl)-amide d'acide 4-(4-isopropyl-phényl)-6-prop-2-ynyloxy-quinazoline-2-carboxylique
(4-Fluoro-3-trifluorométhyl-phényl)-amide d'acide 4-(4-isopropyl-phényl)-6-prop-2-ynyloxy-quinazoline-2-carboxylique
Ester méthylique d'acide 3-{[4-(4-isopropyl-phényl)-6-prop-2-ynyloxy-quinazoline-2-carbonyl]-amino}-2-méthyl-benzoïque
Ester méthylique d'acide 3-{[4-(4-isopropyl-phényl)-6-prop-2-ynyloxy-quinazoline-2-carbonyl]-amino}-4-méthyl-benzoïque
Ester méthylique d'acide 3-{[4-(4-isopropyl-phényl)-6-prop-2-ynyloxy-quinazoline-2-carbonyl]-amino}-4-méthoxy-benzoïque
Ester diméthylique d'acide 5-{[4-(4-isopropyl-phényl)-6-prop-2-ynyloxy-quinazoline-2-carbonyl]-amino}-isophtalique
Ester diméthylique d'acide 4-{[4-(4-isopropyl-phényl)-6-prop-2-ynyloxy-quinazoline-2-carbonyl]-amino}-phtalique
(3,5-Dichloro-phényl)-amide d'acide 4-(4-isopropyl-phényl)-6-prop-2-ynyloxy-quinazoline-2-carboxylique
(3,4-Dichloro-phényl)-amide d'acide 4-(4-isopropyl-phényl)-6-prop-2-ynyloxy-quinazoline-2-carboxylique
(3-Chloro-4-fluoro-phényl)-amide d'acide 4-(4-isopropyl-phényl)-6-prop-2-ynyloxy-quinazoline-2-carboxylique
(4-Chloro-3-trifluorométhyl-phényl)-amide d'acide 4-(4-isopropyl-phényl)-6-prop-2-ynyloxy-quinazoline-2-carboxylique
Ester méthylique d'acide 5-{[4-(4-isopropyl-phényl)-6-prop-2-ynyloxy-quinazoline-2-carbonyl]-amino}-pyridine-2-carboxylique
Ester méthylique d'acide 5-{[4-(4-isopropyl-phényl)-6-prop-2-ynyloxy-quinazoline-2-carbonyl]-amino}-nicotinique
Ester isopropylique d'acide 5-{[4-(4-isopropyl-phényl)-6-prop-2-ynyloxy-quinazoline-2-carbonyl]-amino}-nicotinique
[4-(4-isopropyl-phényl)-6-prop-2-ynyloxy-quinazolin-2-yl]-pyrrol-1-yl-méthanone
(5-Méthyl-1H-pyrazol-3-yl)-amide d'acide 4-(4-isopropyl-phényl)-6-prop-2-ynyloxy-quinazoline-2-carboxylique
Ester éthylique d'acide (2-{[4-(4-isopropyl-phényl)-6-prop-2-ynyloxy-quinazoline-2-carbonyl]-amino}-thiazol-4-yl)-acétique
Naphtalén-1-ylamide d'acide 4-(4-isopropyl-phényl)-6-prop-2-ynyloxy-quinazoline-2-carboxylique
Isoquinoléin-8-ylamide d'acide 4-(4-isopropyl-phényl)-6-prop-2-ynyloxy-quinazoline-2-carboxylique
Phtalazin-5-ylamide d'acide 4-(4-isopropyl-phényl)-6-prop-2-ynyloxy-quinazoline-2-carboxylique
Quinoléin-5-ylamide d'acide 4-(4-isopropyl-phényl)-6-prop-2-ynyloxy-quinazoline-2-carboxylique
Quinoléin-8-ylamide d'acide 4-(4-isopropyl-phényl)-6-prop-2-ynyloxy-quinazoline-2-carboxylique
Isoquinoléin-4-ylamide d'acide 4-(4-isopropyl-phényl)-6-prop-2-ynyloxy-quinazoline-2-carboxylique
(5-Acétyl-quinoléin-8-yl)-amide d'acide 4-(4-isopropyl-phényl)-6-prop-2-ynyloxy-quinazoline-2-carboxylique
(3-Bromo-6-méthoxy-quinoléin-8-yl)-amide d'acide 4-(4-isopropyl-phényl)-6-prop-2-ynyloxy-quinazoline-2-carboxylique
Quinoléin-2-ylamide d'acide 4-(4-isopropyl-phényl)-6-prop-2-ynyloxy-quinazoline-2-carboxylique
Quinoléin-6-ylamide d'acide 4-(4-isopropyl-phényl)-6-prop-2-ynyloxy-quinazoline-2-carboxylique
(2-Méthyl-quinoléin-6-yl)-amide d'acide 4-(4-isopropyl-phényl)-5-prop-2-ynyloxy-quinazoline-2-carboxylique
Ester éthylique d'acide {[4-(4-isopropyl-phényl)-6-prop-2-ynyloxy-quinazoline-2-carbonyl]-amino}-quinoléin-8-yloxy)-acétique
(1H-Benzoimidazol-4-yl)-amide d'acide 4-(4-isopropyl-phényl)-6-prop-2-ynyloxy-quinazoline-2-carboxylique
Benzothiazol-2-ylamide d'acide 4-(4-isopropyl-phényl)-6-prop-2-ynyloxy-quinazoline-2-carboxylique
(Benzo[1,3]dioxol-5-ylméthyl)-amide d'acide 4-(4-isopropyl-phényl)-6-prop-2-ynyloxy-quinazoline-2-carboxylique
(Thiophén-2-ylméthyl)-amide d'acide 4-(4-isopropyl-phényl)-6-prop-2-ynyloxy-quinazoline-2-carboxylique
Ester 3-méthoxy-phénylique d'acide 4-(4-isopropyl-phényl)-6-prop-2-ynyloxy-quinazoline-2-carboxylique
Ester éthylique d'acide 1-(4-isopropyl-phényl)-7-prop-2-ynyloxy-isoquinoléine-3-carboxylique
Ester 1,2-diméthyl-propylique d'acide 1-(4-isopropyl-phényl)-7-prop-2-ynyloxy-isoquinoléine-3-carboxylique
Ester isobutylique d'acide 1-(4-isopropyl-phényl)-7-prop-2-ynyloxy-isoquinoléine-3-carboxylique
Ester cyclopropylméthylique d'acide 1-(4-isopropyl-phényl)-7-prop-2-ynyloxy-isoquinoléine-3-carboxylique
Ester benzylique d'acide 1-(4-isopropyl-phényl)-7-prop-2-ynyloxy-isoquinoléine-3-carboxylique
Ester 2-méthoxy-benzylique d'acide 1-(4-isopropyl-phényl)-7-prop-2-ynyloxy-isoquinoléine-3-carboxylique
Ester 3-méthoxy-benzylique d'acide 1-(4-isopropyl-phényl)-7-prop-2-ynyloxy-isoquinoléine-3-carboxylique
Ester 4-méthoxycarbonyl-benzylique d'acide 1-(4-isopropyl-phényl)-7-prop-2-ynyloxy-isoquinléine-3-carboxylique
Ester phénéthylique d'acide 1-(4-isopropyl-phényl)-7-prop-2-ynyloxy-isoquinoléine-3-carboxylique
Ester 1-phényl-éthylique d'acide 1-(4-isopropyl-phényl)-7-prop-2-ynyloxy-isoquinoléine-3-carboxylique
[3-(2-Hydroxy-éthanesulfonyl)-phényl]-amide d'acide 1-(4-isopropyl-phényl)-7-prop-2-ynyloxy-isoquinoléine-3-carboxylique
[1-(4-isopropyl-phényl)-7-prop-2-ynyloxy-isoquinoléin-3-yl]-(3-méthoxy-phényl)-méthanone
[1-(4-isopropyl-phényl)-7-prop-2-ynyloxy-isoquinoléin-3-yl]-(4-méthoxy-phényl)-méthanone.

7. Composé choisi dans le groupe constitué de :
(4-Diméthylamino-phényl)-[4-(4-cyclopropyl-phényl)-6-propargyloxy-quinazolin-2-yl]-méthanone et
4-Éthoxy-phényl)-[4-(4-cyclopropyl-phényl)-6-propargyloxy-quinazolin-2-yl]-méthanone.

8. Composition pharmaceutique comprenant un composé de formule (I) en association avec un excipient, diluant ou véhicule pharmaceutiquement acceptable.

9. Composé de formule (I) pour favoriser la libération de parathormone.

10. Procédé pour la préparation d'un composé de formule (I) selon la revendication 1 ou la revendication 7 sous forme libre ou de sel, comprenant l'étape de :
(i) pour les cas dans lesquels Q est N, réaction d'un composé de formule (III) avec un composé de formule (IV) et un sel d'ammonium en présence d'un solvant adapté : 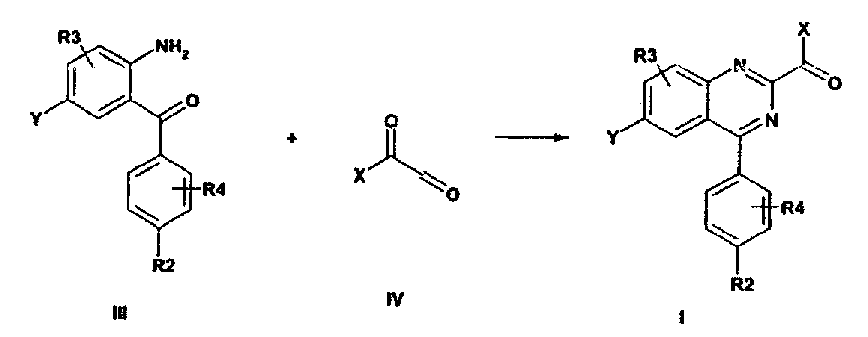 ou
(ii) réaction d'un composé de formule V 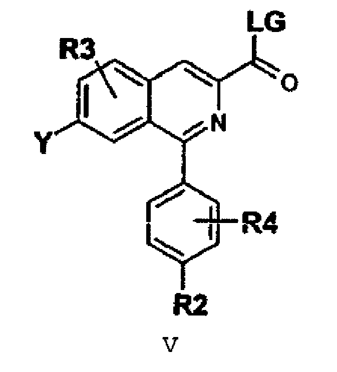 dans laquelle LG représente un groupe partant adapté ; avec un réactif organométallique de formule VI :
X-Met VI
dans des conditions anhydres adaptées ; ou
(iii) réaction d'un composé de formule Va 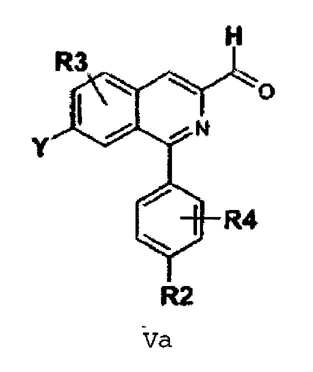 avec un réactif organométallique de formule VI :
X-Met VI
dans des conditions anhydres adaptées suivies par l'oxydation en composé carbonyle par un agent d'oxydation approprié ; ou
(iv) réaction d'un composé de formule VII 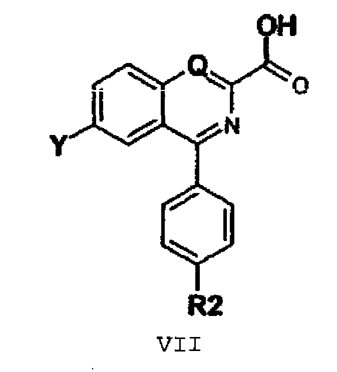 avec un composé X-H où le H fait partie d'un groupe amino ou hydroxy, la réaction étant conduite en présence d'un réactif de coupage ; ou
(v) réaction d'un composé de formule VIII 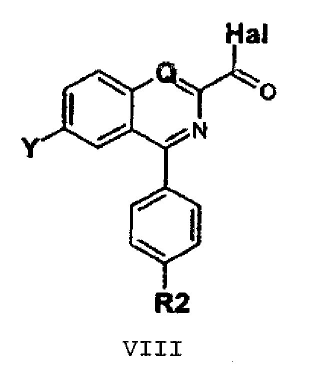
dans laquelle Hal est un halogène ou un groupe partant, avec un composé X-H dans lequel le H fait partie d'un groupe amino ou hydroxy, la réaction étant conduite en présence d'un réactif de couplage.

11. Utilisation d'un composé de formule (I) selon la revendication 1 ou 7 dans la fabrication d'un médicament pour prévenir ou traiter des pathologies osseuses qui sont associées à une augmentation de la déplétion ou la résorption de calcium ou dans lesquelles la stimulation de la formation osseuse et la fixation de calcium dans les os est souhaitable.

12. Combinaison comprenant une quantité thérapeutiquement efficace d'un composé selon l'une quelconque des revendications 1 à 7 et une deuxième substance pharmaceutique choisie parmi : le calcium, une calcitonine ou un analogue ou un dérivé de celle-ci, une hormone stéroïde, un agoniste partiel d'oestrogène ou une combinaison oestrogène-gestagène, un SERM (modulateur de récepteur d'oestrogène sélectif), un anticorps anti-RANKL, un inhibiteur de cathepsine K, la vitamine D ou un analogue de celle-ci ou PTH, un fragment de PTH ou un dérivé de PTH pour traitement simultané, séparé ou séquentiel.
